(19) Europäisches Patentamt / European Patent Office / Office européen des brevets

(11) **EP 2 665 812 B1**

(12) **EUROPEAN PATENT SPECIFICATION**

(45) Date of publication and mention
of the grant of the patent:
**14.09.2016 Bulletin 2016/37**

(51) Int Cl.:
*C12N 9/08* (2006.01)     *C12N 15/80* (2006.01)

(21) Application number: **12700843.1**

(86) International application number:
**PCT/EP2012/050910**

(22) Date of filing: **20.01.2012**

(87) International publication number:
**WO 2012/098246 (26.07.2012 Gazette 2012/30)**

(54) **EXPRESSION OF PLANT PEROXIDASES IN FILAMENTOUS FUNGI**

EXPRESSION VON PFLANZENPEROXIDASEN IN FASERIGEN PILZEN

EXPRESSION DE PEROXYDASES D'ORIGINE VÉGÉTALE DANS DES CHAMPIGNONS FILAMENTEUX

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.01.2011 EP 11151562**

(43) Date of publication of application:
**27.11.2013 Bulletin 2013/48**

(73) Proprietor: **Novozymes A/S**
**2880 Bagsvaerd (DK)**

(72) Inventors:
• **OESTERGAARD, Lars, Henrik**
**2920 Charlottenlund (DK)**
• **KALUM, Lisbeth**
**3500 Vaerloese (DK)**

(74) Representative: **Bauditz, Peter**
**Novozymes A/S**
**Patents**
**Krogshøjvej 36**
**2880 Bagsværd (DK)**

(56) References cited:
WO-A1-2004/069872     WO-A2-98/55629
WO-A2-03/070957

• GOUKA R J ET AL: "Efficient production of secreted proteins by Aspergillus: progress, limitations and prospects", APPLIED MICROBIOLOGY AND BIOTECHNOLOGY, SPRINGER VERLAG, BERLIN, DE, vol. 47, no. 1, 1 January 1997 (1997-01-01), pages 1-11, XP002121584, ISSN: 0175-7598, DOI: DOI:10.1007/S002530050880
• RUCHIKA SHARMA ET AL: "Approaches for refining heterologous protein production in filamentous fungi", WORLD JOURNAL OF MICROBIOLOGY AND BIOTECHNOLOGY, RAPID COMMUNICATIONS OF OXFORD, OXFORD, GB, vol. 25, no. 12, 2 August 2009 (2009-08-02), pages 2083-2094, XP002629470, ISSN: 0959-3993, DOI: DOI:10.1007/S11274-009-0128-X
• PUNT P J ET AL: "Filamentous fungi as cell factories for heterologous protein production", TRENDS IN BIOTECHNOLOGY, ELSEVIER PUBLICATIONS, CAMBRIDGE, GB, vol. 20, no. 5, 1 May 2002 (2002-05-01), pages 200-206, XP004349665, ISSN: 0167-7799, DOI: DOI:10.1016/S0167-7799(02)01933-9
• S. HIRAGA ET AL: "A Large Family of Class III Plant Peroxidases", PLANT AND CELL PHYSIOLOGY, vol. 42, no. 5, 1 May 2001 (2001-05-01), pages 462-468, XP055211479, DOI: 10.1093/pcp/pce061

**Description**

**Reference to a Sequence Listing**

This application contains a Sequence Listing in computer readable form, which is incorporated herein by reference.

**Background of the Invention**

**Field of the Invention**

The present invention relates to methods and compositions for recombinant expression of wildtype plant peroxidases, or peroxidases derived therefrom, in *filamentous fungal* host organisms.

**Description of the Related Art**

Peroxidases and laccases are well-known enzymes belonging to the group of oxidoreductases. Peroxidases belong to enzyme class EC 1.11.1.7, and laccases belong to EC 1.10.3.2. Both enzyme classes are capable of oxidizing substrates, and therefore they are often used in bleaching applications. Commercial applications include bleaching of denim (abraded look on jeans), bleaching of rinse water after a textile dyeing process, and dye transfer inhibition during a laundering process.

Usually plant peroxidases are purified from plants, but this is a complex process with low yields. Alternatively, recombinant expression in bacteria or yeast can be used, but this also results in poor yields. The need for efficient recombinant production of peroxidases and laccases is thus apparent.

However, the scientific literature is absent of examples showing expression of oxidoreductases derived from plants, in filamentous fungi like *Aspergillus. Aspergillus* sp. and other filamentous fungi are often used as highly efficient expression hosts for recombinant expression of enzymes. Since researchers rarely report in the literature what does not work, it is believed that the lack of successful examples of oxidoreductase expression illustrates, that it is not considered possible (a technical prejudice) to express plant-derived oxidoreductases in filamentous fungi.

The assumption that plant-derived oxidoreductases cannot be expressed in e.g. *Aspergillus* sp., is supported by the fact that the inventors of the present invention earlier unsuccessfully attempted expression of a number of plant derived laccases in *Aspergillus* sp.

WO 98/55629 discloses a soybean peroxidase gene family and an assay for detecting soybean peroxidase activity. The application discloses genomic DNA and promoters of soybean peroxidases and their use as promoters for producing transgenic plants, including transgenic soybeans.

**Summary of the Invention**

The inventors of the present invention have found that it is indeed possible expressing plant peroxidases in *Aspergillus* host cells. Accordingly, the present invention provides methods for recombinant expression in an *Aspergillus* host organism a nucleic acid sequence encoding a class III peroxidase from EC 1.11.1.7, wherein the amino acid sequence of the peroxidase comprises the amino acid motifs:

    HFHDCFV;
    GCD[A,G]S[V,I][I,L][I,L]; and
    VSC[A,S]D[I,L][I,L].

In another aspect, the invention provides an *Aspergillus* host organism comprising a nucleic acid sequence encoding a class III peroxidase from EC 1.11.1.7, capable of expressing at least 20 mg/L of peroxidase, wherein the amino acid sequence of the peroxidase comprises the amino acid motifs:

    HFHDCFV;
    GCD[A,G]S[V,I][I,L][I,L]; and
    VSC[A,S]D[I,L][I,L].

**Definitions**

**Sequence Identity:** The relatedness between two amino acid sequences or between two nucleotide sequences is described by the parameter "sequence identity".

For purposes of the present invention, the degree of sequence identity between two amino acid sequences is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 3.0.0 or later. The optional parameters used are gap open penalty of 10, gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows:

$$\text{(Identical Residues x 100)/(Length of Alignment – Total Number of Gaps in Alignment)}$$

For purposes of the present invention, the degree of sequence identity between two deoxyribonucleotide sequences is determined using the Needleman-Wunsch algorithm (Needleman and Wunsch, 1970, *supra*) as implemented in the Needle program of the EMBOSS package (EMBOSS: The European Molecular Biology Open Software Suite, Rice et al., 2000, *supra*), preferably version 3.0.0 or later. The optional parameters used are gap open penalty of 10, gap extension penalty of 0.5, and the EDNAFULL (EMBOSS version of NCBI NUC4.4) substitution matrix. The output of Needle labeled "longest identity" (obtained using the -nobrief option) is used as the percent identity and is calculated as follows:

$$\text{(Identical Deoxyribonucleotides x 100)/(Length of Alignment – Total Number of Gaps in Alignment)}$$

**Coding sequence:** The term "coding sequence" means a polynucleotide, which directly specifies the amino acid sequence of a polypeptide. The boundaries of the coding sequence are generally determined by an open reading frame, which usually begins with the ATG start codon or alternative start codons such as GTG and TTG and ends with a stop codon such as TAA, TAG, and TGA. The coding sequence may be a DNA, cDNA, synthetic, or recombinant polynucleotide.

**cDNA:** The term "cDNA" means a DNA molecule that can be prepared by reverse transcription from a mature, spliced, mRNA molecule obtained from a eukaryotic cell. cDNA lacks intron sequences that may be present in the corresponding genomic DNA. The initial, primary RNA transcript is a precursor to mRNA that is processed through a series of steps, including splicing, before appearing as mature spliced mRNA.

**Nucleic acid construct:** The term "nucleic acid construct" means a nucleic acid molecule, either single- or double-stranded, which is isolated from a naturally occurring gene or is modified to contain segments of nucleic acids in a manner that would not otherwise exist in nature or which is synthetic. The term nucleic acid construct is synonymous with the term "expression cassette" when the nucleic acid construct contains the control sequences required for expression of a coding sequence of the present invention.

**Control sequences:** The term "control sequences" means all components necessary for the expression of a polynucleotide encoding a polypeptide of the present invention. Each control sequence may be native or foreign to the polynucleotide encoding the polypeptide or native or foreign to each other. Such control sequences include, but are not limited to, a leader, polyadenylation sequence, propeptide sequence, promoter, signal peptide sequence, and transcription terminator. At a minimum, the control sequences include a promoter, and transcriptional and translational stop signals. The control sequences may be provided with linkers for the purpose of introducing specific restriction sites facilitating ligation of the control sequences with the coding region of the polynucleotide encoding a polypeptide.

**Operably linked:** The term "operably linked" means a configuration in which a control sequence is placed at an appropriate position relative to the coding sequence of a polynucleotide such that the control sequence directs the expression of the coding sequence.

**Expression:** The term "expression" includes any step involved in the production of the polypeptide including, but not limited to, transcription, post-transcriptional modification, translation, post-translational modification, and secretion.

**Expression vector:** The term "expression vector" means a linear or circular DNA molecule that comprises a polynucleotide encoding a polypeptide and is operably linked to additional nucleotides that provide for its expression.

**Host cell:** The term "host cell" or "host organism" means any cell type that is susceptible to transformation, transfection, transduction, and the like with a nucleic acid construct or expression vector comprising a polynucleotide of the present invention. The term "host cell" encompasses any progeny of a parent cell that is not identical to the parent cell due to mutations that occur during replication.

**Detailed Description of the Invention**

Peroxidases

EC-numbers may be used for classification of enzymes. Reference is made to the Recommendations of the Nomenclature Committee of the International Union of Biochemistry and Molecular Biology, Academic Press Inc., 1992.

It is to be understood that the term enzyme, as well as the various enzymes and enzyme classes mentioned herein, encompass wild-type enzymes, as well as any variant thereof that retains the activity in question. Such variants may be produced by recombinant techniques. The wild-type enzymes may also be produced by recombinant techniques, or by isolation and purification from the natural source.

In a particular embodiment the enzyme in question is well-defined, meaning that only one major enzyme component is present. This can be inferred e.g. by fractionation on an appropriate size-exclusion column. Such well-defined, or purified, or highly purified, enzyme can be obtained as is known in the art and/or described in publications relating to the specific enzyme in question.

A peroxidase according to the invention is a plant peroxidase enzyme comprised by the enzyme classification EC 1.11.1.7, or any fragment derived therefrom, exhibiting peroxidase activity. Plant peroxidases belong to class III peroxidases.

Class III peroxidases or the secreted plant peroxidases (EC 1.11.1.7) are found only in plants, where they form large multigenic families. Although their primary sequence differs in some points from the classes I and II, their three-dimensional structures are very similar to those of class II, and they also possess calcium ions, disulfide bonds, and an N-terminal signal for secretion.

Class III peroxidases are additionally able to undertake a second cyclic reaction, called hydroxylic, which is distinct from the peroxidative one. During the hydroxylic cycle, peroxidases pass through a Fe(II) state and use mainly the superoxide anion ($O_2^-$) to generate hydroxyl radicals (OH). Class III peroxidases, by using both these cycles, are known to participate in many different plant processes from germination to senescence, for example, auxin metabolism, cell wall elongation and stiffening, or protection against pathogens (see also Passardi et al. "The class III peroxidase multigenic family in rice and its evolution in land plants", Phytochemistry, 65(13), pp. 1879-93 (2004)).

The amino acid sequence of the peroxidase includes characteristic motifs of plant peroxidases. The peroxidase comprises the amino acid motifs:

HFHDCFV (SEQ ID NO:5);
GCD[A,G]S[V,I][I,L][I,L] (SEQ ID NO:69); and
VSC[A,S]D[I,L][I,L] (SEQ ID NO:70).

More preferably, the peroxidase comprises one, two or three amino acid motifs selected from the group consisting of:

HFHDCFV (SEQ ID NO:5);
GCD[A,G]S[V,I]LL (SEQ ID NO:6); and
VSC[A,S]D[I,L]L (SEQ ID NO:7).

Most preferably, the peroxidase comprises one, two or three amino acid motifs selected from the group consisting of:

HFHDCFV (SEQ ID NO:5);
GCD[A,G]S[V,I]L (SEQ ID NO:68); and
VSC[A,S]D[I,L]L (SEQ ID NO:7)

The peroxidase of the invention comprises an amino acid sequence which has at least 65% identity, such as at least 70% identity, at least 75% identity, at least 80% identity, at least 85% identity, at least 90% identity, or at least 95% identity to the amino acid sequence of SEQ ID NO:2, SEQ ID NO:4, amino acids 38 to 354 of SEQ ID NO: 45, amino acids 30 to 362 of SEQ ID NO: 55, or amino acids 23 to 324 of SEQ ID NO: 67.

In an embodiment, the peroxidase consists of an amino acid sequence which has at least 65% identity, such as at least 70% identity, at least 75% identity, at least 80% identity, at least 85% identity, at least 90% identity, or at least 95% identity to the amino acid sequence of SEQ ID NO:2, SEQ ID NO:4, amino acids 38 to 354 of SEQ ID NO: 45, amino acids 30 to 362 of SEQ ID NO: 55, or amino acids 23 to 324 of SEQ ID NO: 67.

In another embodiment, the peroxidase may be identical to, or have one or several amino acid differences as compared to, the amino acid sequence of SEQ ID NO:2, SEQ ID NO:4, amino acids 38 to 354 of SEQ ID NO: 45, amino acids 30 to 362 of SEQ ID NO: 55, or amino acids 23 to 324 of SEQ ID NO: 67; such as at the most 10 amino acid differences; or at the most 9, 8, 7, 6, 5, 4, 3, 2, or 1 amino acid difference(s), as compared to the amino acid sequence of SEQ ID NO:2, SEQ ID NO:4, amino acids 38 to 354 of SEQ ID NO: 45, amino acids 30 to 362 of SEQ ID NO: 55, or amino acids 23 to 324 of SEQ ID NO: 67.

Preferably, the peroxidase of the invention is a soybean peroxidase (e.g. SEQ ID NO:2) or is derived from a soybean peroxidase; or a royal palm tree peroxidase (e.g. SEQ ID NO:4) or is derived from a royal palm tree peroxidase; or a poplar peroxidase (e.g. amino acids 38 to 354 of SEQ ID NO: 45) or is derived from a poplar peroxidase; or a maize peroxidase (e.g. amino acids 30 to 362 of SEQ ID NO: 55) or is derived from a maize peroxidase; or a tobacco peroxidase (e.g. amino acids 23 to 324 of SEQ ID NO: 67) or is derived from a tobacco peroxidase.

Determination of Peroxidase Activity (POXU)

One peroxidase unit (POXU) is the amount of enzyme which catalyze the conversion of one $\mu$mole hydrogen peroxide per minute at 30°C in an aqueous solution of:

0.1 M phosphate buffer, pH 7.0;
0.88 mM hydrogen peroxide; and
1.67 mM 2,2'-azino-bis(3-ethylbenzothiazoline-6-sulfonate) (ABTS).

The reaction is continued for 60 seconds (15 seconds after mixing) while the change in absorbance at 418 nm is measured. The absorbance should be in the range of 0.15 to 0.30. Peroxidase activity is calculated using an absorption coefficient of oxidized ABTS of 36 mM$^{-1}$ cm$^{-1}$, and a stoichiometry of one $\mu$mole $H_2O_2$ converted per two $\mu$mole ABTS oxidized.

Methods and uses of the invention

Commonly, plant peroxidases are purified from plants, but this is a complex process with low yields. Alternatively, recombinant expression in bacteria or yeast can be used, but this often results in poor yields and/or difficult purification. The need for efficient recombinant production of plant derived peroxidases is thus apparent.

According to the present invention, wildtype plant peroxidases, and peroxidases derived therefrom, can be produced as recombinant protein in a filamentous fungal host cell, which often solves the problem of poor yields and/or difficult purification.

Recombinant expression of proteins is not always straight forward and it is hard to predict whether the desired product can in fact be produced in a particular production host organism and whether product yields will be sufficient for establishing an economical production.

Several parameters can lead to a lack of expression in filamentous fungal hosts. In general expression of a secreted and correctly processed peroxidase in a filamentous fungus involves a number of steps any of which could be a limiting step.

First the inserted peroxidase gene is transcribed to hnRNA. Then the hnRNA is transported from the nucleus to the cytosol, and during this process it is maturated to mRNA. Generally, a mRNA pool is established in the cytosol in order to sustain translation. The mRNA is then translated to a protein precursor, and this precursor is subsequently secreted to the endoplasmatic reticulum (ER) either co-translationally or post-translationally. Upon translocation into the ER the secretion signal peptide is cleaved of by a signal peptidase, and the resulting protein is folded in the ER. Secretion of the protein to the golgi apparatus follows when proper folding has been recognized by the cell. Here the propeptide will be cleaved to release the mature peroxidase. Thus numerous possibilities exist for preventing sufficient expression of a gene sequence in a given host organism.

In order to provide efficient expression of a polynucleotide sequence encoding a desired protein the translation process has to be efficient. One object of the present invention is therefore to optimize the mRNA sequence encoding the peroxidase protein in order to obtain sufficient expression in a filamentous fungal host cell.

In one embodiment, the present invention relates to a method for recombinant expression of a wild type plant peroxidase in a filamentous fungal host organism comprising expressing a modified nucleic acid sequence encoding a wild type plant peroxidase in a filamentous fungal host organism, wherein the modified nucleic acid sequence differs in at least one codon from the wild type nucleic acid sequence encoding the wild type plant peroxidase.

The modified nucleic acid sequence may be obtained by a) providing a wild type nucleic acid sequence encoding a wild type plant peroxidase and b) modifying at least one codon of said nucleic acid sequence so that the modified nucleic acid sequence differs in at least one codon from each wild type nucleic acid sequence encoding the wild type plant peroxidase. Methods for modifying nucleic acid sequences are well known to a person skilled in the art. In a particular embodiment said modification does not change the identity of the amino acid encoded by said codon.

Thus in another aspect the object of the present invention is provided by a method for recombinant expression of a wild type plant peroxidase in an *Aspergillus* host organism, comprising the steps:

i) providing a nucleic acid sequence encoding a class III peroxidase from EC 1.11.1.7, said nucleic acid sequence comprising at least one modified codon, wherein the modification does not change the amino acid encoded by said codon and the nucleic acid sequence of said codon is different compared to the corresponding codon in the nucleic acid sequence encoding the wild type gene;

ii) expressing the modified nucleic acid sequence in the *Aspergillus* host.

The starting nucleic acid sequence to be modified according to this embodiment is a wild type nucleic acid sequence encoding the plant peroxidase of interest.

Modifications according to the invention, comprises any modification of the base triplet and in a particular embodiment they comprise any modification which does not change the identity of the amino acid encoded by said codon, i.e. the amino acid encoded by the original codon and the modified codon is the same. In most cases the modification will be at the third position, however, in a few cases the modification may also be at the first or the second position. How to modify a codon also without modifying the resulting amino acid is known to the skilled person.

For both of the above embodiments, the number of codon which should differ or the number of modifications needed in order to obtain sufficient expression may vary. Thus according to a further embodiment of the invention, the modified nucleic acid sequence differs in at least 2 codons from each wild type nucleic acid sequence encoding said wild type plant peroxidase or at least 2 codons have been modified, particularly at least 3 codons, more particularly at least 5 codons, more particularly at least 10 codons, more particularly at least 15 codons, even more particularly at least 25 codons.

It has furthermore been found, that by changing the codon usage of the wild type nucleic acid sequence to be selected among the codons preferably used by the filamentous fungus used as a host, the expression of a peroxidase of the invention is now possible. Such codons are said to be "optimized" for expression.

Due to the degeneracy of the genetic code and the preference of certain preferred codons in particular organisms/cells the expression level of a protein in a given host cell can in some instances be improved by optimizing the codon usage. In the present case, the yields of plant peroxidase were excellent when the wild type nucleic acid sequences encoding SEQ ID NO: 2, SEQ ID NO: 4, amino acids 38 to 354 of SEQ ID NO: 45, amino acids 30 to 362 of SEQ ID NO: 55, and amino acids 23 to 324 of SEQ ID NO: 67 were optimized by codon optimization and expressed in *Aspergillus.*

In the present invention "codon optimized" means that due to the degeneracy of the genetic code more than one triplet codon can be used for each amino acid. Some codons will be preferred in a particular organism and by changing the codon usage in a wild type gene to a codon usage preferred in a particular expression host organism the codons are said to be optimized. Codon optimization can be performed e.g. as described in Gustafsson et al., 2004, (Trends in Biotechnology vol. 22 (7); Codon bias and heterologous protein expression), and US 6,818,752.

Codon optimization may be based on the average codon usage for the host organism or it can be based on the codon usage for a particular gene which is known to be expressed in high amounts in a particular host cell.

In one embodiment of the invention the peroxidase protein is encoded by a modified nucleic acid sequence codon optimized in at least 10 % of the codons, more particularly at least 20%, or at least 30%, or at least 40%, or particularly at least 50%, more particularly at least 60%, and more particularly at least 75%. Thus the modified nucleic acid sequence may differ in at least 10% of the codons from each wild type nucleic acid sequence encoding said wild type peroxidase, more particularly in at least 20%, or in at least 30%, or in at least 40%, or particularly in at least 50%, more particularly in at least 60%, and more particularly in at least 75%. In particular said codons may differ because they have been codon optimized as compared with a wild type nucleic acid sequence encoding a wild type plant peroxidase.

Particularly 100% of the nucleic acid sequence has been codon optimized to match the preferred codons used in filamentous fungi.

In a particular embodiment the codon optimization is based on the codon usage of alpha amylase from *Aspergillus oryzae*, also known as Fungamyl™ (WO 2005/019443; SEQ ID NO: 2), which is a protein known to be expressed in high levels in filamentous fungi. In the present context an expression level corresponding to at least 20%, preferably at least 30%, more preferably at least 40%, even more preferably at least 50%, of the total amount of secreted protein constitutes the protein of interest is considered a high level of expression.

In a particular embodiment, the modified nucleic acid sequence encoding a mature plant peroxidase is selected from the group consisting of SEQ ID NO: 1, SEQ ID NO: 3, amino acids 118 to 1068 of SEQ ID NO: 44, amino acids 94 to 1092 of SEQ ID NO: 54, or amino acids 67 to 972 of SEQ ID NO: 66.

In practice the optimization according to the invention comprises the steps:

i) the nucleic acid sequence encoding the peroxidase of the invention is codon optimized as explained in more detail below;
ii) check the resulting modified sequence for a balanced GC-content (approximately 45-55%); and
iii) check or edit the resulting modified sequence from step ii) as explained below.

Codon optimization protocol:

The codon usage of a single gene, a number of genes or a whole genome can be calculated with the program cusp from the EMBOSS-package (http://www.rfcgr.mrc.ac.uk/Software/EMBOSS/).

The starting point for the optimization is the amino acid sequence of the protein or a nucleic acid sequence coding for the protein together with a codon-table. By a codon-optimized gene, we understand a nucleic acid sequence, encoding a given protein sequence and with the codon statistics given by a codon table.

The codon statistics referred to is a column in the codon-table called "Fract" in the output from cusp-program and which describes the fraction of a given codon among the other synonymous codons. We call this the local score. If for instance 80% of the codons coding for F is TTC and 20% of the codons coding for F are TTT, then the codon TTC has a local score of 0.8 and TTT has a local score of 0.2.

The codons in the codon table are re-ordered by first encoding amino acid (e.g. alphabetically) and then increasingly by the score. In the example above, ordering the codons for F as TTT, TTC. Cumulated scores for the codons are then generated by adding the scores in order. In the example above TTT has a cumulated score of 0.2 and TTC has a cumulated score of 1. The most used codon will always have a cumulated score of 1.

In order to generate a codon optimized gene the following is performed. For each position in the amino acid sequence, a random number between 0 and 1 is generated. This is done by the random-number generator on the computer system on which the program runs. The first codon is chosen as the codon with a cumulated score greater than or equal to the generated random number. If, in the example above, a particular position in the gene is "F" and the random number generator gives 0.5, TTC is chosen as codon.

The strategy for avoiding introns is to make sure that there are no branch points. This was done by making sure that the consensus sequence for branch-point in *Aspergillus oryzae*: CT[AG]A[CT] was not present in the sequence. The sequence [AG]CT[AG]A[AG] may be recognised as a branch point in introns. Thus in a particular embodiment of the present invention such sequences may also be modified or be removed according to a method of the present invention. This was done in a post processing step, where the sequence was scanned for the presence of this motif, and each occurrence was removed by changing codons in the motif to synonymous codons, choosing codons with the best local score first.

A codon table showing the codon usage of the alpha amylase from *Aspergillus oryzae* is given below.

Table 1. Codon usage for the *Aspergillus oryzae* alpha amylase (CUSP codon usage file)

| Codon | Amino acid | Fract | /1000 | Number |
|-------|-----------|-------|-------|--------|
| GCA | A | 0.286 | 24.000 | 12 |
| GCC | A | 0.357 | 30.000 | 15 |
| GCG | A | 0.238 | 20.000 | 10 |
| GCT | A | 0.119 | 10.000 | 5 |
| TGC | C | 0.222 | 4.000 | 2 |
| TGT | C | 0.778 | 14.000 | 7 |
| GAC | D | 0.524 | 44.000 | 22 |
| GAT | D | 0.476 | 40.000 | 20 |
| GAA | E | 0.417 | 10.000 | 5 |
| GAG | E | 0.583 | 14.000 | 7 |
| TTC | F | 0.800 | 24.000 | 12 |
| TTT | F | 0.200 | 6.000 | 3 |
| GGA | G | 0.233 | 20.000 | 10 |
| GGC | G | 0.419 | 36.000 | 18 |
| GGG | G | 0.116 | 10.000 | 5 |
| GGT | G | 0.233 | 20.000 | 10 |
| CAC | H | 0.571 | 8.000 | 4 |
| CAT | H | 0.429 | 6.000 | 3 |
| ATA | I | 0.071 | 4.000 | 2 |
| ATC | I | 0.679 | 38.000 | 19 |

(continued)

| Codon | Amino acid | Fract | /1000 | Number |
|-------|-----------|-------|--------|--------|
| ATT | I | 0.250 | 14.000 | 7 |
| AAA | K | 0.350 | 14.000 | 7 |
| AAG | K | 0.650 | 26.000 | 13 |
| CTA | L | 0.081 | 6.000 | 3 |
| CTC | L | 0.351 | 26.000 | 13 |
| CTG | L | 0.162 | 12.000 | 6 |
| CTT | L | 0.108 | 8.000 | 4 |
| TTA | L | 0.027 | 2.000 | 1 |
| TTG | L | 0.270 | 20.000 | 10 |
| ATG | M | 1.000 | 22.000 | 11 |
| AAC | N | 0.885 | 46.000 | 23 |
| AAT | N | 0.115 | 6.000 | 3 |
| CCA | P | 0.136 | 6.000 | 3 |
| CCC | P | 0.364 | 16.000 | 8 |
| CCG | P | 0.227 | 10.000 | 5 |
| CCT | P | 0.273 | 12.000 | 6 |
| CAA | Q | 0.250 | 10.000 | 5 |
| CAG | Q | 0.750 | 30.000 | 15 |
| AGA | R | 0.000 | 0.000 | 0 |
| AGG | R | 0.300 | 6.000 | 3 |
| CGA | R | 0.200 | 4.000 | 2 |
| CGC | R | 0.200 | 4.000 | 2 |
| CGG | R | 0.200 | 4.000 | 2 |
| CGT | R | 0.100 | 2.000 | 1 |
| AGC | S | 0.162 | 12.000 | 6 |
| AGT | S | 0.108 | 8.000 | 4 |
| TCA | S | 0.108 | 8.000 | 4 |
| TCC | S | 0.243 | 18.000 | 9 |
| TCG | S | 0.270 | 20.000 | 10 |
| TCT | S | 0.108 | 8.000 | 4 |
| ACA | T | 0.250 | 20.000 | 10 |
| ACC | T | 0.325 | 26.000 | 13 |
| ACG | T | 0.200 | 16.000 | 8 |
| ACT | T | 0.225 | 18.000 | 9 |
| GTA | V | 0.129 | 8.000 | 4 |
| GTC | V | 0.387 | 24.000 | 12 |
| GTG | V | 0.323 | 20.000 | 10 |
| GTT | V | 0.161 | 10.000 | 5 |
| TGG | W | 1.000 | 24.000 | 12 |
| TAC | Y | 0.686 | 48.000 | 24 |
| TAT | Y | 0.314 | 22.000 | 11 |
| TAA | * | 0.000 | 0.000 | 0 |
| TAG | * | 0.000 | 0.000 | 0 |
| TGA | * | 1.000 | 2.000 | 1 |

Introns

Eukaryotic genes may be interrupted by intervening sequences (introns) which must be modified in precursor transcripts in order to produce functional mRNAs. This process of intron removal is known as pre-mRNA splicing. Usually, a branchpoint sequence of an intron is necessary for intron splicing through the formation of a lariat. Signals for splicing reside directly at the boundaries of the intron splice sites. The boundaries of intron splice sites usually have the consensus intron sequences GT and AG at their 5' and 3' extremities, respectively. While no 3' splice sites other than AG have been reported, there are reports of a few exceptions to the 5' GT splice site. For example, there are precedents where CT or GC is substituted for GT at the 5' boundary. There is also a strong preference for the nucleotide bases ANGT to follow GT where N is A, C, G, or T (primarily A or T in *Saccharomyces* species), but there is no marked preference for any particular nucleotides to precede the GT splice site. The 3' splice site AG is primarily preceded by a pyrimidine nucleotide base (Py), i.e., C or T.

The number of introns that can interrupt a fungal gene ranges from one to twelve or more introns (Rymond and Rosbash, 1992, In, E.W. Jones, J.R. Pringle, and J.R. Broach, editors, The Molecular and Cellular Biology of the Yeast Saccharomyces, pages 143-192, Cold Spring Harbor Laboratory Press, Plainview, New York; Gurr et al., 1987, In Kinghorn, J.R. (ed.), Gene Structure in Eukaryotic Microbes, pages 93-139, IRL Press, Oxford). They may be distributed throughout a gene or situated towards the 5' or 3' end of a gene. In *Saccharomyces cerevisiae*, introns are located primarily at the 5' end of the gene. Introns may be generally less than 1 kb in size, and usually are less than 400 bp in size in yeast and less than 100 bp in filamentous fungi.

The *Saccharomyces cerevisiae* intron branchpoint sequence 5'-TACTAAC-3' rarely appears in filamentous fungal introns (Gurr *et al*., 1987, *supra).* Sequence stretches closely or loosely resembling TACTAAC are seen at equivalent points in filamentous fungal introns with a general consensus NRCTRAC where N is A, C, G, or T, and R is A or G. For example, the fourth position T is invariant in both the *Neurospora crassa* and *Aspergillus nidulans* putative consensus sequences. Furthermore, nucleotides G, A, and C predominate in over 80% of the positions 3, 6, and 7, respectively, although position 7 in *Aspergillus nidulans* is more flexible with only 65% C. However, positions 1, 2, 5, and 8 are much less strict in both *Neurospora crassa* and *Aspergillus nidulans*. Other filamentous fungi have similar branchpoint stretches at equivalent positions in their introns, but the sampling is too small to discern any definite trends.

The heterologous expression of a gene encoding a polypeptide in a fungal host strain may result in the host strain incorrectly recognizing a region within the coding sequence of the gene as an intervening sequence or intron. For example, it has been found that intron-containing genes of filamentous fungi are incorrectly spliced in *Saccharomyces cerevisiae* (Gurr et al., 1987, In Kinghorn, J.R. (ed.), Gene Structure in Eukaryotic Microbes, pages 93-139, IRL Press, Oxford). Since the region is not recognized as an intron by the parent strain from which the gene was obtained, the intron is called a cryptic intron. This improper recognition of an intron, referred to herein as a cryptic intron, may lead to aberrant splicing of the precursor mRNA molecules resulting in no production of biologically active polypeptide or in the production of several populations of polypeptide products with varying biological activity.

"Cryptic intron" is defined herein as a region of a coding sequence that is incorrectly recognized as an intron which is excised from the primary mRNA transcript. A cryptic intron preferably has 10 to 1500 nucleotides, more preferably 20 to 1000 nucleotides, even more preferably 30 to 300 nucleotides, and most preferably 30 to 100 nucleotides.

The presence of cryptic introns can in particular be a problem when trying to express proteins in organisms which have a less strict requirement to what sequences are necessary in order to define an intron. Such "sloppy" recognition can result e.g. when trying to express recombinant proteins in fungal expression systems.

Cryptic introns can be identified by the use of Reverse Transcription Polymerase Chain Reaction (RT-PCR). In RT_PCR, mRNA is reverse transcribed into single stranded cDNA that can be PCR amplified to double stranded cDNA. PCR primers can then be designed to amplify parts of the single stranded or double stranded cDNA, and sequence analysis of the resulting PCR products compared to the sequence of the genomic DNA reveals the presence and exact location of cryptic introns (T. Kumazaki et al. (1999) J. Cell. Sci. 112, 1449 - 1453).

According to one embodiment of the invention the modification introduced into the wild type gene sequence will optimize the mRNA for expression in a particular host organism. In the present invention the host organism or host cell is an *Aspergillus* host cell.

Host organism

The host organism (host cell) of the invention is an *Aspergillus* cell. In a preferred embodiment, the host cell is an *Aspergillus awamori, Aspergillus foetidus, Aspergillus japonicus*, *Aspergillus aculeatus, Aspergillus niger, Aspergillus nidulans* or *Aspergillus oryzae* cell.

In a particular embodiment the host cell is an *A. oryzae* or *A. niger* cell.

In a preferred embodiment of the invention the host cell is a protease deficient or protease minus strain.

This may e.g. be the protease deficient strain *Aspergillus oryzae* JaL 125 having the alkaline protease gene named "*alp*" deleted. This strain is described in WO 97/35956 (Novozymes), or EP patent no. 429,490, or the TPAP free host cell, in particular a strain of *A. niger,* disclosed in WO 96/14404. Further, also host cell, especially *A. niger* or *A. oryzae,* with reduced production of the transcriptional activator (prtT) as described in WO 01/68864 is specifically contemplated according to the invention.

Transformation of fungi

Fungal cells may be transformed by a process involving protoplast formation, transformation of the protoplasts, and regeneration of the cell wall in a manner known *per se.* Suitable procedures for transformation of *Aspergillus* host cells are described in EP 238 023 and Yelton et al., 1984, Proceedings of the National Academy of Sciences USA 81: 1470-1474. Suitable methods for transforming *Fusarium* species are described by Malardier et al., 1989, Gene 78: 147-156 and WO 96/00787. Yeast may be transformed using the procedures described by Becker and Guarente, In Abelson, J.N. and Simon, M.I., editors, Guide to Yeast Genetics and Molecular Biology, Methods in Enzymology, Volume 194, pp 182-187, Academic Press, Inc., New York; Ito et al., 1983, Journal of Bacteriology 153: 163; and Hinnen et al., 1978, Proceedings of the National Academy of Sciences USA 75: 1920.

Methods of Production

The present invention also relates to expression of the modified nucleic acid sequence in order to produce the peroxidase described in the invention. Expression comprises (a) cultivating a filamentous fungus expressing the peroxidase from the modified nucleic acid sequence; and (b) recovering the peroxidase. The filamentous fungus is of the genus *Aspergillus,* and more preferably *Aspergillus oryzae* or *Aspergillus niger.*

In the production methods of the present invention, the cells are cultivated in a nutrient medium suitable for production of the polypeptide using methods known in the art. For example, the cell may be cultivated by shake flask cultivation, small-scale or large-scale fermentation (including continuous, batch, fed-batch, or solid state fermentations) in laboratory or industrial fermentors performed in a suitable medium and under conditions allowing the polypeptide to be expressed and/or isolated. The cultivation takes place in a suitable nutrient medium comprising carbon and nitrogen sources and inorganic salts, using procedures known in the art. Suitable media are available from commercial suppliers or may be prepared according to published compositions (*e.g.*, in catalogues of the American Type Culture Collection). If the polypeptide is secreted into the nutrient medium, the polypeptide can be recovered directly from the medium. If the polypeptide is not secreted, it can be recovered from cell lysates.

The polypeptides may be detected using methods known in the art that are specific for the polypeptides, such as N-terminal sequencing of the polypeptide. These detection methods may include use of specific antibodies. The resulting polypeptide may be recovered by methods known in the art. For example, the polypeptide may be recovered from the nutrient medium by conventional procedures including, but not limited to, centrifugation, filtration, extraction, spray-drying, evaporation, or precipitation.

The polypeptides of the present invention may be purified by a variety of procedures known in the art including, but not limited to, chromatography (*e.g.*, ion exchange, affinity, hydrophobic, chromatofocusing, and size exclusion), electrophoretic procedures (*e.g.*, preparative isoelectric focusing), differential solubility (*e.g.*, ammonium sulfate precipitation), SDS-PAGE, or extraction (see, *e.g.,* Protein Purification, J.-C. Janson and Lars Ryden, editors, VCH Publishers, New York, 1989).

In a further aspect the present invention discloses a modified nucleic acid sequence encoding a wildtype plant peroxidase, such as soy bean peroxidase (e.g. SEQ ID NO:2), royal palm tree peroxidase (e.g. SEQ ID NO:4), poplar peroxidase (e.g. amino acids 38 to 354 of SEQ ID NO: 45), maize peroxidase (e.g. amino acids 30 to 362 of SEQ ID NO: 55), or tobacco peroxidase (e.g. amino acids 23 to 324 of SEQ ID NO: 67), and capable of expression in a filamentous fungal host organism, which modified nucleic acid sequence is obtainable by:

  i) providing the wild type nucleic acid sequence encoding the peroxidase;
  ii) modifying at least one codon, wherein the modification does not change the amino acid encoded by said codon and the nucleic acid sequence of said codon is different compared to the corresponding codon in the wild type gene.

In the present context the term "capable of expression in a filamentous host" means that the yield of the peroxidase protein should be at least 1.5 mg/l, more particularly at least 2.5 mg/l, more particularly at least 5 mg/l, more particularly at least 10 mg/l, even more particularly at least 20 mg/l, or more particularly 0.5 g/L, or more particularly 1 g/L, or more particularly 5 g/L, or more particularly 10 g/L, or more particularly 20 g/L.

Specific examples of modified nucleic acid sequences encoding a peroxidase as described in the invention and modified as described in the invention in order to provide expression of the peroxidase protein in an *Aspergillus* host are shown in SEQ ID NO: 1 (soy bean peroxidase), SEQ ID NO: 3 (royal palm tree peroxidase), amino acids 118 to 1068 of SEQ ID NO: 44 (poplar peroxidase), amino acids 94 to 1092 of SEQ ID NO: 54 (maize peroxidase), and amino acids 67 to 972 of SEQ ID NO: 66 (tobacco peroxidase). The information disclosed herein will allow the skilled person to isolate other modified nucleic acid sequences following the directions above, which sequences can also be expressed in *Aspergillus* and such sequences are also comprised within the scope of the present invention.

Methods and compositions

In a first aspect, the present invention provides a method for recombinant expression of a plant peroxidase, comprising expressing in an *Aspergillus* host organism a nucleic acid sequence encoding a class III peroxidase from EC 1.11.1.7, wherein the amino acid sequence of the peroxidase comprises the amino acid motifs:

HFHDCFV;
GCD[A,G]S[V,I][I,L][I,L]; and
VSC[A,S]D[I,L][I,L].

Preferably, the motifs are selected from the group consisting of:

HFHDCFV;
GCD[A,G]S[V,I]LL; and
VSC[A,S]D[I,L]L.

In another embodiment, the amino acid sequence of the peroxidase has at least 65% identity, preferably at least 70% identity, at least 75% identity, at least 80% identity, at least 85% identity, at least 90% identity, or at least 95% identity, to the amino acid sequence of SEQ ID NO:2, SEQ ID NO:4, amino acids 38 to 354 of SEQ ID NO: 45, amino acids 30 to 362 of SEQ ID NO: 55, or amino acids 23 to 324 of SEQ ID NO: 67.

In another embodiment, the peroxidase consists of the amino acid sequence of SEQ ID NO:2, SEQ ID NO:4, amino acids 38 to 354 of SEQ ID NO: 45, amino acids 30 to 362 of SEQ ID NO: 55, or amino acids 23 to 324 of SEQ ID NO: 67.

The nucleic acid sequence may be attached to suitable control sequence(s) that provide for expression of the peroxidase.

In another embodiment, at least one codon of the nucleic acid sequence is optimized for translation in a filamentous fungal host organism. Preferably, at least half of the codons of the nucleic acid sequence are optimized for translation in a filamentous fungal host organism. More preferably, the nucleic acid sequence is codon optimized in at least 10% of the codons, preferably at least 20% of the codons, more preferably at least 30% of the codons, more preferably at least 50% of the codons, and most preferably at least 75% of the codons. Most preferably, the optimized codon(s) corresponds to the codon usage of alpha amylase from *Aspergillus oryzae*.

In another embodiment, the *Aspergillus* host organism is *Aspergillus awamori, Aspergillus foetidus, Aspergillus japonicus, Aspergillus niger, Aspergillus nidulans,* or *Aspergillus oryzae*.

Most preferably, the filamentous fungal host organism is *Aspergillus oryzae* or *Aspergillus niger*.

In a second aspect, the present invention provides an *Aspergillus* host organism comprising a nucleic acid sequence encoding a class III peroxidase from EC 1.11.1.7, capable of expressing at least 20 mg/L of peroxidase, wherein the amino acid sequence of the peroxidase comprises the amino acid motifs:

HFHDCFV;
GCD[A,G]S[V,I][I,L][I,L]; and
VSC[A,S]D[I,L][I,L].

Embodiments of the *Aspergillus* host organism, the nucleic acid sequence, and the peroxidase, are the same as the embodiments mentioned above in the first aspect of the invention.

The present invention is further described by the following examples that should not be construed as limiting the scope of the invention.

## EXAMPLES

Plasmid pENI2516 was described in WO 2004/069872, Example 2.

*Aspergillus oryzae* strain ToC1512 was described in WO 2005/070962, Example 11.

Primer 1:

5'-TCCTGACCTAGGACAGCTCACACCCACTTTC-3' (SEQ ID NO: 36)

Primer 2:

5'-ACAGGTCTTAAGTCATTTGGACTGGGCGACG-3' (SEQ ID NO: 37)

Primer 3:

5'-TGCCCGCCTAGGAGACCTCCAGATTGGATTCTATAAC-3' (SEQ ID NO: 38)

Primer 4:

5'-ATCATA CTTAAG TTATCAGGAGTTGACCACGGAACAG-3' (SEQ ID NO: 39)

Primer 5:

5'-TAATCCTAGGTCAGCTCACACCTACCTTCTAC-3' (SEQ ID NO: 40)

Primer 6:

5'-GGTACCCTTAAGTCAAATCGAC-3' (SEQ ID NO: 41)

Primer 7:

5'-TAATCCTAGGTGCCGGTCTCAAAGTGGGATTCTAC-3' (SEQ ID NO: 42)

Primer 8:

5'-ATTACTTAAGTCAGTTGGTTGCCACGTG-3' (SEQ ID NO: 43)

**EXAMPLE 1**

Cloning and expression of soybean peroxidase

A DNA sequence was designed to encode the amino acid sequence of soybean peroxidase (SEQ ID NO:2) using codon optimization as described above. The gene was specifically designed for expression in *Aspergillus oryzae*, and a restriction site was added at either end to ease cloning. The DNA was subsequently synthezised by a commercial provider.

The synthetic gene encoding the peroxidase was ligated into the multiple cloning site of plasmid pENI2516 as a BamHI-AflIII fragment to generate construct SEQ ID NO:8 using standard technologies of molecular biology. This construct was used as template in a PCR reaction with Primer 1 and Primer 2 resulting in a fragment with approximate size 1095 bp. The PCR product contains restriction sites at either end which allows ligation of an AvrII-AflII fragment into existing plasmids to generate constructs SEQ ID NOs: 10, 12, 14, 16, 18 and 20. These constructs contain different secretion signal and prepro sequences known to work well in *Aspergillus oryzae*. All constructed plasmids were initially transformed into *E. coli* strain Top10 and the inserts were sequenced to confirm nucleotide sequences. The plasmid was subsequently transformed into *Aspergillus oryzae* strain ToC1512 for expression trials.

The transformed strain of *A. oryzae* was grown for expression of peroxidase enzyme. Typically, 200 $\mu$L of YP growth medium was inoculated with spores from strains grown on sucrose agar added 10 mM NaNO$_3$. The cultures were grown in a 96 well sterile microplate for 3-4 days at 34°C without shaking. Expression of peroxidase was confirmed by presence of a band with the correct molecular weight on SDS-PAGE and by ability to bleach indigo carmine in presence of 10-phenothiazinepropionic acid (PPT):

    100 $\mu$L 100 mM Britton-Robinson buffer pH 6
    2 $\mu$L 10 mM indigo carmine
    4 $\mu$L 10 mM PPT (in 96% ethanol)
    2 $\mu$L 0.3% hydrogen peroxidase
    10 $\mu$L supernatant of fermentation broth

The enzymatic activity was monitored by change in absorbance at 610 nm for 10 minutes. The identity of the expressed the peroxidase was confirmed by mass-spectroscopic analysis of fragments from a tryptic in-gel digest.

All constructs resulted in expression of at least about 0.5 g/l of active soybean peroxidase.

**EXAMPLE 2**

Cloning and expression of Royal palm tree peroxidase

The amino acid sequence of Royal palm tree peroxidase (SEQ ID NO:4) is publicly available (Uniprot D1MPT2), but there is no information about the native secretion signal. The amino acids encoded in secretion signal of the soybean peroxidase were therefore fused to the N-terminal of the mature amino acid sequence of the royal palm tree peroxidase. A DNA sequence was designed to encode this amino acid sequence using codon optimization, as described above, for expression in *Aspergillus oryzae*. A suitable restriction site was added at either end to ease cloning and the DNA was synthezised by a commercial provider.

The synthetic gene encoding the peroxidase was ligated into the multiple cloning site of plasmid pENI2516 as a BamHI-AflII fragment to generate construct SEQ ID NO: 34 using standard technologies of molecular biology. This construct was used as template in a PCR reaction with Primer 3 and Primer 4 resulting in a fragment with approximate size 1029 bp. The PCR product contains restriction sites at either end which allows ligation of an AvrII-AflII fragment into existing plasmids to generate constructs SEQ ID NOs: 22, 24, 26, 28, 30 and 32. These constructs contain different secretion signal and prepro sequences known to work well in *Aspergillus oryzae*. All constructed plasmids were initially transformed into *E. coli* strain TOP10 and the inserts were sequenced to confirm nucleotide sequences. The plasmid was subsequently transformed into *Aspergillus oryzae* strain ToC1512 for expression trials.

The transformed strain of *A. oryzae* was grown for expression of peroxidase enzyme. Typically, 200 $\mu$L of YP growth medium was inoculated with spores from strains grown on sucrose agar added 10 mM $NaNO_3$. The cultures were grown in a 96 well sterile microplate for 3-4 days at 34°C without shaking. Expression of peroxidase was confirmed by presence of a band with the correct molecular weight on SDS-PAGE and by activity on ABTS:

20 $\mu$L 10 mM ABTS
20 $\mu$L 0.3% hydrogen peroxidase
140 $\mu$L 100 mM Britton-Robinson buffer pH 3
10 $\mu$L Supernatant of fermentation broth

The enzymatic activity was monitored by change in absorbance at 405 nm for 5 minutes. The identity of the expressed the peroxidase was confirmed by mass-spectroscopic analysis of fragments from a tryptic in-gel digest.

All constructs resulted in expression of at least about 0.5 g/l of active royal palm tree peroxidase.

**EXAMPLE 3**

Cloning and expression of poplar peroxidase

A DNA sequence was designed to encode the amino acid sequence of poplar peroxidase (mature peroxidise is amino acids 38 to 354 of SEQ ID NO: 45) using codon optimization as described above. The gene was specifically designed for expression in *Aspergillus oryzae* and a restriction site was added at either end to ease cloning. The DNA was subsequently synthezised by a commercial provider.

The synthetic gene encoding the peroxidase was ligated into the multiple cloning site of plasmid pENI2516 as a BamHI-AflIII fragment to generate construct SEQ ID NO: 44 using standard technologies of molecular biology. This construct was used as template in a PCR reaction with Primer 5 and Primer 6 resulting in a fragment with approximate size 977 bp. The PCR product contains restriction sites at either end which allows ligation of an AvrII-AflIII fragment into existing plasmids to generate constructs SEQ ID NOs: 46, 48, 50, and 52. These constructs contain different secretion signal and prepro sequences known to work well in *Aspergillus oryzae*. All constructed plasmids were initially transformed into *E. coli* strain Top10 and the inserts were sequenced to confirm nucleotide sequences. The plasmid was subsequently transformed into *Aspergillus oryzae* strain ToC1512 for expression trials.

The transformed strain of *A. oryzae* was grown for expression of peroxidase enzyme. Typically, 200 $\mu$L of YP growth medium was inoculated with spores from strains grown on sucrose agar added 10 mM $NaNO_3$. The cultures were grown in a 96 well sterile microplate for 3-4 days at 34°C without shaking. Expression of peroxidase was confirmed by presence of a band with the correct molecular weight on SDS-PAGE and by activity on ABTS:

20 $\mu$L 10 mM ABTS
20 $\mu$L 0.3% hydrogen peroxide
140 $\mu$L 100 mM Britton-Robinson buffer pH 3

10 μL Supernatant of fermentation broth

The enzymatic activity was monitored by change in absorbance at 405 nm for 5 minutes. All constructs resulted in expression of at least about 0.5 g/l of active poplar peroxidase.

**EXAMPLE 4**

Cloning and expression of maize peroxidase

A DNA sequence was designed to encode the amino acid sequence of maize peroxidase (mature peroxidase is amino acids 30 to 362 of SEQ ID NO: 55) using codon optimization as described above. The gene was specifically designed for expression in *Aspergillus oryzae* and a restriction site was added at either end to ease cloning. The DNA was subsequently synthezised by a commercial provider.

The synthetic gene encoding the peroxidase was ligated into the multiple cloning site of plasmid pENI2516 as a BamHI-AflIII fragment to generate construct SEQ ID NO: 54 using standard technologies of molecular biology. This construct was used as template in a PCR reaction with Primer 7 and Primer 8 resulting in a fragment with approximate size 1023 bp. The PCR product contains restriction sites at either end which allows ligation of an AvrII-AflIII fragment into existing plasmids to generate constructs SEQ ID NOs: 56, 58, 60, 62, and 64. These constructs contain different secretion signal and prepro sequences known to work well in *Aspergillus oryzae*. All constructed plasmids were initially transformed into *E. coli* strain Top10 and the inserts were sequenced to confirm nucleotide sequences. The plasmid was subsequently transformed into *Aspergillus oryzae* strain ToC1512 for expression trials.

The transformed strain of *A. oryzae* was grown for expression of peroxidase enzyme. Typically, 200 μL of YP growth medium was inoculated with spores from strains grown on sucrose agar added 10 mM $NaNO_3$. The cultures were grown in a 96 well sterile microplate for 3-4 days at 34°C without shaking. Expression of peroxidase was confirmed by presence of a band with the correct molecular weight on SDS-PAGE and by activity on ABTS:

20 μL 10 mM ABTS
20 μL 0.3% hydrogen peroxidase
140 μL 100 mM Britton-Robinson buffer pH 3
10 μL Supernatant of fermentation broth

The enzymatic activity was monitored by change in absorbance at 405 nm for 5 minutes. All constructs resulted in expression of at least about 0.5 g/l of active maize peroxidase.

**EXAMPLE 5**

Cloning and expression of tobacco peroxidase

A DNA sequence was designed to encode the amino acid sequence of tobacco peroxidase (mature peroxidase is amino acids 23 to 324 of SEQ ID NO: 67) using codon optimization as described above. The gene was specifically designed for expression in *Aspergillus oryzae* and a restriction site was added at either end to ease cloning. The DNA was subsequently synthezised by a commercial provider.

The synthetic gene encoding the peroxidase was ligated into the multiple cloning site of plasmid pENI2516 as a BamHI-AflIII fragment to generate construct SEQ ID NO: 66 using standard technologies of molecular biology. The constructed plasmid was initially transformed into *E. coli* strain Top10 and the insert was sequenced to confirm nucleotide sequence. The plasmid was subsequently transformed into *Aspergillus oryzae* strain ToC1512 for expression trials.

The transformed strain of *A. oryzae* was grown for expression of peroxidase enzyme. Typically, 200 μL of YP growth medium was inoculated with spores from the strain grown on sucrose agar added 10 mM $NaNO_3$. The cultures were grown in a 96 well sterile microplate for 3-4 days at 34°C without shaking. Expression of peroxidase was confirmed by presence of a band with the correct molecular weight on SDS-PAGE and by activity on ABTS:

20 μL 10 mM ABTS
20 μL 0.3% hydrogen peroxide
140 μL 100 mM Britton-Robinson buffer pH 3
10 μL Supernatant of fermentation broth

The enzymatic activity was monitored by change in absorbance at 405 nm for 5 minutes. The construct resulted in expression of at least about 0.5 g/l of active tobacco peroxidase.

SEQUENCE LISTING

<110> Novozymes A/S

<120> Expression of Plant Peroxidases in Filamentous Fungi

<130> 11776-WO-PCT

<160> 70

<170> PatentIn version 3.5

<210> 1
<211> 978
<212> DNA
<213> Glycine max

<220>
<221> CDS
<222> (1)..(978)

<400> 1

```
cag ctc aca ccc act ttc tac agg gaa acc tgt ccc aac ttg ttc ccc      48
Gln Leu Thr Pro Thr Phe Tyr Arg Glu Thr Cys Pro Asn Leu Phe Pro
1               5                   10                  15

att gtg ttc ggc gtc atc ttc gat gcg tcg ttc acc gac ccc agg atc      96
Ile Val Phe Gly Val Ile Phe Asp Ala Ser Phe Thr Asp Pro Arg Ile
                20                  25                  30

gga gcc tcg ctc atg cgc ctc cat ttc cac gac tgt ttc gtc cag ggc     144
Gly Ala Ser Leu Met Arg Leu His Phe His Asp Cys Phe Val Gln Gly
            35                  40                  45

tgt gac ggt tcc gtc ttg ttg aac aac acc gac acc atc gag tcc gag     192
Cys Asp Gly Ser Val Leu Leu Asn Asn Thr Asp Thr Ile Glu Ser Glu
        50                  55                  60

cag gac gcg ctc ccc aac atc aac tcc atc cga ggc ctc gat gtc gtg     240
Gln Asp Ala Leu Pro Asn Ile Asn Ser Ile Arg Gly Leu Asp Val Val
65                  70                  75                  80

aac gac atc aaa acc gca gtg gaa aac tcc tgt ccc gat acg gtc tcc     288
Asn Asp Ile Lys Thr Ala Val Glu Asn Ser Cys Pro Asp Thr Val Ser
                85                  90                  95

tgt gca gac atc ttg gcg att gca gcc gag atc gca tcg gtc ctc gga     336
Cys Ala Asp Ile Leu Ala Ile Ala Ala Glu Ile Ala Ser Val Leu Gly
            100                 105                 110

ggc ggt cct ggc tgg cct gtg ccg ctc gga cga cgg gac tcg ttg aca     384
Gly Gly Pro Gly Trp Pro Val Pro Leu Gly Arg Arg Asp Ser Leu Thr
            115                 120                 125

gca aac agg acg ctc gca aac cag aac ttg cct gcg cct ttc ttc aac     432
Ala Asn Arg Thr Leu Ala Asn Gln Asn Leu Pro Ala Pro Phe Phe Asn
            130                 135                 140

ctc acc cag ttg aag gcc tcc ttc gca gtc cag ggc ctc aac aca ctc     480
Leu Thr Gln Leu Lys Ala Ser Phe Ala Val Gln Gly Leu Asn Thr Leu
145                 150                 155                 160
```

```
gac ctc gtc aca ctc tcg gga ggt cac acc ttc gga cga gca cgc tgt         528
Asp Leu Val Thr Leu Ser Gly Gly His Thr Phe Gly Arg Ala Arg Cys
            165             170             175

tcg acc ttc att aac cgc ctc tac aac ttc tcc aac acg ggc aac ccc         576
Ser Thr Phe Ile Asn Arg Leu Tyr Asn Phe Ser Asn Thr Gly Asn Pro
            180             185             190

gat cct aca ctc aac aca acc tac ttg gag gtg ttg cga gca cgg tgt         624
Asp Pro Thr Leu Asn Thr Thr Tyr Leu Glu Val Leu Arg Ala Arg Cys
            195             200             205

cct cag aac gca acc gga gat aac ctc acc aac ctc gac ctc tcg aca         672
Pro Gln Asn Ala Thr Gly Asp Asn Leu Thr Asn Leu Asp Leu Ser Thr
            210             215             220

ccc gac cag ttc gac aac cgc tac tat tcg aac ttg ctc cag ctc aac         720
Pro Asp Gln Phe Asp Asn Arg Tyr Tyr Ser Asn Leu Leu Gln Leu Asn
225             230             235             240

ggt ctc ttg cag tcg gac cag gag ctc ttc tcg aca cct gga gcg gac         768
Gly Leu Leu Gln Ser Asp Gln Glu Leu Phe Ser Thr Pro Gly Ala Asp
            245             250             255

act atc cct atc gtg aac tcc ttc tcg tcg aac cag aac acc ttc ttc         816
Thr Ile Pro Ile Val Asn Ser Phe Ser Ser Asn Gln Asn Thr Phe Phe
            260             265             270

tcg aac ttc cga gtc tcc atg atc aaa atg ggc aac att gga gtc ttg         864
Ser Asn Phe Arg Val Ser Met Ile Lys Met Gly Asn Ile Gly Val Leu
            275             280             285

aca ggt gat gag ggc gaa atc agg ctc cag tgt aac ttc gtg aac ggc         912
Thr Gly Asp Glu Gly Glu Ile Arg Leu Gln Cys Asn Phe Val Asn Gly
            290             295             300

gac tcg ttc ggt ttg gcc tcg gtc gcc tcg aag gat gcc aag cag aag         960
Asp Ser Phe Gly Leu Ala Ser Val Ala Ser Lys Asp Ala Lys Gln Lys
305             310             315             320

ctc gtc gcc cag tcc aaa                                                  978
Leu Val Ala Gln Ser Lys
            325
```

<210> 2
<211> 326
<212> PRT
<213> Glycine max

<400> 2

```
Gln Leu Thr Pro Thr Phe Tyr Arg Glu Thr Cys Pro Asn Leu Phe Pro
1               5               10              15

Ile Val Phe Gly Val Ile Phe Asp Ala Ser Phe Thr Asp Pro Arg Ile
            20              25              30

Gly Ala Ser Leu Met Arg Leu His Phe His Asp Cys Phe Val Gln Gly
            35              40              45
```

Cys Asp Gly Ser Val Leu Leu Asn Asn Thr Asp Thr Ile Glu Ser Glu
    50                  55                  60

Gln Asp Ala Leu Pro Asn Ile Asn Ser Ile Arg Gly Leu Asp Val Val
65                  70                  75                  80

Asn Asp Ile Lys Thr Ala Val Glu Asn Ser Cys Pro Asp Thr Val Ser
                85                  90                  95

Cys Ala Asp Ile Leu Ala Ile Ala Ala Glu Ile Ala Ser Val Leu Gly
            100                 105                 110

Gly Gly Pro Gly Trp Pro Val Pro Leu Gly Arg Arg Asp Ser Leu Thr
            115                 120                 125

Ala Asn Arg Thr Leu Ala Asn Gln Asn Leu Pro Ala Pro Phe Phe Asn
    130                 135                 140

Leu Thr Gln Leu Lys Ala Ser Phe Ala Val Gln Gly Leu Asn Thr Leu
145                 150                 155                 160

Asp Leu Val Thr Leu Ser Gly Gly His Thr Phe Gly Arg Ala Arg Cys
                165                 170                 175

Ser Thr Phe Ile Asn Arg Leu Tyr Asn Phe Ser Asn Thr Gly Asn Pro
            180                 185                 190

Asp Pro Thr Leu Asn Thr Thr Tyr Leu Glu Val Leu Arg Ala Arg Cys
            195                 200                 205

Pro Gln Asn Ala Thr Gly Asp Asn Leu Thr Asn Leu Asp Leu Ser Thr
    210                 215                 220

Pro Asp Gln Phe Asp Asn Arg Tyr Tyr Ser Asn Leu Leu Gln Leu Asn
225                 230                 235                 240

Gly Leu Leu Gln Ser Asp Gln Glu Leu Phe Ser Thr Pro Gly Ala Asp
            245                 250                 255

Thr Ile Pro Ile Val Asn Ser Phe Ser Ser Asn Gln Asn Thr Phe Phe
            260                 265                 270

Ser Asn Phe Arg Val Ser Met Ile Lys Met Gly Asn Ile Gly Val Leu
            275                 280                 285

Thr Gly Asp Glu Gly Glu Ile Arg Leu Gln Cys Asn Phe Val Asn Gly
    290                 295                 300

```
       Asp Ser Phe Gly Leu Ala Ser Val Ala Ser Lys Asp Ala Lys Gln Lys
       305                 310                 315                 320


       Leu Val Ala Gln Ser Lys
                 325


<210> 3
<211> 912
<212> DNA
<213> Roystonea sp.


<220>
<221> CDS
<222> (1)..(912)


<400> 3


gac ctc cag att gga ttc tat aac acc tcc tgt ccg acc gca gaa tcg      48
Asp Leu Gln Ile Gly Phe Tyr Asn Thr Ser Cys Pro Thr Ala Glu Ser
1                 5                   10                  15

ttg gtc cag cag gcg gtg gca gca gcc ttc gcg aac aac tcc ggc att      96
Leu Val Gln Gln Ala Val Ala Ala Ala Phe Ala Asn Asn Ser Gly Ile
              20                  25                  30

gcc cct ggc ctc atc cgc atg cac ttc cac gac tgt ttc gtc agg ggt     144
Ala Pro Gly Leu Ile Arg Met His Phe His Asp Cys Phe Val Arg Gly
          35                  40                  45

tgt gac gcc tcc gtc ctc ttg gac tcg acc gcc aac aac acg gca gaa     192
Cys Asp Ala Ser Val Leu Leu Asp Ser Thr Ala Asn Asn Thr Ala Glu
      50                  55                  60

aag gat gca atc ccc aac aac ccc tcg ctc agg ggc ttc gag gtg atc     240
Lys Asp Ala Ile Pro Asn Asn Pro Ser Leu Arg Gly Phe Glu Val Ile
65                  70                  75                  80

acc gca gca aag tcg gca gtc gaa gcc gca tgt ccg cag act gtg tcc     288
Thr Ala Ala Lys Ser Ala Val Glu Ala Ala Cys Pro Gln Thr Val Ser
                  85                  90                  95

tgt gcc gac att ctc gcc ttc gca gcc cga gac tcg gcg aac ttg gca     336
Cys Ala Asp Ile Leu Ala Phe Ala Ala Arg Asp Ser Ala Asn Leu Ala
              100                 105                 110

ggc aac att act tac cag gtg ccg tcc gga cga cga gac ggc aca gtg     384
Gly Asn Ile Thr Tyr Gln Val Pro Ser Gly Arg Arg Asp Gly Thr Val
          115                 120                 125

tcc ttg gca tcc gaa gcc aac gcg cag atc ccc tcc cct ctc ttc aac     432
Ser Leu Ala Ser Glu Ala Asn Ala Gln Ile Pro Ser Pro Leu Phe Asn
      130                 135                 140

gcc aca cag ttg atc aac tcg ttc gcg aac aag act ctc act gcc gac     480
Ala Thr Gln Leu Ile Asn Ser Phe Ala Asn Lys Thr Leu Thr Ala Asp
145                 150                 155                 160

gaa atg gtc aca ttg tcc gga gcc cac tcg atc ggc gtg gca cac tgt     528
Glu Met Val Thr Leu Ser Gly Ala His Ser Ile Gly Val Ala His Cys
```

|   |   |   | 165 |   |   |   |   | 170 |   |   |   |   |   | 175 |   |   |   |
|---|---|---|---|---|---|---|---|---|---|---|---|---|---|---|---|---|---|

```
                        165                      170                         175

      tcc tcg ttc acg aac cga ctc tac aac ttc aac tcg ggc tcc ggc atc          576
      Ser Ser Phe Thr Asn Arg Leu Tyr Asn Phe Asn Ser Gly Ser Gly Ile
                  180                 185                 190

      gac ccg aca ctc tcc cct tcg tac gca gca ctc ttg cgc aac aca tgt          624
      Asp Pro Thr Leu Ser Pro Ser Tyr Ala Ala Leu Leu Arg Asn Thr Cys
                  195                 200                 205

      cct gcc aac tcc aca cgg ttc acg cct atc acc gtg tcg ttg gac att          672
      Pro Ala Asn Ser Thr Arg Phe Thr Pro Ile Thr Val Ser Leu Asp Ile
                  210                 215                 220

      atc acc ccg tcg gtc ttg gat aac atg tac tac acc ggt gtc cag ctc          720
      Ile Thr Pro Ser Val Leu Asp Asn Met Tyr Tyr Thr Gly Val Gln Leu
      225                 230                 235                 240

      acc ttg gga ttg ctc acc tcg gat cag gca ctc gtg acg gaa gcc aac          768
      Thr Leu Gly Leu Leu Thr Ser Asp Gln Ala Leu Val Thr Glu Ala Asn
                  245                 250                 255

      ttg tcc gca gcg gtg aaa gca aac gca atg aac ttg act gcg tgg gcg          816
      Leu Ser Ala Ala Val Lys Ala Asn Ala Met Asn Leu Thr Ala Trp Ala
                  260                 265                 270

      tcg aag ttc gcc cag gcc atg gtg aaa atg gga cag atc gaa gtc ctc          864
      Ser Lys Phe Ala Gln Ala Met Val Lys Met Gly Gln Ile Glu Val Leu
                  275                 280                 285

      acg ggt acc cag gga gag atc agg acc aac tgt tcc gtg gtc aac tcc          912
      Thr Gly Thr Gln Gly Glu Ile Arg Thr Asn Cys Ser Val Val Asn Ser
                  290                 295                 300
```

<210> 4
<211> 304
<212> PRT
<213> Roystonea sp.

<400> 4

```
Asp Leu Gln Ile Gly Phe Tyr Asn Thr Ser Cys Pro Thr Ala Glu Ser
1               5                   10                  15

Leu Val Gln Gln Ala Val Ala Ala Ala Phe Ala Asn Asn Ser Gly Ile
            20                  25                  30

Ala Pro Gly Leu Ile Arg Met His Phe His Asp Cys Phe Val Arg Gly
            35                  40                  45

Cys Asp Ala Ser Val Leu Leu Asp Ser Thr Ala Asn Asn Thr Ala Glu
        50                  55                  60

Lys Asp Ala Ile Pro Asn Asn Pro Ser Leu Arg Gly Phe Glu Val Ile
65                  70                  75                  80

Thr Ala Ala Lys Ser Ala Val Glu Ala Ala Cys Pro Gln Thr Val Ser
```

                            85                          90                          95

Cys Ala Asp Ile Leu Ala Phe Ala Ala Arg Asp Ser Ala Asn Leu Ala
            100                 105             110

Gly Asn Ile Thr Tyr Gln Val Pro Ser Gly Arg Arg Asp Gly Thr Val
            115                 120             125

Ser Leu Ala Ser Glu Ala Asn Ala Gln Ile Pro Ser Pro Leu Phe Asn
            130                 135             140

Ala Thr Gln Leu Ile Asn Ser Phe Ala Asn Lys Thr Leu Thr Ala Asp
145                 150                 155                 160

Glu Met Val Thr Leu Ser Gly Ala His Ser Ile Gly Val Ala His Cys
                    165                 170                 175

Ser Ser Phe Thr Asn Arg Leu Tyr Asn Phe Asn Ser Gly Ser Gly Ile
            180                 185                 190

Asp Pro Thr Leu Ser Pro Ser Tyr Ala Ala Leu Leu Arg Asn Thr Cys
            195                 200                 205

Pro Ala Asn Ser Thr Arg Phe Thr Pro Ile Thr Val Ser Leu Asp Ile
            210                 215                 220

Ile Thr Pro Ser Val Leu Asp Asn Met Tyr Tyr Thr Gly Val Gln Leu
225                 230                 235                 240

Thr Leu Gly Leu Leu Thr Ser Asp Gln Ala Leu Val Thr Glu Ala Asn
                    245                 250                 255

Leu Ser Ala Ala Val Lys Ala Asn Ala Met Asn Leu Thr Ala Trp Ala
            260                 265                 270

Ser Lys Phe Ala Gln Ala Met Val Lys Met Gly Gln Ile Glu Val Leu
            275                 280                 285

Thr Gly Thr Gln Gly Glu Ile Arg Thr Asn Cys Ser Val Val Asn Ser
            290                 295                 300

<210> 5
<211> 7
<212> PRT
<213> Artificial

<220>
<223> Motif

<400> 5

```
                              His Phe His Asp Cys Phe Val
                              1               5
```

<210> 6
<211> 8
<212> PRT
<213> Artificial

<220>
<223> Motif

<220>
<221> MISC_FEATURE
<222> (4)..(4)
<223> Xaa is Ala or Gly

<220>
<221> MISC_FEATURE
<222> (6)..(6)
<223> Xaa is Val or Ile

<400> 6

```
                              Gly Cys Asp Xaa Ser Xaa Leu Leu
                              1               5
```

<210> 7
<211> 7
<212> PRT
<213> Artificial

<220>
<223> Motif

<220>
<221> MISC_FEATURE
<222> (4)..(4)
<223> Xaa is Ala or Ser

<220>
<221> MISC_FEATURE
<222> (6)..(6)
<223> Xaa is Ile or Leu

<400> 7

```
                              Val Ser Cys Xaa Asp Xaa Leu
                              1               5
```

<210> 8
<211> 1059
<212> DNA
<213> Artificial

<220>
<223> Artificial construct

<220>
<221> CDS
<222> (1)..(1056)

<220>
<221> sig_peptide
<222> (1)..(78)

<220>
<221> mat_peptide
<222> (79)..(1056)

<400> 8

```
atg ggc tcg atg cgc ttg ctc gtg gtc gcc ctc ctc tgt gcc ttc gcg      48
Met Gly Ser Met Arg Leu Leu Val Val Ala Leu Leu Cys Ala Phe Ala
    -25                 -20                 -15

atg cat gca ggc ttc tcg gtg tcg tat gca cag ctc aca ccc act ttc      96
Met His Ala Gly Phe Ser Val Ser Tyr Ala Gln Leu Thr Pro Thr Phe
-10                 -5                 -1  1                 5

tac agg gaa acc tgt ccc aac ttg ttc ccc att gtg ttc ggc gtc atc     144
Tyr Arg Glu Thr Cys Pro Asn Leu Phe Pro Ile Val Phe Gly Val Ile
                10                 15                 20

ttc gat gcg tcg ttc acc gac ccc agg atc gga gcc tcg ctc atg cgc     192
Phe Asp Ala Ser Phe Thr Asp Pro Arg Ile Gly Ala Ser Leu Met Arg
            25                 30                 35

ctc cat ttc cac gac tgt ttc gtc cag ggc tgt gac ggt tcc gtc ttg     240
Leu His Phe His Asp Cys Phe Val Gln Gly Cys Asp Gly Ser Val Leu
            40                 45                 50

ttg aac aac acc gac acc atc gag tcc gag cag gac gcg ctc ccc aac     288
Leu Asn Asn Thr Asp Thr Ile Glu Ser Glu Gln Asp Ala Leu Pro Asn
55                 60                 65                 70

atc aac tcc atc cga ggc ctc gat gtc gtg aac gac atc aaa acc gca     336
Ile Asn Ser Ile Arg Gly Leu Asp Val Val Asn Asp Ile Lys Thr Ala
                75                 80                 85

gtg gaa aac tcc tgt ccc gat acg gtc tcc tgt gca gac atc ttg gcg     384
Val Glu Asn Ser Cys Pro Asp Thr Val Ser Cys Ala Asp Ile Leu Ala
                90                 95                 100

att gca gcc gag atc gca tcg gtc ctc gga ggc ggt cct ggc tgg cct     432
Ile Ala Ala Glu Ile Ala Ser Val Leu Gly Gly Gly Pro Gly Trp Pro
            105                 110                 115

gtg ccg ctc gga cga cgg gac tcg ttg aca gca aac agg acg ctc gca     480
Val Pro Leu Gly Arg Arg Asp Ser Leu Thr Ala Asn Arg Thr Leu Ala
            120                 125                 130

aac cag aac ttg cct gcg cct ttc ttc aac ctc acc cag ttg aag gcc     528
Asn Gln Asn Leu Pro Ala Pro Phe Phe Asn Leu Thr Gln Leu Lys Ala
135                 140                 145                 150

tcc ttc gca gtc cag ggc ctc aac aca ctc gac ctc gtc aca ctc tcg     576
Ser Phe Ala Val Gln Gly Leu Asn Thr Leu Asp Leu Val Thr Leu Ser
                155                 160                 165
```

```
gga ggt cac acc ttc gga cga gca cgc tgt tcg acc ttc att aac cgc      624
Gly Gly His Thr Phe Gly Arg Ala Arg Cys Ser Thr Phe Ile Asn Arg
        170             175             180

ctc tac aac ttc tcc aac acg ggc aac ccc gat cct aca ctc aac aca      672
Leu Tyr Asn Phe Ser Asn Thr Gly Asn Pro Asp Pro Thr Leu Asn Thr
        185             190             195

acc tac ttg gag gtg ttg cga gca cgg tgt cct cag aac gca acc gga      720
Thr Tyr Leu Glu Val Leu Arg Ala Arg Cys Pro Gln Asn Ala Thr Gly
        200             205             210

gat aac ctc acc aac ctc gac ctc tcg aca ccc gac cag ttc gac aac      768
Asp Asn Leu Thr Asn Leu Asp Leu Ser Thr Pro Asp Gln Phe Asp Asn
215             220             225             230

cgc tac tat tcg aac ttg ctc cag ctc aac ggt ctc ttg cag tcg gac      816
Arg Tyr Tyr Ser Asn Leu Leu Gln Leu Asn Gly Leu Leu Gln Ser Asp
            235             240             245

cag gag ctc ttc tcg aca cct gga gcg gac act atc cct atc gtg aac      864
Gln Glu Leu Phe Ser Thr Pro Gly Ala Asp Thr Ile Pro Ile Val Asn
        250             255             260

tcc ttc tcg tcg aac cag aac acc ttc ttc tcg aac ttc cga gtc tcc      912
Ser Phe Ser Ser Asn Gln Asn Thr Phe Phe Ser Asn Phe Arg Val Ser
        265             270             275

atg atc aaa atg ggc aac att gga gtc ttg aca ggt gat gag ggc gaa      960
Met Ile Lys Met Gly Asn Ile Gly Val Leu Thr Gly Asp Glu Gly Glu
        280             285             290

atc agg ctc cag tgt aac ttc gtg aac ggc gac tcg ttc ggt ttg gcc     1008
Ile Arg Leu Gln Cys Asn Phe Val Asn Gly Asp Ser Phe Gly Leu Ala
295             300             305             310

tcg gtc gcc tcg aag gat gcc aag cag aag ctc gtc gcc cag tcc aaa     1056
Ser Val Ala Ser Lys Asp Ala Lys Gln Lys Leu Val Ala Gln Ser Lys
            315             320             325

tga                                                                  1059
```

<210> 9
<211> 352
<212> PRT
<213> Artificial

<220>
<223> Synthetic Construct

<400> 9

Met Gly Ser Met Arg Leu Leu Val Val Ala Leu Leu Cys Ala Phe Ala
    −25                 −20                 −15

Met His Ala Gly Phe Ser Val Ser Tyr Ala Gln Leu Thr Pro Thr Phe
−10                 −5              −1   1               5

Tyr Arg Glu Thr Cys Pro Asn Leu Phe Pro Ile Val Phe Gly Val Ile
            10              15              20

Phe Asp Ala Ser Phe Thr Asp Pro Arg Ile Gly Ala Ser Leu Met Arg
        25                  30                  35

Leu His Phe His Asp Cys Phe Val Gln Gly Cys Asp Gly Ser Val Leu
        40                  45                  50

Leu Asn Asn Thr Asp Thr Ile Glu Ser Glu Gln Asp Ala Leu Pro Asn
55                  60                  65                  70

Ile Asn Ser Ile Arg Gly Leu Asp Val Val Asn Asp Ile Lys Thr Ala
                75                  80                  85

Val Glu Asn Ser Cys Pro Asp Thr Val Ser Cys Ala Asp Ile Leu Ala
                90                  95                  100

Ile Ala Ala Glu Ile Ala Ser Val Leu Gly Gly Gly Pro Gly Trp Pro
        105                 110                 115

Val Pro Leu Gly Arg Arg Asp Ser Leu Thr Ala Asn Arg Thr Leu Ala
        120                 125                 130

Asn Gln Asn Leu Pro Ala Pro Phe Phe Asn Leu Thr Gln Leu Lys Ala
135                 140                 145                 150

Ser Phe Ala Val Gln Gly Leu Asn Thr Leu Asp Leu Val Thr Leu Ser
                155                 160                 165

Gly Gly His Thr Phe Gly Arg Ala Arg Cys Ser Thr Phe Ile Asn Arg
            170                 175                 180

Leu Tyr Asn Phe Ser Asn Thr Gly Asn Pro Asp Pro Thr Leu Asn Thr
        185                 190                 195

Thr Tyr Leu Glu Val Leu Arg Ala Arg Cys Pro Gln Asn Ala Thr Gly
        200                 205                 210

Asp Asn Leu Thr Asn Leu Asp Leu Ser Thr Pro Asp Gln Phe Asp Asn
215                 220                 225                 230

Arg Tyr Tyr Ser Asn Leu Leu Gln Leu Asn Gly Leu Leu Gln Ser Asp
                235                 240                 245

Gln Glu Leu Phe Ser Thr Pro Gly Ala Asp Thr Ile Pro Ile Val Asn
            250                 255                 260

Ser Phe Ser Ser Asn Gln Asn Thr Phe Phe Ser Asn Phe Arg Val Ser

28

```
                    265                        270                          275


        Met Ile Lys Met Gly Asn Ile Gly Val Leu Thr Gly Asp Glu Gly Glu
            280                 285                 290


        Ile Arg Leu Gln Cys Asn Phe Val Asn Gly Asp Ser Phe Gly Leu Ala
        295                 300                 305                     310


        Ser Val Ala Ser Lys Asp Ala Lys Gln Lys Leu Val Ala Gln Ser Lys
                        315                 320                     325
```

<210> 10
<211> 1092
<212> DNA
<213> Artificial

<220>
<223> Artificial construct

<220>
<221> CDS
<222> (1)..(1089)

<220>
<221> sig_peptide
<222> (1)..(75)

<220>
<221> mat_peptide
<222> (112)..(1089)

<400> 10

```
atg aag ttc ttc acc acc atc ctc agc acc gcc agc ctt gtt gct gct          48
Met Lys Phe Phe Thr Thr Ile Leu Ser Thr Ala Ser Leu Val Ala Ala
        -35             -30                 -25


ctc ccc gcc gct gtt gac tcg aac cat acc ccg gcc gct cct gaa ctt          96
Leu Pro Ala Ala Val Asp Ser Asn His Thr Pro Ala Ala Pro Glu Leu
        -20             -15                 -10


gtt gcc cgc cta gga cag ctc aca ccc act ttc tac agg gaa acc tgt         144
Val Ala Arg Leu Gly Gln Leu Thr Pro Thr Phe Tyr Arg Glu Thr Cys
-5              -1  1            5                       10


ccc aac ttg ttc ccc att gtg ttc ggc gtc atc ttc gat gcg tcg ttc         192
Pro Asn Leu Phe Pro Ile Val Phe Gly Val Ile Phe Asp Ala Ser Phe
            15              20                  25


acc gac ccc agg atc gga gcc tcg ctc atg cgc ctc cat ttc cac gac         240
Thr Asp Pro Arg Ile Gly Ala Ser Leu Met Arg Leu His Phe His Asp
        30              35                  40


tgt ttc gtc cag ggc tgt gac ggt tcc gtc ttg ttg aac aac acc gac         288
Cys Phe Val Gln Gly Cys Asp Gly Ser Val Leu Leu Asn Asn Thr Asp
    45              50                  55


acc atc gag tcc gag cag gac gcg ctc ccc aac atc aac tcc atc cga         336
Thr Ile Glu Ser Glu Gln Asp Ala Leu Pro Asn Ile Asn Ser Ile Arg
```

60 65 70 75

```
ggc ctc gat gtc gtg aac gac atc aaa acc gca gtg gaa aac tcc tgt          384
Gly Leu Asp Val Val Asn Asp Ile Lys Thr Ala Val Glu Asn Ser Cys
            80                  85                  90

ccc gat acg gtc tcc tgt gca gac atc ttg gcg att gca gcc gag atc          432
Pro Asp Thr Val Ser Cys Ala Asp Ile Leu Ala Ile Ala Ala Glu Ile
            95                 100                 105

gca tcg gtc ctc gga ggc ggt cct ggc tgg cct gtg ccg ctc gga cga          480
Ala Ser Val Leu Gly Gly Gly Pro Gly Trp Pro Val Pro Leu Gly Arg
            110                115                 120

cgg gac tcg ttg aca gca aac agg acg ctc gca aac cag aac ttg cct          528
Arg Asp Ser Leu Thr Ala Asn Arg Thr Leu Ala Asn Gln Asn Leu Pro
            125                130                 135

gcg cct ttc ttc aac ctc acc cag ttg aag gcc tcc ttc gca gtc cag          576
Ala Pro Phe Phe Asn Leu Thr Gln Leu Lys Ala Ser Phe Ala Val Gln
140                145                 150                 155

ggc ctc aac aca ctc gac ctc gtc aca ctc tcg gga ggt cac acc ttc          624
Gly Leu Asn Thr Leu Asp Leu Val Thr Leu Ser Gly Gly His Thr Phe
            160                165                 170

gga cga gca cgc tgt tcg acc ttc att aac cgc ctc tac aac ttc tcc          672
Gly Arg Ala Arg Cys Ser Thr Phe Ile Asn Arg Leu Tyr Asn Phe Ser
            175                180                 185

aac acg ggc aac ccc gat cct aca ctc aac aca acc tac ttg gag gtg          720
Asn Thr Gly Asn Pro Asp Pro Thr Leu Asn Thr Thr Tyr Leu Glu Val
            190                195                 200

ttg cga gca cgg tgt cct cag aac gca acc gga gat aac ctc acc aac          768
Leu Arg Ala Arg Cys Pro Gln Asn Ala Thr Gly Asp Asn Leu Thr Asn
            205                210                 215

ctc gac ctc tcg aca ccc gac cag ttc gac aac cgc tac tat tcg aac          816
Leu Asp Leu Ser Thr Pro Asp Gln Phe Asp Asn Arg Tyr Tyr Ser Asn
220                225                 230                 235

ttg ctc cag ctc aac ggt ctc ttg cag tcg gac cag gag ctc ttc tcg          864
Leu Leu Gln Leu Asn Gly Leu Leu Gln Ser Asp Gln Glu Leu Phe Ser
            240                245                 250

aca cct gga gcg gac act atc cct atc gtg aac tcc ttc tcg tcg aac          912
Thr Pro Gly Ala Asp Thr Ile Pro Ile Val Asn Ser Phe Ser Ser Asn
            255                260                 265

cag aac acc ttc ttc tcg aac ttc cga gtc tcc atg atc aaa atg ggc          960
Gln Asn Thr Phe Phe Ser Asn Phe Arg Val Ser Met Ile Lys Met Gly
            270                275                 280

aac att gga gtc ttg aca ggt gat gag ggc gaa atc agg ctc cag tgt         1008
Asn Ile Gly Val Leu Thr Gly Asp Glu Gly Glu Ile Arg Leu Gln Cys
            285                290                 295

aac ttc gtg aac ggc gac tcg ttc ggt ttg gcc tcg gtc gcc tcg aag         1056
Asn Phe Val Asn Gly Asp Ser Phe Gly Leu Ala Ser Val Ala Ser Lys
300                305                 310                 315

gat gcc aag cag aag ctc gtc gcc cag tcc aaa tga                         1092
Asp Ala Lys Gln Lys Leu Val Ala Gln Ser Lys
```

31

```
Asp Ala Lys Gln Lys Leu Val Ala Gln Ser Lys
            320                 325
```

<210> 11
<211> 363
<212> PRT
<213> Artificial

<220>
<223> Synthetic Construct

<400> 11

```
Met Lys Phe Phe Thr Thr Ile Leu Ser Thr Ala Ser Leu Val Ala Ala
    -35             -30             -25

Leu Pro Ala Ala Val Asp Ser Asn His Thr Pro Ala Ala Pro Glu Leu
    -20             -15             -10

Val Ala Arg Leu Gly Gln Leu Thr Pro Thr Phe Tyr Arg Glu Thr Cys
-5           -1  1               5               10

Pro Asn Leu Phe Pro Ile Val Phe Gly Val Ile Phe Asp Ala Ser Phe
            15              20              25

Thr Asp Pro Arg Ile Gly Ala Ser Leu Met Arg Leu His Phe His Asp
        30              35              40

Cys Phe Val Gln Gly Cys Asp Gly Ser Val Leu Leu Asn Asn Thr Asp
    45              50              55

Thr Ile Glu Ser Glu Gln Asp Ala Leu Pro Asn Ile Asn Ser Ile Arg
60              65              70              75

Gly Leu Asp Val Val Asn Asp Ile Lys Thr Ala Val Glu Asn Ser Cys
            80              85              90

Pro Asp Thr Val Ser Cys Ala Asp Ile Leu Ala Ile Ala Ala Glu Ile
        95              100             105

Ala Ser Val Leu Gly Gly Gly Pro Gly Trp Pro Val Pro Leu Gly Arg
        110             115             120

Arg Asp Ser Leu Thr Ala Asn Arg Thr Leu Ala Asn Gln Asn Leu Pro
        125             130             135

Ala Pro Phe Phe Asn Leu Thr Gln Leu Lys Ala Ser Phe Ala Val Gln
140             145             150             155

Gly Leu Asn Thr Leu Asp Leu Val Thr Leu Ser Gly Gly His Thr Phe
```

160            165            170

```
Gly Arg Ala Arg Cys Ser Thr Phe Ile Asn Arg Leu Tyr Asn Phe Ser
            175                 180                 185

Asn Thr Gly Asn Pro Asp Pro Thr Leu Asn Thr Thr Tyr Leu Glu Val
            190                 195                 200

Leu Arg Ala Arg Cys Pro Gln Asn Ala Thr Gly Asp Asn Leu Thr Asn
            205                 210                 215

Leu Asp Leu Ser Thr Pro Asp Gln Phe Asp Asn Arg Tyr Tyr Ser Asn
220                 225                 230                 235

Leu Leu Gln Leu Asn Gly Leu Leu Gln Ser Asp Gln Glu Leu Phe Ser
                240                 245                 250

Thr Pro Gly Ala Asp Thr Ile Pro Ile Val Asn Ser Phe Ser Ser Asn
            255                 260                 265

Gln Asn Thr Phe Phe Ser Asn Phe Arg Val Ser Met Ile Lys Met Gly
            270                 275                 280

Asn Ile Gly Val Leu Thr Gly Asp Glu Gly Glu Ile Arg Leu Gln Cys
            285                 290                 295

Asn Phe Val Asn Gly Asp Ser Phe Gly Leu Ala Ser Val Ala Ser Lys
300                 305                 310                 315

Asp Ala Lys Gln Lys Leu Val Ala Gln Ser Lys
                320                 325
```

<210> 12
<211> 1056
<212> DNA
<213> Artificial

<220>
<223> Artificial construct

<220>
<221> CDS
<222> (1)..(1053)

<220>
<221> sig_peptide
<222> (1)..(75)

<220>
<221> mat_peptide

<222> (76)..(1053)

<400> 12

```
atg aag ttc ttc acc acc atc ctc agc acc gcc agc ctt gtt gct gct        48
Met Lys Phe Phe Thr Thr Ile Leu Ser Thr Ala Ser Leu Val Ala Ala
-25              -20              -15                  -10

ctc ccc gcc gct gtt gac tcc cta gga cag ctc aca ccc act ttc tac        96
Leu Pro Ala Ala Val Asp Ser Leu Gly Gln Leu Thr Pro Thr Phe Tyr
            -5              -1  1                  5

agg gaa acc tgt ccc aac ttg ttc ccc att gtg ttc ggc gtc atc ttc       144
Arg Glu Thr Cys Pro Asn Leu Phe Pro Ile Val Phe Gly Val Ile Phe
        10              15                  20

gat gcg tcg ttc acc gac ccc agg atc gga gcc tcg ctc atg cgc ctc       192
Asp Ala Ser Phe Thr Asp Pro Arg Ile Gly Ala Ser Leu Met Arg Leu
    25              30                  35

cat ttc cac gac tgt ttc gtc cag ggc tgt gac ggt tcc gtc ttg ttg       240
His Phe His Asp Cys Phe Val Gln Gly Cys Asp Gly Ser Val Leu Leu
40                  45                  50                  55

aac aac acc gac acc atc gag tcc gag cag gac gcg ctc ccc aac atc       288
Asn Asn Thr Asp Thr Ile Glu Ser Glu Gln Asp Ala Leu Pro Asn Ile
            60              65                  70

aac tcc atc cga ggc ctc gat gtc gtg aac gac atc aaa acc gca gtg       336
Asn Ser Ile Arg Gly Leu Asp Val Val Asn Asp Ile Lys Thr Ala Val
            75              80                  85

gaa aac tcc tgt ccc gat acg gtc tcc tgt gca gac atc ttg gcg att       384
Glu Asn Ser Cys Pro Asp Thr Val Ser Cys Ala Asp Ile Leu Ala Ile
        90              95                  100

gca gcc gag atc gca tcg gtc ctc gga ggc ggt cct ggc tgg cct gtg       432
Ala Ala Glu Ile Ala Ser Val Leu Gly Gly Gly Pro Gly Trp Pro Val
        105             110                 115

ccg ctc gga cga cgg gac tcg ttg aca gca aac agg acg ctc gca aac       480
Pro Leu Gly Arg Arg Asp Ser Leu Thr Ala Asn Arg Thr Leu Ala Asn
120             125                 130                 135

cag aac ttg cct gcg cct ttc ttc aac ctc acc cag ttg aag gcc tcc       528
Gln Asn Leu Pro Ala Pro Phe Phe Asn Leu Thr Gln Leu Lys Ala Ser
            140             145                 150

ttc gca gtc cag ggc ctc aac aca ctc gac ctc gtc aca ctc tcg gga       576
Phe Ala Val Gln Gly Leu Asn Thr Leu Asp Leu Val Thr Leu Ser Gly
        155             160                 165

ggt cac acc ttc gga cga gca cgc tgt tcg acc ttc att aac cgc ctc       624
Gly His Thr Phe Gly Arg Ala Arg Cys Ser Thr Phe Ile Asn Arg Leu
        170             175                 180

tac aac ttc tcc aac acg ggc aac ccc gat cct aca ctc aac aca acc       672
Tyr Asn Phe Ser Asn Thr Gly Asn Pro Asp Pro Thr Leu Asn Thr Thr
        185             190                 195

tac ttg gag gtg ttg cga gca cgg tgt cct cag aac gca acc gga gat       720
Tyr Leu Glu Val Leu Arg Ala Arg Cys Pro Gln Asn Ala Thr Gly Asp
200             205                 210                 215

aac ctc acc aac ctc gac ctc tcg aca ccc gac cag ttc gac aac cgc       768
```

```
Asn Leu Thr Asn Leu Asp Leu Ser Thr Pro Asp Gln Phe Asp Asn Arg
            220             225             230

tac tat tcg aac ttg ctc cag ctc aac ggt ctc ttg cag tcg gac cag    816
Tyr Tyr Ser Asn Leu Leu Gln Leu Asn Gly Leu Leu Gln Ser Asp Gln
            235             240             245

gag ctc ttc tcg aca cct gga gcg gac act atc cct atc gtg aac tcc    864
Glu Leu Phe Ser Thr Pro Gly Ala Asp Thr Ile Pro Ile Val Asn Ser
        250             255             260

ttc tcg tcg aac cag aac acc ttc ttc tcg aac ttc cga gtc tcc atg    912
Phe Ser Ser Asn Gln Asn Thr Phe Phe Ser Asn Phe Arg Val Ser Met
    265             270             275

atc aaa atg ggc aac att gga gtc ttg aca ggt gat gag ggc gaa atc    960
Ile Lys Met Gly Asn Ile Gly Val Leu Thr Gly Asp Glu Gly Glu Ile
280             285             290             295

agg ctc cag tgt aac ttc gtg aac ggc gac tcg ttc ggt ttg gcc tcg   1008
Arg Leu Gln Cys Asn Phe Val Asn Gly Asp Ser Phe Gly Leu Ala Ser
            300             305             310

gtc gcc tcg aag gat gcc aag cag aag ctc gtc gcc cag tcc aaa tga   1056
Val Ala Ser Lys Asp Ala Lys Gln Lys Leu Val Ala Gln Ser Lys
            315             320             325
```

<210> 13
<211> 351
<212> PRT
<213> Artificial

<220>
<223> Synthetic Construct

<400> 13

```
Met Lys Phe Phe Thr Thr Ile Leu Ser Thr Ala Ser Leu Val Ala Ala
-25               -20               -15                         -10


Leu Pro Ala Ala Val Asp Ser Leu Gly Gln Leu Thr Pro Thr Phe Tyr
              -5               -1  1                   5


Arg Glu Thr Cys Pro Asn Leu Phe Pro Ile Val Phe Gly Val Ile Phe
        10              15              20


Asp Ala Ser Phe Thr Asp Pro Arg Ile Gly Ala Ser Leu Met Arg Leu
    25              30              35


His Phe His Asp Cys Phe Val Gln Gly Cys Asp Gly Ser Val Leu Leu
40              45              50                          55


Asn Asn Thr Asp Thr Ile Glu Ser Glu Gln Asp Ala Leu Pro Asn Ile
            60              65              70


Asn Ser Ile Arg Gly Leu Asp Val Val Asn Asp Ile Lys Thr Ala Val
```

```
                    75                      80                      85


      Glu Asn Ser Cys Pro Asp Thr Val Ser Cys Ala Asp Ile Leu Ala Ile
              90                      95                     100


      Ala Ala Glu Ile Ala Ser Val Leu Gly Gly Gly Pro Gly Trp Pro Val
          105                     110                     115


      Pro Leu Gly Arg Arg Asp Ser Leu Thr Ala Asn Arg Thr Leu Ala Asn
      120                     125                     130                     135


      Gln Asn Leu Pro Ala Pro Phe Phe Asn Leu Thr Gln Leu Lys Ala Ser
                      140                     145                     150


      Phe Ala Val Gln Gly Leu Asn Thr Leu Asp Leu Val Thr Leu Ser Gly
                  155                     160                     165


      Gly His Thr Phe Gly Arg Ala Arg Cys Ser Thr Phe Ile Asn Arg Leu
              170                     175                     180


      Tyr Asn Phe Ser Asn Thr Gly Asn Pro Asp Pro Thr Leu Asn Thr Thr
          185                     190                     195


      Tyr Leu Glu Val Leu Arg Ala Arg Cys Pro Gln Asn Ala Thr Gly Asp
      200                     205                     210                     215


      Asn Leu Thr Asn Leu Asp Leu Ser Thr Pro Asp Gln Phe Asp Asn Arg
                      220                     225                     230


      Tyr Tyr Ser Asn Leu Leu Gln Leu Asn Gly Leu Leu Gln Ser Asp Gln
                  235                     240                     245


      Glu Leu Phe Ser Thr Pro Gly Ala Asp Thr Ile Pro Ile Val Asn Ser
                  250                     255                     260


      Phe Ser Ser Asn Gln Asn Thr Phe Phe Ser Asn Phe Arg Val Ser Met
          265                     270                     275


      Ile Lys Met Gly Asn Ile Gly Val Leu Thr Gly Asp Glu Gly Glu Ile
      280                     285                     290                     295


      Arg Leu Gln Cys Asn Phe Val Asn Gly Asp Ser Phe Gly Leu Ala Ser
                  300                     305                     310


      Val Ala Ser Lys Asp Ala Lys Gln Lys Leu Val Ala Gln Ser Lys
                  315                     320                     325
```

<210> 14
<211> 1086
<212> DNA
<213> Artificial

<220>
<223> Artificial construct

<220>
<221> CDS
<222> (1)..(1083)

<220>
<221> sig_peptide
<222> (1)..(69)

<220>
<221> mat_peptide
<222> (106)..(1083)

<400> 14

```
atg aga tta tcg act tcg agt ctc ttc ctt tcc gtg tct ctg ctg ggg        48
Met Arg Leu Ser Thr Ser Ser Leu Phe Leu Ser Val Ser Leu Leu Gly
-35             -30             -25             -20

aag ctg gcc ctc ggg agc cct ttg ccc caa cag cag cga tat ggc aaa        96
Lys Leu Ala Leu Gly Ser Pro Leu Pro Gln Gln Gln Arg Tyr Gly Lys
                -15             -10             -5

cgc cta gga cag ctc aca ccc act ttc tac agg gaa acc tgt ccc aac       144
Arg Leu Gly Gln Leu Thr Pro Thr Phe Tyr Arg Glu Thr Cys Pro Asn
        -1  1           5               10

ttg ttc ccc att gtg ttc ggc gtc atc ttc gat gcg tcg ttc acc gac       192
Leu Phe Pro Ile Val Phe Gly Val Ile Phe Asp Ala Ser Phe Thr Asp
        15              20              25

ccc agg atc gga gcc tcg ctc atg cgc ctc cat ttc cac gac tgt ttc       240
Pro Arg Ile Gly Ala Ser Leu Met Arg Leu His Phe His Asp Cys Phe
30              35              40              45

gtc cag ggc tgt gac ggt tcc gtc ttg ttg aac aac acc gac acc atc       288
Val Gln Gly Cys Asp Gly Ser Val Leu Leu Asn Asn Thr Asp Thr Ile
                50              55              60

gag tcc gag cag gac gcg ctc ccc aac atc aac tcc atc cga ggc ctc       336
Glu Ser Glu Gln Asp Ala Leu Pro Asn Ile Asn Ser Ile Arg Gly Leu
                65              70              75

gat gtc gtg aac gac atc aaa acc gca gtg gaa aac tcc tgt ccc gat       384
Asp Val Val Asn Asp Ile Lys Thr Ala Val Glu Asn Ser Cys Pro Asp
                80              85              90

acg gtc tcc tgt gca gac atc ttg gcg att gca gcc gag atc gca tcg       432
Thr Val Ser Cys Ala Asp Ile Leu Ala Ile Ala Ala Glu Ile Ala Ser
    95              100             105

gtc ctc gga ggc ggt cct ggc tgg cct gtg ccg ctc gga cga cgg gac       480
Val Leu Gly Gly Gly Pro Gly Trp Pro Val Pro Leu Gly Arg Arg Asp
110             115             120             125

tcg ttg aca gca aac agg acg ctc gca aac cag aac ttg cct gcg cct       528
```

```
            Ser Leu Thr Ala Asn Arg Thr Leu Ala Asn Gln Asn Leu Pro Ala Pro
                        130                 135                 140


            ttc ttc aac ctc acc cag ttg aag gcc tcc ttc gca gtc cag ggc ctc          576
            Phe Phe Asn Leu Thr Gln Leu Lys Ala Ser Phe Ala Val Gln Gly Leu
                        145                 150                 155


            aac aca ctc gac ctc gtc aca ctc tcg gga ggt cac acc ttc gga cga          624
            Asn Thr Leu Asp Leu Val Thr Leu Ser Gly Gly His Thr Phe Gly Arg
                        160                 165                 170


            gca cgc tgt tcg acc ttc att aac cgc ctc tac aac ttc tcc aac acg          672
            Ala Arg Cys Ser Thr Phe Ile Asn Arg Leu Tyr Asn Phe Ser Asn Thr
                        175                 180                 185


            ggc aac ccc gat cct aca ctc aac aca acc tac ttg gag gtg ttg cga          720
            Gly Asn Pro Asp Pro Thr Leu Asn Thr Thr Tyr Leu Glu Val Leu Arg
            190                 195                 200                 205


            gca cgg tgt cct cag aac gca acc gga gat aac ctc acc aac ctc gac          768
            Ala Arg Cys Pro Gln Asn Ala Thr Gly Asp Asn Leu Thr Asn Leu Asp
                        210                 215                 220


            ctc tcg aca ccc gac cag ttc gac aac cgc tac tat tcg aac ttg ctc          816
            Leu Ser Thr Pro Asp Gln Phe Asp Asn Arg Tyr Tyr Ser Asn Leu Leu
                        225                 230                 235


            cag ctc aac ggt ctc ttg cag tcg gac cag gag ctc ttc tcg aca cct          864
            Gln Leu Asn Gly Leu Leu Gln Ser Asp Gln Glu Leu Phe Ser Thr Pro
                        240                 245                 250


            gga gcg gac act atc cct atc gtg aac tcc ttc tcg tcg aac cag aac          912
            Gly Ala Asp Thr Ile Pro Ile Val Asn Ser Phe Ser Ser Asn Gln Asn
            255                 260                 265


            acc ttc ttc tcg aac ttc cga gtc tcc atg atc aaa atg ggc aac att          960
            Thr Phe Phe Ser Asn Phe Arg Val Ser Met Ile Lys Met Gly Asn Ile
            270                 275                 280                 285


            gga gtc ttg aca ggt gat gag ggc gaa atc agg ctc cag tgt aac ttc         1008
            Gly Val Leu Thr Gly Asp Glu Gly Glu Ile Arg Leu Gln Cys Asn Phe
                        290                 295                 300


            gtg aac ggc gac tcg ttc ggt ttg gcc tcg gtc gcc tcg aag gat gcc         1056
            Val Asn Gly Asp Ser Phe Gly Leu Ala Ser Val Ala Ser Lys Asp Ala
                        305                 310                 315


            aag cag aag ctc gtc gcc cag tcc aaa tga                                 1086
            Lys Gln Lys Leu Val Ala Gln Ser Lys
                        320                 325
```

<210> 15
<211> 361
<212> PRT
<213> Artificial

<220>
<223> Synthetic Construct

<400> 15

Met Arg Leu Ser Thr Ser Ser Leu Phe Leu Ser Val Ser Leu Leu Gly

|     | -35 |     |     |     | -30 |     |     |     | -25 |     |     |     | -20 |
| --- | --- | --- | --- | --- | --- | --- | --- | --- | --- | --- | --- | --- | --- |

Lys Leu Ala Leu Gly Ser Pro Leu Pro Gln Gln Gln Arg Tyr Gly Lys
                  -15                  -10                -5

Arg Leu Gly Gln Leu Thr Pro Thr Phe Tyr Arg Glu Thr Cys Pro Asn
           -1  1         5                  10

Leu Phe Pro Ile Val Phe Gly Val Ile Phe Asp Ala Ser Phe Thr Asp
     15                20              25

Pro Arg Ile Gly Ala Ser Leu Met Arg Leu His Phe His Asp Cys Phe
30                 35             40              45

Val Gln Gly Cys Asp Gly Ser Val Leu Leu Asn Asn Thr Asp Thr Ile
           50             55              60

Glu Ser Glu Gln Asp Ala Leu Pro Asn Ile Asn Ser Ile Arg Gly Leu
         65            70              75

Asp Val Val Asn Asp Ile Lys Thr Ala Val Glu Asn Ser Cys Pro Asp
     80               85              90

Thr Val Ser Cys Ala Asp Ile Leu Ala Ile Ala Ala Glu Ile Ala Ser
     95             100          105

Val Leu Gly Gly Gly Pro Gly Trp Pro Val Pro Leu Gly Arg Arg Asp
110              115          120          125

Ser Leu Thr Ala Asn Arg Thr Leu Ala Asn Gln Asn Leu Pro Ala Pro
         130           135          140

Phe Phe Asn Leu Thr Gln Leu Lys Ala Ser Phe Ala Val Gln Gly Leu
       145           150          155

Asn Thr Leu Asp Leu Val Thr Leu Ser Gly Gly His Thr Phe Gly Arg
       160           165          170

Ala Arg Cys Ser Thr Phe Ile Asn Arg Leu Tyr Asn Phe Ser Asn Thr
     175            180          185

Gly Asn Pro Asp Pro Thr Leu Asn Thr Thr Tyr Leu Glu Val Leu Arg
190              195          200          205

Ala Arg Cys Pro Gln Asn Ala Thr Gly Asp Asn Leu Thr Asn Leu Asp
         210           215          220

```
Leu Ser Thr Pro Asp Gln Phe Asp Asn Arg Tyr Tyr Ser Asn Leu Leu
            225             230             235

Gln Leu Asn Gly Leu Leu Gln Ser Asp Gln Glu Leu Phe Ser Thr Pro
            240             245             250

Gly Ala Asp Thr Ile Pro Ile Val Asn Ser Phe Ser Ser Asn Gln Asn
            255             260             265

Thr Phe Phe Ser Asn Phe Arg Val Ser Met Ile Lys Met Gly Asn Ile
270             275             280             285

Gly Val Leu Thr Gly Asp Glu Gly Glu Ile Arg Leu Gln Cys Asn Phe
            290             295             300

Val Asn Gly Asp Ser Phe Gly Leu Ala Ser Val Ala Ser Lys Asp Ala
            305             310             315

Lys Gln Lys Leu Val Ala Gln Ser Lys
            320             325
```

<210> 16
<211> 1050
<212> DNA
<213> Artificial

<220>
<223> Artificial construct

<220>
<221> CDS
<222> (1)..(1047)

<220>
<221> sig_peptide
<222> (1)..(69)

<220>
<221> mat_peptide
<222> (70)..(1047)

<400> 16

```
atg aga tta tcg act tcg agt ctc ttc ctt tcc gtg tct ctg ctg ggg      48
Met Arg Leu Ser Thr Ser Ser Leu Phe Leu Ser Val Ser Leu Leu Gly
        -20             -15             -10

aag ctg gcc ctc ggc cta gga cag ctc aca ccc act ttc tac agg gaa      96
Lys Leu Ala Leu Gly Leu Gly Gln Leu Thr Pro Thr Phe Tyr Arg Glu
        -5              -1  1               5

acc tgt ccc aac ttg ttc ccc att gtg ttc ggc gtc atc ttc gat gcg     144
Thr Cys Pro Asn Leu Phe Pro Ile Val Phe Gly Val Ile Phe Asp Ala
10                  15              20                  25

tcg ttc acc gac ccc agg atc gga gcc tcg ctc atg cgc ctc cat ttc     192
```

```
        Ser Phe Thr Asp Pro Arg Ile Gly Ala Ser Leu Met Arg Leu His Phe
                        30                  35                  40

        cac gac tgt ttc gtc cag ggc tgt gac ggt tcc gtc ttg ttg aac aac    240
        His Asp Cys Phe Val Gln Gly Cys Asp Gly Ser Val Leu Leu Asn Asn
                        45                  50                  55

        acc gac acc atc gag tcc gag cag gac gcg ctc ccc aac atc aac tcc    288
        Thr Asp Thr Ile Glu Ser Glu Gln Asp Ala Leu Pro Asn Ile Asn Ser
                        60                  65                  70

        atc cga ggc ctc gat gtc gtg aac gac atc aaa acc gca gtg gaa aac    336
        Ile Arg Gly Leu Asp Val Val Asn Asp Ile Lys Thr Ala Val Glu Asn
                75                  80                  85

        tcc tgt ccc gat acg gtc tcc tgt gca gac atc ttg gcg att gca gcc    384
        Ser Cys Pro Asp Thr Val Ser Cys Ala Asp Ile Leu Ala Ile Ala Ala
        90                  95                  100                 105

        gag atc gca tcg gtc ctc gga ggc ggt cct ggc tgg cct gtg ccg ctc    432
        Glu Ile Ala Ser Val Leu Gly Gly Gly Pro Gly Trp Pro Val Pro Leu
                        110                 115                 120

        gga cga cgg gac tcg ttg aca gca aac agg acg ctc gca aac cag aac    480
        Gly Arg Arg Asp Ser Leu Thr Ala Asn Arg Thr Leu Ala Asn Gln Asn
                        125                 130                 135

        ttg cct gcg cct ttc ttc aac ctc acc cag ttg aag gcc tcc ttc gca    528
        Leu Pro Ala Pro Phe Phe Asn Leu Thr Gln Leu Lys Ala Ser Phe Ala
                        140                 145                 150

        gtc cag ggc ctc aac aca ctc gac ctc gtc aca ctc tcg gga ggt cac    576
        Val Gln Gly Leu Asn Thr Leu Asp Leu Val Thr Leu Ser Gly Gly His
                        155                 160                 165

        acc ttc gga cga gca cgc tgt tcg acc ttc att aac cgc ctc tac aac    624
        Thr Phe Gly Arg Ala Arg Cys Ser Thr Phe Ile Asn Arg Leu Tyr Asn
        170                 175                 180                 185

        ttc tcc aac acg ggc aac ccc gat cct aca ctc aac aca acc tac ttg    672
        Phe Ser Asn Thr Gly Asn Pro Asp Pro Thr Leu Asn Thr Thr Tyr Leu
                        190                 195                 200

        gag gtg ttg cga gca cgg tgt cct cag aac gca acc gga gat aac ctc    720
        Glu Val Leu Arg Ala Arg Cys Pro Gln Asn Ala Thr Gly Asp Asn Leu
                        205                 210                 215

        acc aac ctc gac ctc tcg aca ccc gac cag ttc gac aac cgc tac tat    768
        Thr Asn Leu Asp Leu Ser Thr Pro Asp Gln Phe Asp Asn Arg Tyr Tyr
                        220                 225                 230

        tcg aac ttg ctc cag ctc aac ggt ctc ttg cag tcg gac cag gag ctc    816
        Ser Asn Leu Leu Gln Leu Asn Gly Leu Leu Gln Ser Asp Gln Glu Leu
                        235                 240                 245

        ttc tcg aca cct gga gcg gac act atc cct atc gtg aac tcc ttc tcg    864
        Phe Ser Thr Pro Gly Ala Asp Thr Ile Pro Ile Val Asn Ser Phe Ser
        250                 255                 260                 265

        tcg aac cag aac acc ttc ttc tcg aac ttc cga gtc tcc atg atc aaa    912
        Ser Asn Gln Asn Thr Phe Phe Ser Asn Phe Arg Val Ser Met Ile Lys
                        270                 275                 280
```

```
atg ggc aac att gga gtc ttg aca ggt gat gag ggc gaa atc agg ctc      960
Met Gly Asn Ile Gly Val Leu Thr Gly Asp Glu Gly Glu Ile Arg Leu
            285                 290                 295

cag tgt aac ttc gtg aac ggc gac tcg ttc ggt ttg gcc tcg gtc gcc     1008
Gln Cys Asn Phe Val Asn Gly Asp Ser Phe Gly Leu Ala Ser Val Ala
            300                 305                 310

tcg aag gat gcc aag cag aag ctc gtc gcc cag tcc aaa tga             1050
Ser Lys Asp Ala Lys Gln Lys Leu Val Ala Gln Ser Lys
            315                 320                 325
```

<210> 17
<211> 349
<212> PRT
<213> Artificial

<220>
<223> Synthetic Construct

<400> 17

```
Met Arg Leu Ser Thr Ser Ser Leu Phe Leu Ser Val Ser Leu Leu Gly
            -20                 -15                 -10

Lys Leu Ala Leu Gly Leu Gly Gln Leu Thr Pro Thr Phe Tyr Arg Glu
             -5                 -1  1                  5

Thr Cys Pro Asn Leu Phe Pro Ile Val Phe Gly Val Ile Phe Asp Ala
10                  15                 20                      25

Ser Phe Thr Asp Pro Arg Ile Gly Ala Ser Leu Met Arg Leu His Phe
                30                  35                      40

His Asp Cys Phe Val Gln Gly Cys Asp Gly Ser Val Leu Leu Asn Asn
            45                  50                  55

Thr Asp Thr Ile Glu Ser Glu Gln Asp Ala Leu Pro Asn Ile Asn Ser
             60                  65                  70

Ile Arg Gly Leu Asp Val Val Asn Asp Ile Lys Thr Ala Val Glu Asn
             75                  80                  85

Ser Cys Pro Asp Thr Val Ser Cys Ala Asp Ile Leu Ala Ile Ala Ala
90                  95                  100                     105

Glu Ile Ala Ser Val Leu Gly Gly Gly Pro Gly Trp Pro Val Pro Leu
                110                 115                 120

Gly Arg Arg Asp Ser Leu Thr Ala Asn Arg Thr Leu Ala Asn Gln Asn
                125                 130                 135
```

48

```
Leu Pro Ala Pro Phe Phe Asn Leu Thr Gln Leu Lys Ala Ser Phe Ala
        140             145             150

Val Gln Gly Leu Asn Thr Leu Asp Leu Val Thr Leu Ser Gly Gly His
        155             160             165

Thr Phe Gly Arg Ala Arg Cys Ser Thr Phe Ile Asn Arg Leu Tyr Asn
170             175             180             185

Phe Ser Asn Thr Gly Asn Pro Asp Pro Thr Leu Asn Thr Thr Tyr Leu
            190             195             200

Glu Val Leu Arg Ala Arg Cys Pro Gln Asn Ala Thr Gly Asp Asn Leu
        205             210             215

Thr Asn Leu Asp Leu Ser Thr Pro Asp Gln Phe Asp Asn Arg Tyr Tyr
        220             225             230

Ser Asn Leu Leu Gln Leu Asn Gly Leu Leu Gln Ser Asp Gln Glu Leu
        235             240             245

Phe Ser Thr Pro Gly Ala Asp Thr Ile Pro Ile Val Asn Ser Phe Ser
250             255             260             265

Ser Asn Gln Asn Thr Phe Phe Ser Asn Phe Arg Val Ser Met Ile Lys
            270             275             280

Met Gly Asn Ile Gly Val Leu Thr Gly Asp Glu Gly Glu Ile Arg Leu
            285             290             295

Gln Cys Asn Phe Val Asn Gly Asp Ser Phe Gly Leu Ala Ser Val Ala
        300             305             310

Ser Lys Asp Ala Lys Gln Lys Leu Val Ala Gln Ser Lys
    315             320             325
```

<210> 18
<211> 1062
<212> DNA
<213> Artificial

<220>
<223> Artificial construct

<220>
<221> CDS
<222> (1)..(1059)

<220>
<221> sig_peptide

<222> (1)..(63)

<220>
<221> mat_peptide
<222> (82)..(1059)

<400> 18

```
atg aag cta ctc tct ctg acc ggt gtg gct ggt gtg ctt gcg act tgc      48
Met Lys Leu Leu Ser Leu Thr Gly Val Ala Gly Val Leu Ala Thr Cys
        -25                 -20                 -15

gtt gca gcc act cct ttg gtg aag cgc cta gga cag ctc aca ccc act      96
Val Ala Ala Thr Pro Leu Val Lys Arg Leu Gly Gln Leu Thr Pro Thr
    -10                 -5                  -1  1                 5

ttc tac agg gaa acc tgt ccc aac ttg ttc ccc att gtg ttc ggc gtc     144
Phe Tyr Arg Glu Thr Cys Pro Asn Leu Phe Pro Ile Val Phe Gly Val
                10                  15                  20

atc ttc gat gcg tcg ttc acc gac ccc agg atc gga gcc tcg ctc atg     192
Ile Phe Asp Ala Ser Phe Thr Asp Pro Arg Ile Gly Ala Ser Leu Met
            25                  30                  35

cgc ctc cat ttc cac gac tgt ttc gtc cag ggc tgt gac ggt tcc gtc     240
Arg Leu His Phe His Asp Cys Phe Val Gln Gly Cys Asp Gly Ser Val
            40                  45                  50

ttg ttg aac aac acc gac acc atc gag tcc gag cag gac gcg ctc ccc     288
Leu Leu Asn Asn Thr Asp Thr Ile Glu Ser Glu Gln Asp Ala Leu Pro
    55                  60                  65

aac atc aac tcc atc cga ggc ctc gat gtc gtg aac gac atc aaa acc     336
Asn Ile Asn Ser Ile Arg Gly Leu Asp Val Val Asn Asp Ile Lys Thr
70                  75                  80                  85

gca gtg gaa aac tcc tgt ccc gat acg gtc tcc tgt gca gac atc ttg     384
Ala Val Glu Asn Ser Cys Pro Asp Thr Val Ser Cys Ala Asp Ile Leu
                90                  95                  100

gcg att gca gcc gag atc gca tcg gtc ctc gga ggc ggt cct ggc tgg     432
Ala Ile Ala Ala Glu Ile Ala Ser Val Leu Gly Gly Gly Pro Gly Trp
            105                 110                 115

cct gtg ccg ctc gga cga cgg gac tcg ttg aca gca aac agg acg ctc     480
Pro Val Pro Leu Gly Arg Arg Asp Ser Leu Thr Ala Asn Arg Thr Leu
            120                 125                 130

gca aac cag aac ttg cct gcg cct ttc ttc aac ctc acc cag ttg aag     528
Ala Asn Gln Asn Leu Pro Ala Pro Phe Phe Asn Leu Thr Gln Leu Lys
    135                 140                 145

gcc tcc ttc gca gtc cag ggc ctc aac aca ctc gac ctc gtc aca ctc     576
Ala Ser Phe Ala Val Gln Gly Leu Asn Thr Leu Asp Leu Val Thr Leu
150                 155                 160                 165

tcg gga ggt cac acc ttc gga cga gca cgc tgt tcg acc ttc att aac     624
Ser Gly Gly His Thr Phe Gly Arg Ala Arg Cys Ser Thr Phe Ile Asn
                170                 175                 180

cgc ctc tac aac ttc tcc aac acg ggc aac ccc gat cct aca ctc aac     672
Arg Leu Tyr Asn Phe Ser Asn Thr Gly Asn Pro Asp Pro Thr Leu Asn
            185                 190                 195
```

```
aca acc tac ttg gag gtg ttg cga gca cgg tgt cct cag aac gca acc      720
Thr Thr Tyr Leu Glu Val Leu Arg Ala Arg Cys Pro Gln Asn Ala Thr
        200             205             210

gga gat aac ctc acc aac ctc gac ctc tcg aca ccc gac cag ttc gac      768
Gly Asp Asn Leu Thr Asn Leu Asp Leu Ser Thr Pro Asp Gln Phe Asp
    215             220             225

aac cgc tac tat tcg aac ttg ctc cag ctc aac ggt ctc ttg cag tcg      816
Asn Arg Tyr Tyr Ser Asn Leu Leu Gln Leu Asn Gly Leu Leu Gln Ser
230             235             240             245

gac cag gag ctc ttc tcg aca cct gga gcg gac act atc cct atc gtg      864
Asp Gln Glu Leu Phe Ser Thr Pro Gly Ala Asp Thr Ile Pro Ile Val
        250             255             260

aac tcc ttc tcg tcg aac cag aac acc ttc ttc tcg aac ttc cga gtc      912
Asn Ser Phe Ser Ser Asn Gln Asn Thr Phe Phe Ser Asn Phe Arg Val
        265             270             275

tcc atg atc aaa atg ggc aac att gga gtc ttg aca ggt gat gag ggc      960
Ser Met Ile Lys Met Gly Asn Ile Gly Val Leu Thr Gly Asp Glu Gly
        280             285             290

gaa atc agg ctc cag tgt aac ttc gtg aac ggc gac tcg ttc ggt ttg     1008
Glu Ile Arg Leu Gln Cys Asn Phe Val Asn Gly Asp Ser Phe Gly Leu
    295             300             305

gcc tcg gtc gcc tcg aag gat gcc aag cag aag ctc gtc gcc cag tcc     1056
Ala Ser Val Ala Ser Lys Asp Ala Lys Gln Lys Leu Val Ala Gln Ser
310             315             320             325

aaa tga                                                              1062
Lys
```

<210> 19
<211> 353
<212> PRT
<213> Artificial

<220>
<223> Synthetic Construct

<400> 19

```
Met Lys Leu Leu Ser Leu Thr Gly Val Ala Gly Val Leu Ala Thr Cys
    -25             -20             -15

Val Ala Ala Thr Pro Leu Val Lys Arg Leu Gly Gln Leu Thr Pro Thr
    -10             -5          -1  1               5

Phe Tyr Arg Glu Thr Cys Pro Asn Leu Phe Pro Ile Val Phe Gly Val
            10              15              20

Ile Phe Asp Ala Ser Phe Thr Asp Pro Arg Ile Gly Ala Ser Leu Met
        25              30              35
```

52

```
Arg Leu His Phe His Asp Cys Phe Val Gln Gly Cys Asp Gly Ser Val
        40              45              50

Leu Leu Asn Asn Thr Asp Thr Ile Glu Ser Glu Gln Asp Ala Leu Pro
        55              60              65

Asn Ile Asn Ser Ile Arg Gly Leu Asp Val Val Asn Asp Ile Lys Thr
70              75              80              85

Ala Val Glu Asn Ser Cys Pro Asp Thr Val Ser Cys Ala Asp Ile Leu
            90              95              100

Ala Ile Ala Ala Glu Ile Ala Ser Val Leu Gly Gly Gly Pro Gly Trp
        105             110             115

Pro Val Pro Leu Gly Arg Arg Asp Ser Leu Thr Ala Asn Arg Thr Leu
        120             125             130

Ala Asn Gln Asn Leu Pro Ala Pro Phe Phe Asn Leu Thr Gln Leu Lys
        135             140             145

Ala Ser Phe Ala Val Gln Gly Leu Asn Thr Leu Asp Leu Val Thr Leu
150             155             160             165

Ser Gly Gly His Thr Phe Gly Arg Ala Arg Cys Ser Thr Phe Ile Asn
            170             175             180

Arg Leu Tyr Asn Phe Ser Asn Thr Gly Asn Pro Asp Pro Thr Leu Asn
        185             190             195

Thr Thr Tyr Leu Glu Val Leu Arg Ala Arg Cys Pro Gln Asn Ala Thr
        200             205             210

Gly Asp Asn Leu Thr Asn Leu Asp Leu Ser Thr Pro Asp Gln Phe Asp
215             220             225

Asn Arg Tyr Tyr Ser Asn Leu Leu Gln Leu Asn Gly Leu Leu Gln Ser
230             235             240             245

Asp Gln Glu Leu Phe Ser Thr Pro Gly Ala Asp Thr Ile Pro Ile Val
            250             255             260

Asn Ser Phe Ser Ser Asn Gln Asn Thr Phe Phe Ser Asn Phe Arg Val
            265             270             275

Ser Met Ile Lys Met Gly Asn Ile Gly Val Leu Thr Gly Asp Glu Gly
        280             285             290
```

```
        Glu Ile Arg Leu Gln Cys Asn Phe Val Asn Gly Asp Ser Phe Gly Leu
            295                 300                 305

        Ala Ser Val Ala Ser Lys Asp Ala Lys Gln Lys Leu Val Ala Gln Ser
        310                 315                 320                 325

        Lys
```

<210> 20
<211> 1044
<212> DNA
<213> Artificial

<220>
<223> Artificial construct

<220>
<221> CDS
<222> (1)..(1041)

<220>
<221> sig_peptide
<222> (1)..(63)

<220>
<221> mat_peptide
<222> (64)..(1041)

<400> 20

```
atg aag cta ctc tct ctg acc ggt gtg gct ggt gtg ctt gcg act tgc       48
Met Lys Leu Leu Ser Leu Thr Gly Val Ala Gly Val Leu Ala Thr Cys
    -20                 -15                 -10

gtt gca gcc cta gga cag ctc aca ccc act ttc tac agg gaa acc tgt       96
Val Ala Ala Leu Gly Gln Leu Thr Pro Thr Phe Tyr Arg Glu Thr Cys
 -5                 -1   1                 5                   10

ccc aac ttg ttc ccc att gtg ttc ggc gtc atc ttc gat gcg tcg ttc      144
Pro Asn Leu Phe Pro Ile Val Phe Gly Val Ile Phe Asp Ala Ser Phe
            15                  20                  25

acc gac ccc agg atc gga gcc tcg ctc atg cgc ctc cat ttc cac gac      192
Thr Asp Pro Arg Ile Gly Ala Ser Leu Met Arg Leu His Phe His Asp
            30                  35                  40

tgt ttc gtc cag ggc tgt gac ggt tcc gtc ttg ttg aac aac acc gac      240
Cys Phe Val Gln Gly Cys Asp Gly Ser Val Leu Leu Asn Asn Thr Asp
        45                  50                  55

acc atc gag tcc gag cag gac gcg ctc ccc aac atc aac tcc atc cga      288
Thr Ile Glu Ser Glu Gln Asp Ala Leu Pro Asn Ile Asn Ser Ile Arg
60                  65                  70                  75

ggc ctc gat gtc gtg aac gac atc aaa acc gca gtg gaa aac tcc tgt      336
Gly Leu Asp Val Val Asn Asp Ile Lys Thr Ala Val Glu Asn Ser Cys
                80                  85                  90
```

```
ccc gat acg gtc tcc tgt gca gac atc ttg gcg att gca gcc gag atc          384
Pro Asp Thr Val Ser Cys Ala Asp Ile Leu Ala Ile Ala Ala Glu Ile
            95              100             105

gca tcg gtc ctc gga ggc ggt cct ggc tgg cct gtg ccg ctc gga cga          432
Ala Ser Val Leu Gly Gly Gly Pro Gly Trp Pro Val Pro Leu Gly Arg
            110             115             120

cgg gac tcg ttg aca gca aac agg acg ctc gca aac cag aac ttg cct          480
Arg Asp Ser Leu Thr Ala Asn Arg Thr Leu Ala Asn Gln Asn Leu Pro
            125             130             135

gcg cct ttc ttc aac ctc acc cag ttg aag gcc tcc ttc gca gtc cag          528
Ala Pro Phe Phe Asn Leu Thr Gln Leu Lys Ala Ser Phe Ala Val Gln
            140             145             150             155

ggc ctc aac aca ctc gac ctc gtc aca ctc tcg gga ggt cac acc ttc          576
Gly Leu Asn Thr Leu Asp Leu Val Thr Leu Ser Gly Gly His Thr Phe
            160             165             170

gga cga gca cgc tgt tcg acc ttc att aac cgc ctc tac aac ttc tcc          624
Gly Arg Ala Arg Cys Ser Thr Phe Ile Asn Arg Leu Tyr Asn Phe Ser
            175             180             185

aac acg ggc aac ccc gat cct aca ctc aac aca acc tac ttg gag gtg          672
Asn Thr Gly Asn Pro Asp Pro Thr Leu Asn Thr Thr Tyr Leu Glu Val
            190             195             200

ttg cga gca cgg tgt cct cag aac gca acc gga gat aac ctc acc aac          720
Leu Arg Ala Arg Cys Pro Gln Asn Ala Thr Gly Asp Asn Leu Thr Asn
            205             210             215

ctc gac ctc tcg aca ccc gac cag ttc gac aac cgc tac tat tcg aac          768
Leu Asp Leu Ser Thr Pro Asp Gln Phe Asp Asn Arg Tyr Tyr Ser Asn
220             225             230             235

ttg ctc cag ctc aac ggt ctc ttg cag tcg gac cag gag ctc ttc tcg          816
Leu Leu Gln Leu Asn Gly Leu Leu Gln Ser Asp Gln Glu Leu Phe Ser
            240             245             250

aca cct gga gcg gac act atc cct atc gtg aac tcc ttc tcg tcg aac          864
Thr Pro Gly Ala Asp Thr Ile Pro Ile Val Asn Ser Phe Ser Ser Asn
            255             260             265

cag aac acc ttc ttc tcg aac ttc cga gtc tcc atg atc aaa atg ggc          912
Gln Asn Thr Phe Phe Ser Asn Phe Arg Val Ser Met Ile Lys Met Gly
            270             275             280

aac att gga gtc ttg aca ggt gat gag ggc gaa atc agg ctc cag tgt          960
Asn Ile Gly Val Leu Thr Gly Asp Glu Gly Glu Ile Arg Leu Gln Cys
            285             290             295

aac ttc gtg aac ggc gac tcg ttc ggt ttg gcc tcg gtc gcc tcg aag          1008
Asn Phe Val Asn Gly Asp Ser Phe Gly Leu Ala Ser Val Ala Ser Lys
300             305             310             315

gat gcc aag cag aag ctc gtc gcc cag tcc aaa tga                          1044
Asp Ala Lys Gln Lys Leu Val Ala Gln Ser Lys
            320             325
```

<210> 21

<211> 347
<212> PRT
<213> Artificial

<220>
<223> Synthetic Construct

<400> 21

```
Met Lys Leu Leu Ser Leu Thr Gly Val Ala Gly Val Leu Ala Thr Cys
    -20                 -15                 -10

Val Ala Ala Leu Gly Gln Leu Thr Pro Thr Phe Tyr Arg Glu Thr Cys
 -5             -1  1             5                     10

Pro Asn Leu Phe Pro Ile Val Phe Gly Val Ile Phe Asp Ala Ser Phe
            15                  20              25

Thr Asp Pro Arg Ile Gly Ala Ser Leu Met Arg Leu His Phe His Asp
        30              35              40

Cys Phe Val Gln Gly Cys Asp Gly Ser Val Leu Leu Asn Asn Thr Asp
    45              50              55

Thr Ile Glu Ser Glu Gln Asp Ala Leu Pro Asn Ile Asn Ser Ile Arg
60              65              70                  75

Gly Leu Asp Val Val Asn Asp Ile Lys Thr Ala Val Glu Asn Ser Cys
            80              85                  90

Pro Asp Thr Val Ser Cys Ala Asp Ile Leu Ala Ile Ala Ala Glu Ile
        95              100             105

Ala Ser Val Leu Gly Gly Gly Pro Gly Trp Pro Val Pro Leu Gly Arg
        110             115             120

Arg Asp Ser Leu Thr Ala Asn Arg Thr Leu Ala Asn Gln Asn Leu Pro
    125             130             135

Ala Pro Phe Phe Asn Leu Thr Gln Leu Lys Ala Ser Phe Ala Val Gln
140             145             150             155

Gly Leu Asn Thr Leu Asp Leu Val Thr Leu Ser Gly Gly His Thr Phe
            160             165             170

Gly Arg Ala Arg Cys Ser Thr Phe Ile Asn Arg Leu Tyr Asn Phe Ser
        175             180             185

Asn Thr Gly Asn Pro Asp Pro Thr Leu Asn Thr Thr Tyr Leu Glu Val
        190             195             200
```

57

```
Leu Arg Ala Arg Cys Pro Gln Asn Ala Thr Gly Asp Asn Leu Thr Asn
    205             210             215

Leu Asp Leu Ser Thr Pro Asp Gln Phe Asp Asn Arg Tyr Tyr Ser Asn
220             225             230                 235

Leu Leu Gln Leu Asn Gly Leu Leu Gln Ser Asp Gln Glu Leu Phe Ser
                240             245                 250

Thr Pro Gly Ala Asp Thr Ile Pro Ile Val Asn Ser Phe Ser Ser Asn
            255             260             265

Gln Asn Thr Phe Phe Ser Asn Phe Arg Val Ser Met Ile Lys Met Gly
        270             275             280

Asn Ile Gly Val Leu Thr Gly Asp Glu Gly Glu Ile Arg Leu Gln Cys
    285             290             295

Asn Phe Val Asn Gly Asp Ser Phe Gly Leu Ala Ser Val Ala Ser Lys
300             305             310                 315

Asp Ala Lys Gln Lys Leu Val Ala Gln Ser Lys
            320             325
```

<210> 22
<211> 1026
<212> DNA
<213> Artificial

<220>
<223> Artificial construct

<220>
<221> CDS
<222> (1)..(1023)

<220>
<221> sig_peptide
<222> (1)..(75)

<220>
<221> mat_peptide
<222> (112)..(1023)

<400> 22

```
atg aag ttc ttc acc acc atc ctc agc acc gcc agc ctt gtt gct gct      48
Met Lys Phe Phe Thr Thr Ile Leu Ser Thr Ala Ser Leu Val Ala Ala
    -35             -30                 -25


ctc ccc gcc gct gtt gac tcg aac cat acc ccg gcc gct cct gaa ctt      96
Leu Pro Ala Ala Val Asp Ser Asn His Thr Pro Ala Ala Pro Glu Leu
    -20             -15                 -10
```

```
gtt gcc cgc cta gga gac ctc cag att gga ttc tat aac acc tcc tgt      144
Val Ala Arg Leu Gly Asp Leu Gln Ile Gly Phe Tyr Asn Thr Ser Cys
-5              -1  1                   5                   10

ccg acc gca gaa tcg ttg gtc cag cag gcg gtg gca gca gcc ttc gcg      192
Pro Thr Ala Glu Ser Leu Val Gln Gln Ala Val Ala Ala Ala Phe Ala
                15                  20                  25

aac aac tcc ggc att gcc cct ggc ctc atc cgc atg cac ttc cac gac      240
Asn Asn Ser Gly Ile Ala Pro Gly Leu Ile Arg Met His Phe His Asp
            30                  35                  40

tgt ttc gtc agg ggt tgt gac gcc tcc gtc ctc ttg gac tcg acc gcc      288
Cys Phe Val Arg Gly Cys Asp Ala Ser Val Leu Leu Asp Ser Thr Ala
        45                  50                  55

aac aac acg gca gaa aag gat gca atc ccc aac aac ccc tcg ctc agg      336
Asn Asn Thr Ala Glu Lys Asp Ala Ile Pro Asn Asn Pro Ser Leu Arg
60                  65                  70                  75

ggc ttc gag gtg atc acc gca gca aag tcg gca gtc gaa gcc gca tgt      384
Gly Phe Glu Val Ile Thr Ala Ala Lys Ser Ala Val Glu Ala Ala Cys
                80                  85                  90

ccg cag act gtg tcc tgt gcc gac att ctc gcc ttc gca gcc cga gac      432
Pro Gln Thr Val Ser Cys Ala Asp Ile Leu Ala Phe Ala Ala Arg Asp
            95                  100                 105

tcg gcg aac ttg gca ggc aac att act tac cag gtg ccg tcc gga cga      480
Ser Ala Asn Leu Ala Gly Asn Ile Thr Tyr Gln Val Pro Ser Gly Arg
            110                 115                 120

cga gac ggc aca gtg tcc ttg gca tcc gaa gcc aac gcg cag atc ccc      528
Arg Asp Gly Thr Val Ser Leu Ala Ser Glu Ala Asn Ala Gln Ile Pro
            125                 130                 135

tcc cct ctc ttc aac gcc aca cag ttg atc aac tcg ttc gcg aac aag      576
Ser Pro Leu Phe Asn Ala Thr Gln Leu Ile Asn Ser Phe Ala Asn Lys
140                 145                 150                 155

act ctc act gcc gac gaa atg gtc aca ttg tcc gga gcc cac tcg atc      624
Thr Leu Thr Ala Asp Glu Met Val Thr Leu Ser Gly Ala His Ser Ile
                160                 165                 170

ggc gtg gca cac tgt tcc tcg ttc acg aac cga ctc tac aac ttc aac      672
Gly Val Ala His Cys Ser Ser Phe Thr Asn Arg Leu Tyr Asn Phe Asn
            175                 180                 185

tcg ggc tcc ggc atc gac ccg aca ctc tcc cct tcg tac gca gca ctc      720
Ser Gly Ser Gly Ile Asp Pro Thr Leu Ser Pro Ser Tyr Ala Ala Leu
            190                 195                 200

ttg cgc aac aca tgt cct gcc aac tcc aca cgg ttc acg cct atc acc      768
Leu Arg Asn Thr Cys Pro Ala Asn Ser Thr Arg Phe Thr Pro Ile Thr
            205                 210                 215

gtg tcg ttg gac att atc acc ccg tcg gtc ttg gat aac atg tac tac      816
Val Ser Leu Asp Ile Ile Thr Pro Ser Val Leu Asp Asn Met Tyr Tyr
220                 225                 230                 235

acc ggt gtc cag ctc acc ttg gga ttg ctc acc tcg gat cag gca ctc      864
Thr Gly Val Gln Leu Thr Leu Gly Leu Leu Thr Ser Asp Gln Ala Leu
            240                 245                 250
```

```
gtg acg gaa gcc aac ttg tcc gca gcg gtg aaa gca aac gca atg aac          912
Val Thr Glu Ala Asn Leu Ser Ala Ala Val Lys Ala Asn Ala Met Asn
        255                 260                 265

ttg act gcg tgg gcg tcg aag ttc gcc cag gcc atg gtg aaa atg gga          960
Leu Thr Ala Trp Ala Ser Lys Phe Ala Gln Ala Met Val Lys Met Gly
        270                 275                 280

cag atc gaa gtc ctc acg ggt acc cag gga gag atc agg acc aac tgt         1008
Gln Ile Glu Val Leu Thr Gly Thr Gln Gly Glu Ile Arg Thr Asn Cys
        285                 290                 295

tcc gtg gtc aac tcc tga                                                  1026
Ser Val Val Asn Ser
300
```

<210> 23
<211> 341
<212> PRT
<213> Artificial

<220>
<223> Synthetic Construct

<400> 23

```
Met Lys Phe Phe Thr Thr Ile Leu Ser Thr Ala Ser Leu Val Ala Ala
    -35                 -30                 -25

Leu Pro Ala Ala Val Asp Ser Asn His Thr Pro Ala Ala Pro Glu Leu
    -20                 -15                 -10

Val Ala Arg Leu Gly Asp Leu Gln Ile Gly Phe Tyr Asn Thr Ser Cys
-5              -1  1           5                   10

Pro Thr Ala Glu Ser Leu Val Gln Gln Ala Val Ala Ala Phe Ala
            15                  20                  25

Asn Asn Ser Gly Ile Ala Pro Gly Leu Ile Arg Met His Phe His Asp
            30                  35                  40

Cys Phe Val Arg Gly Cys Asp Ala Ser Val Leu Leu Asp Ser Thr Ala
    45                  50                  55

Asn Asn Thr Ala Glu Lys Asp Ala Ile Pro Asn Asn Pro Ser Leu Arg
60                  65                  70                  75

Gly Phe Glu Val Ile Thr Ala Ala Lys Ser Ala Val Glu Ala Ala Cys
                80                  85                  90

Pro Gln Thr Val Ser Cys Ala Asp Ile Leu Ala Phe Ala Ala Arg Asp
            95                  100                 105
```

Ser Ala Asn Leu Ala Gly Asn Ile Thr Tyr Gln Val Pro Ser Gly Arg
        110             115             120

Arg Asp Gly Thr Val Ser Leu Ala Ser Glu Ala Asn Ala Gln Ile Pro
        125             130             135

Ser Pro Leu Phe Asn Ala Thr Gln Leu Ile Asn Ser Phe Ala Asn Lys
140             145             150             155

Thr Leu Thr Ala Asp Glu Met Val Thr Leu Ser Gly Ala His Ser Ile
                160             165             170

Gly Val Ala His Cys Ser Ser Phe Thr Asn Arg Leu Tyr Asn Phe Asn
            175             180             185

Ser Gly Ser Gly Ile Asp Pro Thr Leu Ser Pro Ser Tyr Ala Ala Leu
        190             195             200

Leu Arg Asn Thr Cys Pro Ala Asn Ser Thr Arg Phe Thr Pro Ile Thr
        205             210             215

Val Ser Leu Asp Ile Ile Thr Pro Ser Val Leu Asp Asn Met Tyr Tyr
220             225             230             235

Thr Gly Val Gln Leu Thr Leu Gly Leu Leu Thr Ser Asp Gln Ala Leu
            240             245             250

Val Thr Glu Ala Asn Leu Ser Ala Ala Val Lys Ala Asn Ala Met Asn
            255             260             265

Leu Thr Ala Trp Ala Ser Lys Phe Ala Gln Ala Met Val Lys Met Gly
            270             275             280

Gln Ile Glu Val Leu Thr Gly Thr Gln Gly Glu Ile Arg Thr Asn Cys
        285             290             295

Ser Val Val Asn Ser
300

<210> 24
<211> 990
<212> DNA
<213> Artificial

<220>
<223> Artificial construct

<220>

<221> CDS
<222> (1)..(987)

<220>
<221> sig_peptide
<222> (1)..(75)

<220>
<221> mat_peptide
<222> (76)..(987)

<400> 24

```
atg aag ttc ttc acc acc atc ctc agc acc gcc agc ctt gtt gct gct        48
Met Lys Phe Phe Thr Thr Ile Leu Ser Thr Ala Ser Leu Val Ala Ala
-25              -20              -15              -10

ctc ccc gcc gct gtt gac tcc cta gga gac ctc cag att gga ttc tat        96
Leu Pro Ala Ala Val Asp Ser Leu Gly Asp Leu Gln Ile Gly Phe Tyr
             -5               -1  1                   5

aac acc tcc tgt ccg acc gca gaa tcg ttg gtc cag cag gcg gtg gca       144
Asn Thr Ser Cys Pro Thr Ala Glu Ser Leu Val Gln Gln Ala Val Ala
             10                  15                  20

gca gcc ttc gcg aac aac tcc ggc att gcc cct ggc ctc atc cgc atg       192
Ala Ala Phe Ala Asn Asn Ser Gly Ile Ala Pro Gly Leu Ile Arg Met
         25              30                  35

cac ttc cac gac tgt ttc gtc agg ggt tgt gac gcc tcc gtc ctc ttg       240
His Phe His Asp Cys Phe Val Arg Gly Cys Asp Ala Ser Val Leu Leu
40                  45                  50                  55

gac tcg acc gcc aac aac acg gca gaa aag gat gca atc ccc aac aac       288
Asp Ser Thr Ala Asn Asn Thr Ala Glu Lys Asp Ala Ile Pro Asn Asn
                 60                  65                  70

ccc tcg ctc agg ggc ttc gag gtg atc acc gca gca aag tcg gca gtc       336
Pro Ser Leu Arg Gly Phe Glu Val Ile Thr Ala Ala Lys Ser Ala Val
             75                  80                  85

gaa gcc gca tgt ccg cag act gtg tcc tgt gcc gac att ctc gcc ttc       384
Glu Ala Ala Cys Pro Gln Thr Val Ser Cys Ala Asp Ile Leu Ala Phe
         90                  95                  100

gca gcc cga gac tcg gcg aac ttg gca ggc aac att act tac cag gtg       432
Ala Ala Arg Asp Ser Ala Asn Leu Ala Gly Asn Ile Thr Tyr Gln Val
         105             110                 115

ccg tcc gga cga cga gac ggc aca gtg tcc ttg gca tcc gaa gcc aac       480
Pro Ser Gly Arg Arg Asp Gly Thr Val Ser Leu Ala Ser Glu Ala Asn
120             125                 130                 135

gcg cag atc ccc tcc cct ctc ttc aac gcc aca cag ttg atc aac tcg       528
Ala Gln Ile Pro Ser Pro Leu Phe Asn Ala Thr Gln Leu Ile Asn Ser
             140                 145                 150

ttc gcg aac aag act ctc act gcc gac gaa atg gtc aca ttg tcc gga       576
Phe Ala Asn Lys Thr Leu Thr Ala Asp Glu Met Val Thr Leu Ser Gly
             155                 160                 165

gcc cac tcg atc ggc gtg gca cac tgt tcc tcg ttc acg aac cga ctc       624
Ala His Ser Ile Gly Val Ala His Cys Ser Ser Phe Thr Asn Arg Leu
         170                 175                 180
```

```
tac aac ttc aac tcg ggc tcc ggc atc gac ccg aca ctc tcc cct tcg          672
Tyr Asn Phe Asn Ser Gly Ser Gly Ile Asp Pro Thr Leu Ser Pro Ser
    185             190             195

tac gca gca ctc ttg cgc aac aca tgt cct gcc aac tcc aca cgg ttc          720
Tyr Ala Ala Leu Leu Arg Asn Thr Cys Pro Ala Asn Ser Thr Arg Phe
200             205             210             215

acg cct atc acc gtg tcg ttg gac att atc acc ccg tcg gtc ttg gat          768
Thr Pro Ile Thr Val Ser Leu Asp Ile Ile Thr Pro Ser Val Leu Asp
            220             225             230

aac atg tac tac acc ggt gtc cag ctc acc ttg gga ttg ctc acc tcg          816
Asn Met Tyr Tyr Thr Gly Val Gln Leu Thr Leu Gly Leu Leu Thr Ser
            235             240             245

gat cag gca ctc gtg acg gaa gcc aac ttg tcc gca gcg gtg aaa gca          864
Asp Gln Ala Leu Val Thr Glu Ala Asn Leu Ser Ala Ala Val Lys Ala
        250             255             260

aac gca atg aac ttg act gcg tgg gcg tcg aag ttc gcc cag gcc atg          912
Asn Ala Met Asn Leu Thr Ala Trp Ala Ser Lys Phe Ala Gln Ala Met
    265             270             275

gtg aaa atg gga cag atc gaa gtc ctc acg ggt acc cag gga gag atc          960
Val Lys Met Gly Gln Ile Glu Val Leu Thr Gly Thr Gln Gly Glu Ile
280             285             290             295

agg acc aac tgt tcc gtg gtc aac tcc tga                                  990
Arg Thr Asn Cys Ser Val Val Asn Ser
            300
```

<210> 25
<211> 329
<212> PRT
<213> Artificial

<220>
<223> Synthetic Construct

<400> 25

```
Met Lys Phe Phe Thr Thr Ile Leu Ser Thr Ala Ser Leu Val Ala Ala
-25             -20             -15             -10

Leu Pro Ala Ala Val Asp Ser Leu Gly Asp Leu Gln Ile Gly Phe Tyr
            -5              -1  1               5

Asn Thr Ser Cys Pro Thr Ala Glu Ser Leu Val Gln Gln Ala Val Ala
        10              15              20

Ala Ala Phe Ala Asn Asn Ser Gly Ile Ala Pro Gly Leu Ile Arg Met
    25              30              35

His Phe His Asp Cys Phe Val Arg Gly Cys Asp Ala Ser Val Leu Leu
40              45              50              55
```

```
Asp Ser Thr Ala Asn Asn Thr Ala Glu Lys Asp Ala Ile Pro Asn Asn
            60              65              70

Pro Ser Leu Arg Gly Phe Glu Val Ile Thr Ala Ala Lys Ser Ala Val
            75              80              85

Glu Ala Ala Cys Pro Gln Thr Val Ser Cys Ala Asp Ile Leu Ala Phe
            90              95              100

Ala Ala Arg Asp Ser Ala Asn Leu Ala Gly Asn Ile Thr Tyr Gln Val
            105             110             115

Pro Ser Gly Arg Arg Asp Gly Thr Val Ser Leu Ala Ser Glu Ala Asn
120             125             130             135

Ala Gln Ile Pro Ser Pro Leu Phe Asn Ala Thr Gln Leu Ile Asn Ser
            140             145             150

Phe Ala Asn Lys Thr Leu Thr Ala Asp Glu Met Val Thr Leu Ser Gly
            155             160             165

Ala His Ser Ile Gly Val Ala His Cys Ser Ser Phe Thr Asn Arg Leu
            170             175             180

Tyr Asn Phe Asn Ser Gly Ser Gly Ile Asp Pro Thr Leu Ser Pro Ser
            185             190             195

Tyr Ala Ala Leu Leu Arg Asn Thr Cys Pro Ala Asn Ser Thr Arg Phe
200             205             210             215

Thr Pro Ile Thr Val Ser Leu Asp Ile Ile Thr Pro Ser Val Leu Asp
            220             225             230

Asn Met Tyr Tyr Thr Gly Val Gln Leu Thr Leu Gly Leu Leu Thr Ser
            235             240             245

Asp Gln Ala Leu Val Thr Glu Ala Asn Leu Ser Ala Ala Val Lys Ala
            250             255             260

Asn Ala Met Asn Leu Thr Ala Trp Ala Ser Lys Phe Ala Gln Ala Met
            265             270             275

Val Lys Met Gly Gln Ile Glu Val Leu Thr Gly Thr Gln Gly Glu Ile
280             285             290             295

Arg Thr Asn Cys Ser Val Val Asn Ser
            300
```

<210> 26
<211> 1020
<212> DNA
<213> Artificial

<220>
<223> Artificial construct

<220>
<221> CDS
<222> (1)..(1017)

<220>
<221> sig_peptide
<222> (1)..(69)

<220>
<221> mat_peptide
<222> (106)..(1017)

<400> 26

```
atg aga tta tcg act tcg agt ctc ttc ctt tcc gtg tct ctg ctg ggg       48
Met Arg Leu Ser Thr Ser Ser Leu Phe Leu Ser Val Ser Leu Leu Gly
-35             -30             -25             -20

aag ctg gcc ctc ggg agc cct ttg ccc caa cag cag cga tat ggc aaa       96
Lys Leu Ala Leu Gly Ser Pro Leu Pro Gln Gln Gln Arg Tyr Gly Lys
            -15             -10             -5

cgc cta gga gac ctc cag att gga ttc tat aac acc tcc tgt ccg acc      144
Arg Leu Gly Asp Leu Gln Ile Gly Phe Tyr Asn Thr Ser Cys Pro Thr
        -1  1             5               10

gca gaa tcg ttg gtc cag cag gcg gtg gca gca gcc ttc gcg aac aac      192
Ala Glu Ser Leu Val Gln Gln Ala Val Ala Ala Ala Phe Ala Asn Asn
    15              20              25

tcc ggc att gcc cct ggc ctc atc cgc atg cac ttc cac gac tgt ttc      240
Ser Gly Ile Ala Pro Gly Leu Ile Arg Met His Phe His Asp Cys Phe
30              35              40              45

gtc agg ggt tgt gac gcc tcc gtc ctc ttg gac tcg acc gcc aac aac      288
Val Arg Gly Cys Asp Ala Ser Val Leu Leu Asp Ser Thr Ala Asn Asn
            50              55              60

acg gca gaa aag gat gca atc ccc aac aac ccc tcg ctc agg ggc ttc      336
Thr Ala Glu Lys Asp Ala Ile Pro Asn Asn Pro Ser Leu Arg Gly Phe
            65              70              75

gag gtg atc acc gca gca aag tcg gca gtc gaa gcc gca tgt ccg cag      384
Glu Val Ile Thr Ala Ala Lys Ser Ala Val Glu Ala Ala Cys Pro Gln
        80              85              90

act gtg tcc tgt gcc gac att ctc gcc ttc gca gcc cga gac tcg gcg      432
Thr Val Ser Cys Ala Asp Ile Leu Ala Phe Ala Ala Arg Asp Ser Ala
    95              100             105

aac ttg gca ggc aac att act tac cag gtg ccg tcc gga cga cga gac      480
Asn Leu Ala Gly Asn Ile Thr Tyr Gln Val Pro Ser Gly Arg Arg Asp
110             115             120             125
```

```
ggc aca gtg tcc ttg gca tcc gaa gcc aac gcg cag atc ccc tcc cct          528
Gly Thr Val Ser Leu Ala Ser Glu Ala Asn Ala Gln Ile Pro Ser Pro
                130             135             140

ctc ttc aac gcc aca cag ttg atc aac tcg ttc gcg aac aag act ctc          576
Leu Phe Asn Ala Thr Gln Leu Ile Asn Ser Phe Ala Asn Lys Thr Leu
                145             150             155

act gcc gac gaa atg gtc aca ttg tcc gga gcc cac tcg atc ggc gtg          624
Thr Ala Asp Glu Met Val Thr Leu Ser Gly Ala His Ser Ile Gly Val
                160             165             170

gca cac tgt tcc tcg ttc acg aac cga ctc tac aac ttc aac tcg ggc          672
Ala His Cys Ser Ser Phe Thr Asn Arg Leu Tyr Asn Phe Asn Ser Gly
                175             180             185

tcc ggc atc gac ccg aca ctc tcc cct tcg tac gca gca ctc ttg cgc          720
Ser Gly Ile Asp Pro Thr Leu Ser Pro Ser Tyr Ala Ala Leu Leu Arg
190             195             200             205

aac aca tgt cct gcc aac tcc aca cgg ttc acg cct atc acc gtg tcg          768
Asn Thr Cys Pro Ala Asn Ser Thr Arg Phe Thr Pro Ile Thr Val Ser
                210             215             220

ttg gac att atc acc ccg tcg gtc ttg gat aac atg tac tac acc ggt          816
Leu Asp Ile Ile Thr Pro Ser Val Leu Asp Asn Met Tyr Tyr Thr Gly
                225             230             235

gtc cag ctc acc ttg gga ttg ctc acc tcg gat cag gca ctc gtg acg          864
Val Gln Leu Thr Leu Gly Leu Leu Thr Ser Asp Gln Ala Leu Val Thr
                240             245             250

gaa gcc aac ttg tcc gca gcg gtg aaa gca aac gca atg aac ttg act          912
Glu Ala Asn Leu Ser Ala Ala Val Lys Ala Asn Ala Met Asn Leu Thr
                255             260             265

gcg tgg gcg tcg aag ttc gcc cag gcc atg gtg aaa atg gga cag atc          960
Ala Trp Ala Ser Lys Phe Ala Gln Ala Met Val Lys Met Gly Gln Ile
270             275             280             285

gaa gtc ctc acg ggt acc cag gga gag atc agg acc aac tgt tcc gtg          1008
Glu Val Leu Thr Gly Thr Gln Gly Glu Ile Arg Thr Asn Cys Ser Val
                290             295             300

gtc aac tcc tga                                                           1020
Val Asn Ser
```

<210> 27
<211> 339
<212> PRT
<213> Artificial

<220>
<223> Synthetic Construct

<400> 27

```
        Met Arg Leu Ser Thr Ser Ser Leu Phe Leu Ser Val Ser Leu Leu Gly
        -35             -30             -25             -20
```

```
       Lys Leu Ala Leu Gly Ser Pro Leu Pro Gln Gln Gln Arg Tyr Gly Lys
                       -15             -10                     -5

       Arg Leu Gly Asp Leu Gln Ile Gly Phe Tyr Asn Thr Ser Cys Pro Thr
               -1  1               5                   10

       Ala Glu Ser Leu Val Gln Gln Ala Val Ala Ala Ala Phe Ala Asn Asn
               15              20              25

       Ser Gly Ile Ala Pro Gly Leu Ile Arg Met His Phe His Asp Cys Phe
       30              35              40                      45

       Val Arg Gly Cys Asp Ala Ser Val Leu Leu Asp Ser Thr Ala Asn Asn
                       50              55                      60

       Thr Ala Glu Lys Asp Ala Ile Pro Asn Asn Pro Ser Leu Arg Gly Phe
                       65              70                  75

       Glu Val Ile Thr Ala Ala Lys Ser Ala Val Glu Ala Ala Cys Pro Gln
               80              85                  90

       Thr Val Ser Cys Ala Asp Ile Leu Ala Phe Ala Ala Arg Asp Ser Ala
               95              100                 105

       Asn Leu Ala Gly Asn Ile Thr Tyr Gln Val Pro Ser Gly Arg Arg Asp
       110             115                 120                     125

       Gly Thr Val Ser Leu Ala Ser Glu Ala Asn Ala Gln Ile Pro Ser Pro
                       130             135                 140

       Leu Phe Asn Ala Thr Gln Leu Ile Asn Ser Phe Ala Asn Lys Thr Leu
                       145             150                 155

       Thr Ala Asp Glu Met Val Thr Leu Ser Gly Ala His Ser Ile Gly Val
                       160             165                 170

       Ala His Cys Ser Ser Phe Thr Asn Arg Leu Tyr Asn Phe Asn Ser Gly
               175             180                 185

       Ser Gly Ile Asp Pro Thr Leu Ser Pro Ser Tyr Ala Ala Leu Leu Arg
       190             195                 200                     205

       Asn Thr Cys Pro Ala Asn Ser Thr Arg Phe Thr Pro Ile Thr Val Ser
                       210             215                 220

       Leu Asp Ile Ile Thr Pro Ser Val Leu Asp Asn Met Tyr Tyr Thr Gly
                       225             230                 235
```

```
        Val Gln Leu Thr Leu Gly Leu Leu Thr Ser Asp Gln Ala Leu Val Thr
                240                     245                 250


        Glu Ala Asn Leu Ser Ala Ala Val Lys Ala Asn Ala Met Asn Leu Thr
                255                     260                 265


        Ala Trp Ala Ser Lys Phe Ala Gln Ala Met Val Lys Met Gly Gln Ile
                270                     275                 280                 285


        Glu Val Leu Thr Gly Thr Gln Gly Glu Ile Arg Thr Asn Cys Ser Val
                        290                     295                 300


        Val Asn Ser
```

<210> 28
<211> 984
<212> DNA
<213> Artificial

<220>
<223> Artificial construct

<220>
<221> CDS
<222> (1)..(981)

<220>
<221> sig_peptide
<222> (1)..(69)

<220>
<221> mat_peptide
<222> (70)..(981)

<400> 28

```
atg aga tta tcg act tcg agt ctc ttc ctt tcc gtg tct ctg ctg ggg       48
Met Arg Leu Ser Thr Ser Ser Leu Phe Leu Ser Val Ser Leu Leu Gly
            -20                 -15                 -10

aag ctg gcc ctc ggc cta gga gac ctc cag att gga ttc tat aac acc       96
Lys Leu Ala Leu Gly Leu Gly Asp Leu Gln Ile Gly Phe Tyr Asn Thr
        -5                  -1  1                 5

tcc tgt ccg acc gca gaa tcg ttg gtc cag cag gcg gtg gca gca gcc      144
Ser Cys Pro Thr Ala Glu Ser Leu Val Gln Gln Ala Val Ala Ala Ala
10                      15                  20                  25

ttc gcg aac aac tcc ggc att gcc cct ggc ctc atc cgc atg cac ttc      192
Phe Ala Asn Asn Ser Gly Ile Ala Pro Gly Leu Ile Arg Met His Phe
                    30                  35                  40

cac gac tgt ttc gtc agg ggt tgt gac gcc tcc gtc ctc ttg gac tcg      240
His Asp Cys Phe Val Arg Gly Cys Asp Ala Ser Val Leu Leu Asp Ser
                45                  50                  55
```

```
acc gcc aac aac acg gca gaa aag gat gca atc ccc aac aac ccc tcg      288
Thr Ala Asn Asn Thr Ala Glu Lys Asp Ala Ile Pro Asn Asn Pro Ser
        60              65              70

ctc agg ggc ttc gag gtg atc acc gca gca aag tcg gca gtc gaa gcc      336
Leu Arg Gly Phe Glu Val Ile Thr Ala Ala Lys Ser Ala Val Glu Ala
    75              80              85

gca tgt ccg cag act gtg tcc tgt gcc gac att ctc gcc ttc gca gcc      384
Ala Cys Pro Gln Thr Val Ser Cys Ala Asp Ile Leu Ala Phe Ala Ala
90              95              100             105

cga gac tcg gcg aac ttg gca ggc aac att act tac cag gtg ccg tcc      432
Arg Asp Ser Ala Asn Leu Ala Gly Asn Ile Thr Tyr Gln Val Pro Ser
            110             115             120

gga cga cga gac ggc aca gtg tcc ttg gca tcc gaa gcc aac gcg cag      480
Gly Arg Arg Asp Gly Thr Val Ser Leu Ala Ser Glu Ala Asn Ala Gln
            125             130             135

atc ccc tcc cct ctc ttc aac gcc aca cag ttg atc aac tcg ttc gcg      528
Ile Pro Ser Pro Leu Phe Asn Ala Thr Gln Leu Ile Asn Ser Phe Ala
        140             145             150

aac aag act ctc act gcc gac gaa atg gtc aca ttg tcc gga gcc cac      576
Asn Lys Thr Leu Thr Ala Asp Glu Met Val Thr Leu Ser Gly Ala His
    155             160             165

tcg atc ggc gtg gca cac tgt tcc tcg ttc acg aac cga ctc tac aac      624
Ser Ile Gly Val Ala His Cys Ser Ser Phe Thr Asn Arg Leu Tyr Asn
170             175             180             185

ttc aac tcg ggc tcc ggc atc gac ccg aca ctc tcc cct tcg tac gca      672
Phe Asn Ser Gly Ser Gly Ile Asp Pro Thr Leu Ser Pro Ser Tyr Ala
            190             195             200

gca ctc ttg cgc aac aca tgt cct gcc aac tcc aca cgg ttc acg cct      720
Ala Leu Leu Arg Asn Thr Cys Pro Ala Asn Ser Thr Arg Phe Thr Pro
        205             210             215

atc acc gtg tcg ttg gac att atc acc ccg tcg gtc ttg gat aac atg      768
Ile Thr Val Ser Leu Asp Ile Ile Thr Pro Ser Val Leu Asp Asn Met
        220             225             230

tac tac acc ggt gtc cag ctc acc ttg gga ttg ctc acc tcg gat cag      816
Tyr Tyr Thr Gly Val Gln Leu Thr Leu Gly Leu Leu Thr Ser Asp Gln
235             240             245

gca ctc gtg acg gaa gcc aac ttg tcc gca gcg gtg aaa gca aac gca      864
Ala Leu Val Thr Glu Ala Asn Leu Ser Ala Ala Val Lys Ala Asn Ala
250             255             260             265

atg aac ttg act gcg tgg gcg tcg aag ttc gcc cag gcc atg gtg aaa      912
Met Asn Leu Thr Ala Trp Ala Ser Lys Phe Ala Gln Ala Met Val Lys
            270             275             280

atg gga cag atc gaa gtc ctc acg ggt acc cag gga gag atc agg acc      960
Met Gly Gln Ile Glu Val Leu Thr Gly Thr Gln Gly Glu Ile Arg Thr
            285             290             295

aac tgt tcc gtg gtc aac tcc tga                                      984
Asn Cys Ser Val Val Asn Ser
```

300

<210> 29
<211> 327
<212> PRT
<213> Artificial

<220>
<223> Synthetic Construct

<400> 29

```
Met Arg Leu Ser Thr Ser Ser Leu Phe Leu Ser Val Ser Leu Leu Gly
            -20                 -15                 -10

Lys Leu Ala Leu Gly Leu Gly Asp Leu Gln Ile Gly Phe Tyr Asn Thr
            -5              -1  1                  5

Ser Cys Pro Thr Ala Glu Ser Leu Val Gln Gln Ala Val Ala Ala Ala
10              15                  20                      25

Phe Ala Asn Asn Ser Gly Ile Ala Pro Gly Leu Ile Arg Met His Phe
            30                  35                  40

His Asp Cys Phe Val Arg Gly Cys Asp Ala Ser Val Leu Leu Asp Ser
            45                  50                  55

Thr Ala Asn Asn Thr Ala Glu Lys Asp Ala Ile Pro Asn Asn Pro Ser
            60                  65                  70

Leu Arg Gly Phe Glu Val Ile Thr Ala Ala Lys Ser Ala Val Glu Ala
    75                  80                  85

Ala Cys Pro Gln Thr Val Ser Cys Ala Asp Ile Leu Ala Phe Ala Ala
90              95                  100                     105

Arg Asp Ser Ala Asn Leu Ala Gly Asn Ile Thr Tyr Gln Val Pro Ser
            110                 115                 120

Gly Arg Arg Asp Gly Thr Val Ser Leu Ala Ser Glu Ala Asn Ala Gln
            125                 130                 135

Ile Pro Ser Pro Leu Phe Asn Ala Thr Gln Leu Ile Asn Ser Phe Ala
    140                 145                 150

Asn Lys Thr Leu Thr Ala Asp Glu Met Val Thr Leu Ser Gly Ala His
    155                 160                 165

Ser Ile Gly Val Ala His Cys Ser Ser Phe Thr Asn Arg Leu Tyr Asn
170                 175                 180                     185
```

```
Phe Asn Ser Gly Ser Gly Ile Asp Pro Thr Leu Ser Pro Ser Tyr Ala
            190             195             200

Ala Leu Leu Arg Asn Thr Cys Pro Ala Asn Ser Thr Arg Phe Thr Pro
            205             210             215

Ile Thr Val Ser Leu Asp Ile Ile Thr Pro Ser Val Leu Asp Asn Met
            220             225             230

Tyr Tyr Thr Gly Val Gln Leu Thr Leu Gly Leu Leu Thr Ser Asp Gln
    235             240             245

Ala Leu Val Thr Glu Ala Asn Leu Ser Ala Ala Val Lys Ala Asn Ala
250             255             260             265

Met Asn Leu Thr Ala Trp Ala Ser Lys Phe Ala Gln Ala Met Val Lys
            270             275             280

Met Gly Gln Ile Glu Val Leu Thr Gly Thr Gln Gly Glu Ile Arg Thr
            285             290             295

Asn Cys Ser Val Val Asn Ser
            300
```

<210> 30
<211> 1026
<212> DNA
<213> Artificial

<220>
<223> Artificial construct

<220>
<221> CDS
<222> (1)..(1023)

<220>
<221> sig_peptide
<222> (1)..(63)

<220>
<221> mat_peptide
<222> (112)..(1023)

<400> 30

```
atg aag ttc ttc acc acc atc ctc agc acc gcc agc ctt gtt gct gct          48
Met Lys Phe Phe Thr Thr Ile Leu Ser Thr Ala Ser Leu Val Ala Ala
    -35                     -30                 -25

ctc ccc gcc gct gtt gac tcg aac cat acc ccg gcc gct cct gaa ctt          96
Leu Pro Ala Ala Val Asp Ser Asn His Thr Pro Ala Ala Pro Glu Leu
    -20                 -15                 -10
```

```
gtt gcc cgc cta gga gac ctc cag att gga ttc tat aac acc tcc tgt        144
Val Ala Arg Leu Gly Asp Leu Gln Ile Gly Phe Tyr Asn Thr Ser Cys
-5              -1  1           5                   10

ccg acc gca gaa tcg ttg gtc cag cag gcg gtg gca gca gcc ttc gcg        192
Pro Thr Ala Glu Ser Leu Val Gln Gln Ala Val Ala Ala Ala Phe Ala
            15              20              25

aac aac tcc ggc att gcc cct ggc ctc atc cgc atg cac ttc cac gac        240
Asn Asn Ser Gly Ile Ala Pro Gly Leu Ile Arg Met His Phe His Asp
        30              35              40

tgt ttc gtc agg ggt tgt gac gcc tcc gtc ctc ttg gac tcg acc gcc        288
Cys Phe Val Arg Gly Cys Asp Ala Ser Val Leu Leu Asp Ser Thr Ala
        45              50              55

aac aac acg gca gaa aag gat gca atc ccc aac aac ccc tcg ctc agg        336
Asn Asn Thr Ala Glu Lys Asp Ala Ile Pro Asn Asn Pro Ser Leu Arg
60              65              70              75

ggc ttc gag gtg atc acc gca gca aag tcg gca gtc gaa gcc gca tgt        384
Gly Phe Glu Val Ile Thr Ala Ala Lys Ser Ala Val Glu Ala Ala Cys
            80              85              90

ccg cag act gtg tcc tgt gcc gac att ctc gcc ttc gca gcc cga gac        432
Pro Gln Thr Val Ser Cys Ala Asp Ile Leu Ala Phe Ala Ala Arg Asp
            95              100             105

tcg gcg aac ttg gca ggc aac att act tac cag gtg ccg tcc gga cga        480
Ser Ala Asn Leu Ala Gly Asn Ile Thr Tyr Gln Val Pro Ser Gly Arg
        110             115             120

cga gac ggc aca gtg tcc ttg gca tcc gaa gcc aac gcg cag atc ccc        528
Arg Asp Gly Thr Val Ser Leu Ala Ser Glu Ala Asn Ala Gln Ile Pro
        125             130             135

tcc cct ctc ttc aac gcc aca cag ttg atc aac tcg ttc gcg aac aag        576
Ser Pro Leu Phe Asn Ala Thr Gln Leu Ile Asn Ser Phe Ala Asn Lys
140             145             150             155

act ctc act gcc gac gaa atg gtc aca ttg tcc gga gcc cac tcg atc        624
Thr Leu Thr Ala Asp Glu Met Val Thr Leu Ser Gly Ala His Ser Ile
            160             165             170

ggc gtg gca cac tgt tcc tcg ttc acg aac cga ctc tac aac ttc aac        672
Gly Val Ala His Cys Ser Ser Phe Thr Asn Arg Leu Tyr Asn Phe Asn
            175             180             185

tcg ggc tcc ggc atc gac ccg aca ctc tcc cct tcg tac gca gca ctc        720
Ser Gly Ser Gly Ile Asp Pro Thr Leu Ser Pro Ser Tyr Ala Ala Leu
        190             195             200

ttg cgc aac aca tgt cct gcc aac tcc aca cgg ttc acg cct atc acc        768
Leu Arg Asn Thr Cys Pro Ala Asn Ser Thr Arg Phe Thr Pro Ile Thr
    205             210             215

gtg tcg ttg gac att atc acc ccg tcg gtc ttg gat aac atg tac tac        816
Val Ser Leu Asp Ile Ile Thr Pro Ser Val Leu Asp Asn Met Tyr Tyr
220             225             230             235

acc ggt gtc cag ctc acc ttg gga ttg ctc acc tcg gat cag gca ctc        864
Thr Gly Val Gln Leu Thr Leu Gly Leu Leu Thr Ser Asp Gln Ala Leu
```

```
                    240                         245                        250
gtg acg gaa gcc aac ttg tcc gca gcg gtg aaa gca aac gca atg aac        912
Val Thr Glu Ala Asn Leu Ser Ala Ala Val Lys Ala Asn Ala Met Asn
            255                     260                     265

ttg act gcg tgg gcg tcg aag ttc gcc cag gcc atg gtg aaa atg gga        960
Leu Thr Ala Trp Ala Ser Lys Phe Ala Gln Ala Met Val Lys Met Gly
            270                     275                     280

cag atc gaa gtc ctc acg ggt acc cag gga gag atc agg acc aac tgt       1008
Gln Ile Glu Val Leu Thr Gly Thr Gln Gly Glu Ile Arg Thr Asn Cys
            285                     290                     295

tcc gtg gtc aac tcc tga                                               1026
Ser Val Val Asn Ser
300
```

<210> 31
<211> 341
<212> PRT
<213> Artificial

<220>
<223> Synthetic Construct

<400> 31

```
Met Lys Phe Phe Thr Thr Ile Leu Ser Thr Ala Ser Leu Val Ala Ala
    -35                 -30                 -25

Leu Pro Ala Ala Val Asp Ser Asn His Thr Pro Ala Ala Pro Glu Leu
    -20                 -15                 -10

Val Ala Arg Leu Gly Asp Leu Gln Ile Gly Phe Tyr Asn Thr Ser Cys
-5            -1  1                 5                     10

Pro Thr Ala Glu Ser Leu Val Gln Gln Ala Val Ala Ala Phe Ala
            15                  20                  25

Asn Asn Ser Gly Ile Ala Pro Gly Leu Ile Arg Met His Phe His Asp
        30                  35                  40

Cys Phe Val Arg Gly Cys Asp Ala Ser Val Leu Leu Asp Ser Thr Ala
    45                  50                  55

Asn Asn Thr Ala Glu Lys Asp Ala Ile Pro Asn Asn Pro Ser Leu Arg
60                  65                  70                  75

Gly Phe Glu Val Ile Thr Ala Ala Lys Ser Ala Val Glu Ala Ala Cys
            80                  85                  90

Pro Gln Thr Val Ser Cys Ala Asp Ile Leu Ala Phe Ala Ala Arg Asp
            95                  100                 105
```

```
Ser Ala Asn Leu Ala Gly Asn Ile Thr Tyr Gln Val Pro Ser Gly Arg
        110                 115                 120

Arg Asp Gly Thr Val Ser Leu Ala Ser Glu Ala Asn Ala Gln Ile Pro
        125                 130                 135

Ser Pro Leu Phe Asn Ala Thr Gln Leu Ile Asn Ser Phe Ala Asn Lys
140                 145                 150                 155

Thr Leu Thr Ala Asp Glu Met Val Thr Leu Ser Gly Ala His Ser Ile
                160                 165                 170

Gly Val Ala His Cys Ser Ser Phe Thr Asn Arg Leu Tyr Asn Phe Asn
                175                 180                 185

Ser Gly Ser Gly Ile Asp Pro Thr Leu Ser Pro Ser Tyr Ala Ala Leu
        190                 195                 200

Leu Arg Asn Thr Cys Pro Ala Asn Ser Thr Arg Phe Thr Pro Ile Thr
        205                 210                 215

Val Ser Leu Asp Ile Ile Thr Pro Ser Val Leu Asp Asn Met Tyr Tyr
220                 225                 230                 235

Thr Gly Val Gln Leu Thr Leu Gly Leu Leu Thr Ser Asp Gln Ala Leu
                240                 245                 250

Val Thr Glu Ala Asn Leu Ser Ala Ala Val Lys Ala Asn Ala Met Asn
                255                 260                 265

Leu Thr Ala Trp Ala Ser Lys Phe Ala Gln Ala Met Val Lys Met Gly
        270                 275                 280

Gln Ile Glu Val Leu Thr Gly Thr Gln Gly Glu Ile Arg Thr Asn Cys
        285                 290                 295

Ser Val Val Asn Ser
300
```

<210> 32  
<211> 978  
<212> DNA  
<213> Artificial  

<220>  
<223> Artificial construct  

<220>  
<221> CDS

<222> (1)..(975)

<220>
<221> sig_peptide
<222> (1)..(63)

<220>
<221> mat_peptide
<222> (64)..(975)

<400> 32

```
atg aag cta ctc tct ctg acc ggt gtg gct ggt gtg ctt gcg act tgc        48
Met Lys Leu Leu Ser Leu Thr Gly Val Ala Gly Val Leu Ala Thr Cys
    -20             -15                 -10

gtt gca gcc cta gga gac ctc cag att gga ttc tat aac acc tcc tgt        96
Val Ala Ala Leu Gly Asp Leu Gln Ile Gly Phe Tyr Asn Thr Ser Cys
-5              -1  1           5                   10

ccg acc gca gaa tcg ttg gtc cag cag gcg gtg gca gca gcc ttc gcg       144
Pro Thr Ala Glu Ser Leu Val Gln Gln Ala Val Ala Ala Ala Phe Ala
            15                  20                  25

aac aac tcc ggc att gcc cct ggc ctc atc cgc atg cac ttc cac gac       192
Asn Asn Ser Gly Ile Ala Pro Gly Leu Ile Arg Met His Phe His Asp
            30              35                  40

tgt ttc gtc agg ggt tgt gac gcc tcc gtc ctc ttg gac tcg acc gcc       240
Cys Phe Val Arg Gly Cys Asp Ala Ser Val Leu Leu Asp Ser Thr Ala
        45              50                  55

aac aac acg gca gaa aag gat gca atc ccc aac aac ccc tcg ctc agg       288
Asn Asn Thr Ala Glu Lys Asp Ala Ile Pro Asn Asn Pro Ser Leu Arg
60                  65                  70                  75

ggc ttc gag gtg atc acc gca gca aag tcg gca gtc gaa gcc gca tgt       336
Gly Phe Glu Val Ile Thr Ala Ala Lys Ser Ala Val Glu Ala Ala Cys
                80                  85                  90

ccg cag act gtg tcc tgt gcc gac att ctc gcc ttc gca gcc cga gac       384
Pro Gln Thr Val Ser Cys Ala Asp Ile Leu Ala Phe Ala Ala Arg Asp
            95                  100                 105

tcg gcg aac ttg gca ggc aac att act tac cag gtg ccg tcc gga cga       432
Ser Ala Asn Leu Ala Gly Asn Ile Thr Tyr Gln Val Pro Ser Gly Arg
            110                 115                 120

cga gac ggc aca gtg tcc ttg gca tcc gaa gcc aac gcg cag atc ccc       480
Arg Asp Gly Thr Val Ser Leu Ala Ser Glu Ala Asn Ala Gln Ile Pro
            125                 130                 135

tcc cct ctc ttc aac gcc aca cag ttg atc aac tcg ttc gcg aac aag       528
Ser Pro Leu Phe Asn Ala Thr Gln Leu Ile Asn Ser Phe Ala Asn Lys
140                 145                 150                 155

act ctc act gcc gac gaa atg gtc aca ttg tcc gga gcc cac tcg atc       576
Thr Leu Thr Ala Asp Glu Met Val Thr Leu Ser Gly Ala His Ser Ile
                160                 165                 170

ggc gtg gca cac tgt tcc tcg ttc acg aac cga ctc tac aac ttc aac       624
Gly Val Ala His Cys Ser Ser Phe Thr Asn Arg Leu Tyr Asn Phe Asn
```

                    175                     180                     185

tcg ggc tcc ggc atc gac ccg aca ctc tcc cct tcg tac gca gca ctc          672
Ser Gly Ser Gly Ile Asp Pro Thr Leu Ser Pro Ser Tyr Ala Ala Leu
        190                 195                 200

ttg cgc aac aca tgt cct gcc aac tcc aca cgg ttc acg cct atc acc          720
Leu Arg Asn Thr Cys Pro Ala Asn Ser Thr Arg Phe Thr Pro Ile Thr
        205                 210                 215

gtg tcg ttg gac att atc acc ccg tcg gtc ttg gat aac atg tac tac          768
Val Ser Leu Asp Ile Ile Thr Pro Ser Val Leu Asp Asn Met Tyr Tyr
220                 225                 230                 235

acc ggt gtc cag ctc acc ttg gga ttg ctc acc tcg gat cag gca ctc          816
Thr Gly Val Gln Leu Thr Leu Gly Leu Leu Thr Ser Asp Gln Ala Leu
                240                 245                 250

gtg acg gaa gcc aac ttg tcc gca gcg gtg aaa gca aac gca atg aac          864
Val Thr Glu Ala Asn Leu Ser Ala Ala Val Lys Ala Asn Ala Met Asn
            255                 260                 265

ttg act gcg tgg gcg tcg aag ttc gcc cag gcc atg gtg aaa atg gga          912
Leu Thr Ala Trp Ala Ser Lys Phe Ala Gln Ala Met Val Lys Met Gly
            270                 275                 280

cag atc gaa gtc ctc acg ggt acc cag gga gag atc agg acc aac tgt          960
Gln Ile Glu Val Leu Thr Gly Thr Gln Gly Glu Ile Arg Thr Asn Cys
        285                 290                 295

tcc gtg gtc aac tcc tga                                                   978
Ser Val Val Asn Ser
300

<210> 33
<211> 325
<212> PRT
<213> Artificial

<220>
<223> Synthetic Construct

<400> 33

Met Lys Leu Leu Ser Leu Thr Gly Val Ala Gly Val Leu Ala Thr Cys
    -20                   -15                     -10

Val Ala Ala Leu Gly Asp Leu Gln Ile Gly Phe Tyr Asn Thr Ser Cys
  -5             -1  1            5                 10

Pro Thr Ala Glu Ser Leu Val Gln Gln Ala Val Ala Ala Ala Phe Ala
          15                20               25

Asn Asn Ser Gly Ile Ala Pro Gly Leu Ile Arg Met His Phe His Asp
          30                35               40

Cys Phe Val Arg Gly Cys Asp Ala Ser Val Leu Leu Asp Ser Thr Ala
     45                50               55

```
Asn Asn Thr Ala Glu Lys Asp Ala Ile Pro Asn Asn Pro Ser Leu Arg
60              65          70                  75

Gly Phe Glu Val Ile Thr Ala Ala Lys Ser Ala Val Glu Ala Ala Cys
            80          85                  90

Pro Gln Thr Val Ser Cys Ala Asp Ile Leu Ala Phe Ala Ala Arg Asp
            95          100             105

Ser Ala Asn Leu Ala Gly Asn Ile Thr Tyr Gln Val Pro Ser Gly Arg
        110             115             120

Arg Asp Gly Thr Val Ser Leu Ala Ser Glu Ala Asn Ala Gln Ile Pro
    125             130             135

Ser Pro Leu Phe Asn Ala Thr Gln Leu Ile Asn Ser Phe Ala Asn Lys
140             145             150             155

Thr Leu Thr Ala Asp Glu Met Val Thr Leu Ser Gly Ala His Ser Ile
            160             165             170

Gly Val Ala His Cys Ser Ser Phe Thr Asn Arg Leu Tyr Asn Phe Asn
        175             180             185

Ser Gly Ser Gly Ile Asp Pro Thr Leu Ser Pro Ser Tyr Ala Ala Leu
        190             195             200

Leu Arg Asn Thr Cys Pro Ala Asn Ser Thr Arg Phe Thr Pro Ile Thr
    205             210             215

Val Ser Leu Asp Ile Ile Thr Pro Ser Val Leu Asp Asn Met Tyr Tyr
220             225             230             235

Thr Gly Val Gln Leu Thr Leu Gly Leu Leu Thr Ser Asp Gln Ala Leu
            240             245             250

Val Thr Glu Ala Asn Leu Ser Ala Ala Val Lys Ala Asn Ala Met Asn
        255             260             265

Leu Thr Ala Trp Ala Ser Lys Phe Ala Gln Ala Met Val Lys Met Gly
        270             275             280

Gln Ile Glu Val Leu Thr Gly Thr Gln Gly Glu Ile Arg Thr Asn Cys
    285             290             295

Ser Val Val Asn Ser
```

85

300

<210> 34
<211> 993
<212> DNA
<213> Artificial

<220>
<223> Artificial construct

<220>
<221> CDS
<222> (1)..(990)

<220>
<221> sig_peptide
<222> (1)..(78)

<220>
<221> mat_peptide
<222> (79)..(990)

<400> 34

```
atg ggc tcc atg cga ttg ctc gtc gtc gca ctc ttg tgt gcc ttc gcc         48
Met Gly Ser Met Arg Leu Leu Val Val Ala Leu Leu Cys Ala Phe Ala
    -25             -20                 -15

atg cac gca ggt ttc tcg gtg tcg tat gcc gac ctc cag att gga ttc         96
Met His Ala Gly Phe Ser Val Ser Tyr Ala Asp Leu Gln Ile Gly Phe
-10             -5                 -1  1                 5

tat aac acc tcc tgt ccg acc gca gaa tcg ttg gtc cag cag gcg gtg        144
Tyr Asn Thr Ser Cys Pro Thr Ala Glu Ser Leu Val Gln Gln Ala Val
            10                 15                 20

gca gca gcc ttc gcg aac aac tcc ggc att gcc cct ggc ctc atc cgc        192
Ala Ala Ala Phe Ala Asn Asn Ser Gly Ile Ala Pro Gly Leu Ile Arg
        25                 30                 35

atg cac ttc cac gac tgt ttc gtc agg ggt tgt gac gcc tcc gtc ctc        240
Met His Phe His Asp Cys Phe Val Arg Gly Cys Asp Ala Ser Val Leu
    40                 45                 50

ttg gac tcg acc gcc aac aac acg gca gaa aag gat gca atc ccc aac        288
Leu Asp Ser Thr Ala Asn Asn Thr Ala Glu Lys Asp Ala Ile Pro Asn
55                 60                 65                 70

aac ccc tcg ctc agg ggc ttc gag gtg atc acc gca gca aag tcg gca        336
Asn Pro Ser Leu Arg Gly Phe Glu Val Ile Thr Ala Ala Lys Ser Ala
            75                 80                 85

gtc gaa gcc gca tgt ccg cag act gtg tcc tgt gcc gac att ctc gcc        384
Val Glu Ala Ala Cys Pro Gln Thr Val Ser Cys Ala Asp Ile Leu Ala
        90                 95                 100

ttc gca gcc cga gac tcg gcg aac ttg gca ggc aac att act tac cag        432
Phe Ala Ala Arg Asp Ser Ala Asn Leu Ala Gly Asn Ile Thr Tyr Gln
        105                110                115

gtg ccg tcc gga cga cga gac ggc aca gtg tcc ttg gca tcc gaa gcc        480
Val Pro Ser Gly Arg Arg Asp Gly Thr Val Ser Leu Ala Ser Glu Ala
```

|     |     | 120 |     |     |     |     | 125 |     |     |     |     |     | 130 |     |     |     |
|-----|-----|-----|-----|-----|-----|-----|-----|-----|-----|-----|-----|-----|-----|-----|-----|-----|

```
aac gcg cag atc ccc tcc cct ctc ttc aac gcc aca cag ttg atc aac      528
Asn Ala Gln Ile Pro Ser Pro Leu Phe Asn Ala Thr Gln Leu Ile Asn
135             140             145             150

tcg ttc gcg aac aag act ctc act gcc gac gaa atg gtc aca ttg tcc      576
Ser Phe Ala Asn Lys Thr Leu Thr Ala Asp Glu Met Val Thr Leu Ser
            155             160             165

gga gcc cac tcg atc ggc gtg gca cac tgt tcc tcg ttc acg aac cga      624
Gly Ala His Ser Ile Gly Val Ala His Cys Ser Ser Phe Thr Asn Arg
            170             175             180

ctc tac aac ttc aac tcg ggc tcc ggc atc gac ccg aca ctc tcc cct      672
Leu Tyr Asn Phe Asn Ser Gly Ser Gly Ile Asp Pro Thr Leu Ser Pro
            185             190             195

tcg tac gca gca ctc ttg cgc aac aca tgt cct gcc aac tcc aca cgg      720
Ser Tyr Ala Ala Leu Leu Arg Asn Thr Cys Pro Ala Asn Ser Thr Arg
        200             205             210

ttc acg cct atc acc gtg tcg ttg gac att atc acc ccg tcg gtc ttg      768
Phe Thr Pro Ile Thr Val Ser Leu Asp Ile Ile Thr Pro Ser Val Leu
215             220             225             230

gat aac atg tac tac acc ggt gtc cag ctc acc ttg gga ttg ctc acc      816
Asp Asn Met Tyr Tyr Thr Gly Val Gln Leu Thr Leu Gly Leu Leu Thr
            235             240             245

tcg gat cag gca ctc gtg acg gaa gcc aac ttg tcc gca gcg gtg aaa      864
Ser Asp Gln Ala Leu Val Thr Glu Ala Asn Leu Ser Ala Ala Val Lys
        250             255             260

gca aac gca atg aac ttg act gcg tgg gcg tcg aag ttc gcc cag gcc      912
Ala Asn Ala Met Asn Leu Thr Ala Trp Ala Ser Lys Phe Ala Gln Ala
        265             270             275

atg gtg aaa atg gga cag atc gaa gtc ctc acg ggt acc cag gga gag      960
Met Val Lys Met Gly Gln Ile Glu Val Leu Thr Gly Thr Gln Gly Glu
        280             285             290

atc agg acc aac tgt tcc gtg gtc aac tcc tga                          993
Ile Arg Thr Asn Cys Ser Val Val Asn Ser
295             300
```

<210> 35
<211> 330
<212> PRT
<213> Artificial

<220>
<223> Synthetic Construct

<400> 35

Met Gly Ser Met Arg Leu Leu Val Val Ala Leu Leu Cys Ala Phe Ala
    -25                 -20                 -15

Met His Ala Gly Phe Ser Val Ser Tyr Ala Asp Leu Gln Ile Gly Phe
-10                 -5              -1  1                   5

Tyr Asn Thr Ser Cys Pro Thr Ala Glu Ser Leu Val Gln Gln Ala Val
                10                  15                  20

Ala Ala Ala Phe Ala Asn Asn Ser Gly Ile Ala Pro Gly Leu Ile Arg
            25                  30                  35

Met His Phe His Asp Cys Phe Val Arg Gly Cys Asp Ala Ser Val Leu
        40                  45                  50

Leu Asp Ser Thr Ala Asn Asn Thr Ala Glu Lys Asp Ala Ile Pro Asn
55                  60                  65                  70

Asn Pro Ser Leu Arg Gly Phe Glu Val Ile Thr Ala Ala Lys Ser Ala
                75                  80                  85

Val Glu Ala Ala Cys Pro Gln Thr Val Ser Cys Ala Asp Ile Leu Ala
            90                  95                  100

Phe Ala Ala Arg Asp Ser Ala Asn Leu Ala Gly Asn Ile Thr Tyr Gln
        105                 110                 115

Val Pro Ser Gly Arg Arg Asp Gly Thr Val Ser Leu Ala Ser Glu Ala
    120                 125                 130

Asn Ala Gln Ile Pro Ser Pro Leu Phe Asn Ala Thr Gln Leu Ile Asn
135                 140                 145                 150

Ser Phe Ala Asn Lys Thr Leu Thr Ala Asp Glu Met Val Thr Leu Ser
                155                 160                 165

Gly Ala His Ser Ile Gly Val Ala His Cys Ser Ser Phe Thr Asn Arg
            170                 175                 180

Leu Tyr Asn Phe Asn Ser Gly Ser Gly Ile Asp Pro Thr Leu Ser Pro
        185                 190                 195

Ser Tyr Ala Ala Leu Leu Arg Asn Thr Cys Pro Ala Asn Ser Thr Arg
    200                 205                 210

Phe Thr Pro Ile Thr Val Ser Leu Asp Ile Ile Thr Pro Ser Val Leu
215                 220                 225                 230

Asp Asn Met Tyr Tyr Thr Gly Val Gln Leu Thr Leu Gly Leu Leu Thr
            235                 240                 245

Ser Asp Gln Ala Leu Val Thr Glu Ala Asn Leu Ser Ala Ala Val Lys

250          255          260

```
Ala Asn Ala Met Asn Leu Thr Ala Trp Ala Ser Lys Phe Ala Gln Ala
        265                 270             275

Met Val Lys Met Gly Gln Ile Glu Val Leu Thr Gly Thr Gln Gly Glu
        280                 285             290

Ile Arg Thr Asn Cys Ser Val Val Asn Ser
295                 300
```

<210> 36
<211> 31
<212> DNA
<213> Artificial

<220>
<223> Primer 1

<400> 36
tcctgaccta ggacagctca cacccacttt c      31

<210> 37
<211> 31
<212> DNA
<213> Artificial

<220>
<223> Primer 2

<400> 37
acaggtctta agtcatttgg actgggcgac g      31

<210> 38
<211> 37
<212> DNA
<213> Artificial

<220>
<223> Primer 3

<400> 38
tgcccgccta ggagacctcc agattggatt ctataac      37

<210> 39
<211> 37
<212> DNA
<213> Artificial

<220>
<223> Primer 4

<400> 39
atcatactta agttatcagg agttgaccac ggaacag      37

<210> 40
<211> 32
<212> DNA
<213> Artificial

<220>
<223> Primer 5

<400> 40
taatcctagg tcagctcaca cctaccttct ac          32

<210> 41
<211> 22
<212> DNA
<213> Artificial

<220>
<223> Primer 6

<400> 41
ggtaccctta agtcaaatcg ac          22

<210> 42
<211> 35
<212> DNA
<213> Artificial

<220>
<223> Primer 7

<400> 42
taatcctagg tgccggtctc aaagtgggat tctac          35

<210> 43
<211> 28
<212> DNA
<213> Artificial

<220>
<223> Primer 8

<400> 43
attacttaag tcagttggtt gccacgtg          28

<210> 44
<211> 1077
<212> DNA
<213> Populus sp.

<220>
<221> CDS
<222> (7)..(1068)

<400> 44

```
ggatcc atg gaa agg gtc ttc tcc ttc aaa atg atg atc gac aag gcc          48
       Met Glu Arg Val Phe Ser Phe Lys Met Met Ile Asp Lys Ala
       1               5                  10
```

```
ctc cac ccg ttg gtc gca tcg ctc ttc ttc gtg atc tgg ttc ggt ggc        96
Leu His Pro Leu Val Ala Ser Leu Phe Phe Val Ile Trp Phe Gly Gly
15            20                25                30

tcg ctc ccc tac gca tac gcc cag ctc aca cct acc ttc tac gac ggc        144
Ser Leu Pro Tyr Ala Tyr Ala Gln Leu Thr Pro Thr Phe Tyr Asp Gly
             35                40                45

acc tgt ccc aac gtg tcg acc atc att cgc ggt gtg ctc gca cag gcg        192
Thr Cys Pro Asn Val Ser Thr Ile Ile Arg Gly Val Leu Ala Gln Ala
             50                55                60

ttg cag acc gat ccg cga att ggc gca tcg ttg att cgg ttg cac ttc        240
Leu Gln Thr Asp Pro Arg Ile Gly Ala Ser Leu Ile Arg Leu His Phe
         65                70                75

cat gac tgt ttc gtc gat ggt tgt gac ggc tcg atc ctc ctc gat aac        288
His Asp Cys Phe Val Asp Gly Cys Asp Gly Ser Ile Leu Leu Asp Asn
     80                85                90

acg gac aca atc gag tcc gaa aaa gag gca gca ccc aac aac aac tcg        336
Thr Asp Thr Ile Glu Ser Glu Lys Glu Ala Ala Pro Asn Asn Asn Ser
95                100               105               110

gca agg ggc ttc gat gtc gtc gat aac atg aaa gcc gca gtc gag aac        384
Ala Arg Gly Phe Asp Val Val Asp Asn Met Lys Ala Ala Val Glu Asn
             115               120               125

gcc tgt ccg ggt atc gtc tcg tgt gcg gac atc ctc gcc att gca gcg        432
Ala Cys Pro Gly Ile Val Ser Cys Ala Asp Ile Leu Ala Ile Ala Ala
             130               135               140

gag gaa tcg gtg cgc ttg gca ggc ggt ccc tcc tgg acc gtc ccg ctc        480
Glu Glu Ser Val Arg Leu Ala Gly Gly Pro Ser Trp Thr Val Pro Leu
             145               150               155

gga cga cgg gat tcc ttg atc gca aac cga tcg gga gca aac tcc tcg        528
Gly Arg Arg Asp Ser Leu Ile Ala Asn Arg Ser Gly Ala Asn Ser Ser
         160               165               170

att cct gca ccc tcc gaa tcc ctc gca gtg ctc aaa tcg aag ttc gca        576
Ile Pro Ala Pro Ser Glu Ser Leu Ala Val Leu Lys Ser Lys Phe Ala
175               180               185               190

gcc gtc ggc ttg aac acg tcg tcc gac ttg gtc gcg ttg tcg gga gca        624
Ala Val Gly Leu Asn Thr Ser Ser Asp Leu Val Ala Leu Ser Gly Ala
             195               200               205

cat acg ttc ggt agg gca cag tgt ttg aac ttc att tcg agg ctc tac        672
His Thr Phe Gly Arg Ala Gln Cys Leu Asn Phe Ile Ser Arg Leu Tyr
             210               215               220

aac ttc tcg ggc tcg ggc aac ccc gac ccc aca ttg aac act act tac        720
Asn Phe Ser Gly Ser Gly Asn Pro Asp Pro Thr Leu Asn Thr Thr Tyr
             225               230               235

ctc gca gcg ctc cag cag ttg tgt ccg cag gga ggt aac cga tcc gtg        768
Leu Ala Ala Leu Gln Gln Leu Cys Pro Gln Gly Gly Asn Arg Ser Val
             240               245               250

ttg acc aac ctc gac cga aca aca ccc gac acc ttc gac ggc aac tac        816
Leu Thr Asn Leu Asp Arg Thr Thr Pro Asp Thr Phe Asp Gly Asn Tyr
255               260               265               270
```

```
ttc tcc aac ctc cag acc aac gaa ggc ttg ctc cag tcc gat cag gag      864
Phe Ser Asn Leu Gln Thr Asn Glu Gly Leu Leu Gln Ser Asp Gln Glu
            275                 280                 285

ttg ttc tcc aca aca gga gcc gac acg atc gcg att gtc aac aac ttc      912
Leu Phe Ser Thr Thr Gly Ala Asp Thr Ile Ala Ile Val Asn Asn Phe
            290                 295                 300

tcc tcc aac cag aca gcc ttc ttc gag tcc ttc gtc gtc tcg atg atc      960
Ser Ser Asn Gln Thr Ala Phe Phe Glu Ser Phe Val Val Ser Met Ile
            305                 310                 315

cgg atg gga aac atc tcg ccc ttg acc ggc acc gat ggt gaa att cgg     1008
Arg Met Gly Asn Ile Ser Pro Leu Thr Gly Thr Asp Gly Glu Ile Arg
            320                 325                 330

ttg aac tgt cga atc gtg aac aac tcc acc ggc tcc aac gcg ctc ctc     1056
Leu Asn Cys Arg Ile Val Asn Asn Ser Thr Gly Ser Asn Ala Leu Leu
335                 340                 345                 350

gtc tcg tcg att tgacttaag                                           1077
Val Ser Ser Ile
```

<210> 45
<211> 354
<212> PRT
<213> Populus sp.

<400> 45

```
Met Glu Arg Val Phe Ser Phe Lys Met Met Ile Asp Lys Ala Leu His
1               5               10                  15

Pro Leu Val Ala Ser Leu Phe Phe Val Ile Trp Phe Gly Gly Ser Leu
            20                  25                  30

Pro Tyr Ala Tyr Ala Gln Leu Thr Pro Thr Phe Tyr Asp Gly Thr Cys
            35              40                  45

Pro Asn Val Ser Thr Ile Ile Arg Gly Val Leu Ala Gln Ala Leu Gln
        50                  55                  60

Thr Asp Pro Arg Ile Gly Ala Ser Leu Ile Arg Leu His Phe His Asp
65                  70                  75                  80

Cys Phe Val Asp Gly Cys Asp Gly Ser Ile Leu Leu Asp Asn Thr Asp
                85                  90                  95

Thr Ile Glu Ser Glu Lys Glu Ala Ala Pro Asn Asn Asn Ser Ala Arg
            100                 105                 110

Gly Phe Asp Val Val Asp Asn Met Lys Ala Ala Val Glu Asn Ala Cys
            115                 120                 125
```

```
Pro Gly Ile Val Ser Cys Ala Asp Ile Leu Ala Ile Ala Ala Glu Glu
    130                 135             140

Ser Val Arg Leu Ala Gly Gly Pro Ser Trp Thr Val Pro Leu Gly Arg
    145             150             155                 160

Arg Asp Ser Leu Ile Ala Asn Arg Ser Gly Ala Asn Ser Ser Ile Pro
                165             170                 175

Ala Pro Ser Glu Ser Leu Ala Val Leu Lys Ser Lys Phe Ala Ala Val
            180             185                 190

Gly Leu Asn Thr Ser Ser Asp Leu Val Ala Leu Ser Gly Ala His Thr
        195             200             205

Phe Gly Arg Ala Gln Cys Leu Asn Phe Ile Ser Arg Leu Tyr Asn Phe
    210             215             220

Ser Gly Ser Gly Asn Pro Asp Pro Thr Leu Asn Thr Thr Tyr Leu Ala
225             230             235                 240

Ala Leu Gln Gln Leu Cys Pro Gln Gly Gly Asn Arg Ser Val Leu Thr
            245             250                 255

Asn Leu Asp Arg Thr Thr Pro Asp Thr Phe Asp Gly Asn Tyr Phe Ser
            260             265             270

Asn Leu Gln Thr Asn Glu Gly Leu Leu Gln Ser Asp Gln Glu Leu Phe
        275             280             285

Ser Thr Thr Gly Ala Asp Thr Ile Ala Ile Val Asn Asn Phe Ser Ser
    290             295             300

Asn Gln Thr Ala Phe Phe Glu Ser Phe Val Val Ser Met Ile Arg Met
305             310             315                 320

Gly Asn Ile Ser Pro Leu Thr Gly Thr Asp Gly Glu Ile Arg Leu Asn
            325             330             335

Cys Arg Ile Val Asn Asn Ser Thr Gly Ser Asn Ala Leu Leu Val Ser
            340             345             350

Ser Ile
```

<210> 46
<211> 1065
<212> DNA

<213> Artificial

<220>
<223> Artificial construct

<220>
<221> CDS
<222> (1)..(1062)

<400> 46


```
atg aag ttc ttc acc acc atc ctc agc acc gcc agc ctt gtt gct gct        48
Met Lys Phe Phe Thr Thr Ile Leu Ser Thr Ala Ser Leu Val Ala Ala
1               5                   10                  15

ctc ccc gcc gct gtt gac tcg aac cat acc ccg gcc gct cct gaa ctt        96
Leu Pro Ala Ala Val Asp Ser Asn His Thr Pro Ala Ala Pro Glu Leu
                20                  25                  30

gtt gcc cgc cta ggt cag ctc aca cct acc ttc tac gac ggc acc tgt       144
Val Ala Arg Leu Gly Gln Leu Thr Pro Thr Phe Tyr Asp Gly Thr Cys
            35                  40                  45

ccc aac gtg tcg acc atc att cgc ggt gtg ctc gca cag gcg ttg cag       192
Pro Asn Val Ser Thr Ile Ile Arg Gly Val Leu Ala Gln Ala Leu Gln
        50                  55                  60

acc gat ccg cga att ggc gca tcg ttg att cgg ttg cac ttc cat gac       240
Thr Asp Pro Arg Ile Gly Ala Ser Leu Ile Arg Leu His Phe His Asp
65                  70                  75                  80

tgt ttc gtc gat ggt tgt gac ggc tcg atc ctc ctc gat aac acg gac       288
Cys Phe Val Asp Gly Cys Asp Gly Ser Ile Leu Leu Asp Asn Thr Asp
                85                  90                  95

aca atc gag tcc gaa aaa gag gca gca ccc aac aac aac tcg gca agg       336
Thr Ile Glu Ser Glu Lys Glu Ala Ala Pro Asn Asn Asn Ser Ala Arg
                100                 105                 110

ggc ttc gat gtc gtc gat aac atg aaa gcc gca gtc gag aac gcc tgt       384
Gly Phe Asp Val Val Asp Asn Met Lys Ala Ala Val Glu Asn Ala Cys
            115                 120                 125

ccg ggt atc gtc tcg tgt gcg gac atc ctc gcc att gca gcg gag gaa       432
Pro Gly Ile Val Ser Cys Ala Asp Ile Leu Ala Ile Ala Ala Glu Glu
        130                 135                 140

tcg gtg cgc ttg gca ggc ggt ccc tcc tgg acc gtc ccg ctc gga cga       480
Ser Val Arg Leu Ala Gly Gly Pro Ser Trp Thr Val Pro Leu Gly Arg
145                 150                 155                 160

cgg gat tcc ttg atc gca aac cga tcg gga gca aac tcc tcg att cct       528
Arg Asp Ser Leu Ile Ala Asn Arg Ser Gly Ala Asn Ser Ser Ile Pro
                165                 170                 175

gca ccc tcc gaa tcc ctc gca gtg ctc aaa tcg aag ttc gca gcc gtc       576
Ala Pro Ser Glu Ser Leu Ala Val Leu Lys Ser Lys Phe Ala Ala Val
                180                 185                 190

ggc ttg aac acg tcg tcc gac ttg gtc gcg ttg tcg gga gca cat acg       624
Gly Leu Asn Thr Ser Ser Asp Leu Val Ala Leu Ser Gly Ala His Thr
```

195                         200                         205

```
ttc ggt agg gca cag tgt ttg aac ttc att tcg agg ctc tac aac ttc      672
Phe Gly Arg Ala Gln Cys Leu Asn Phe Ile Ser Arg Leu Tyr Asn Phe
    210             215             220

tcg ggc tcg ggc aac ccc gac ccc aca ttg aac act act tac ctc gca      720
Ser Gly Ser Gly Asn Pro Asp Pro Thr Leu Asn Thr Thr Tyr Leu Ala
225             230             235             240

gcg ctc cag cag ttg tgt ccg cag gga ggt aac cga tcc gtg ttg acc      768
Ala Leu Gln Gln Leu Cys Pro Gln Gly Gly Asn Arg Ser Val Leu Thr
        245             250             255

aac ctc gac cga aca aca ccc gac acc ttc gac ggc aac tac ttc tcc      816
Asn Leu Asp Arg Thr Thr Pro Asp Thr Phe Asp Gly Asn Tyr Phe Ser
        260             265             270

aac ctc cag acc aac gaa ggc ttg ctc cag tcc gat cag gag ttg ttc      864
Asn Leu Gln Thr Asn Glu Gly Leu Leu Gln Ser Asp Gln Glu Leu Phe
        275             280             285

tcc aca aca gga gcc gac acg atc gcg att gtc aac aac ttc tcc tcc      912
Ser Thr Thr Gly Ala Asp Thr Ile Ala Ile Val Asn Asn Phe Ser Ser
    290             295             300

aac cag aca gcc ttc ttc gag tcc ttc gtc gtc tcg atg atc cgg atg      960
Asn Gln Thr Ala Phe Phe Glu Ser Phe Val Val Ser Met Ile Arg Met
305             310             315             320

gga aac atc tcg ccc ttg acc ggc acc gat ggt gaa att cgg ttg aac      1008
Gly Asn Ile Ser Pro Leu Thr Gly Thr Asp Gly Glu Ile Arg Leu Asn
            325             330             335

tgt cga atc gtg aac aac tcc acc ggc tcc aac gcg ctc ctc gtc tcg      1056
Cys Arg Ile Val Asn Asn Ser Thr Gly Ser Asn Ala Leu Leu Val Ser
        340             345             350

tcg att tga                                                          1065
Ser Ile
```

<210> 47
<211> 354
<212> PRT
<213> Artificial

<220>
<223> Synthetic Construct

<400> 47

```
Met Lys Phe Phe Thr Thr Ile Leu Ser Thr Ala Ser Leu Val Ala Ala
1                   5                   10                  15

Leu Pro Ala Ala Val Asp Ser Asn His Thr Pro Ala Ala Pro Glu Leu
            20                  25                  30

Val Ala Arg Leu Gly Gln Leu Thr Pro Thr Phe Tyr Asp Gly Thr Cys
            35                  40                  45
```

```
Pro Asn Val Ser Thr Ile Ile Arg Gly Val Leu Ala Gln Ala Leu Gln
    50                  55                  60

Thr Asp Pro Arg Ile Gly Ala Ser Leu Ile Arg Leu His Phe His Asp
65                  70                  75                  80

Cys Phe Val Asp Gly Cys Asp Gly Ser Ile Leu Leu Asp Asn Thr Asp
                85                  90                  95

Thr Ile Glu Ser Glu Lys Glu Ala Ala Pro Asn Asn Asn Ser Ala Arg
            100                 105                 110

Gly Phe Asp Val Val Asp Asn Met Lys Ala Ala Val Glu Asn Ala Cys
        115                 120                 125

Pro Gly Ile Val Ser Cys Ala Asp Ile Leu Ala Ile Ala Ala Glu Glu
        130                 135                 140

Ser Val Arg Leu Ala Gly Gly Pro Ser Trp Thr Val Pro Leu Gly Arg
145                 150                 155                 160

Arg Asp Ser Leu Ile Ala Asn Arg Ser Gly Ala Asn Ser Ser Ile Pro
                165                 170                 175

Ala Pro Ser Glu Ser Leu Ala Val Leu Lys Ser Lys Phe Ala Ala Val
            180                 185                 190

Gly Leu Asn Thr Ser Ser Asp Leu Val Ala Leu Ser Gly Ala His Thr
        195                 200                 205

Phe Gly Arg Ala Gln Cys Leu Asn Phe Ile Ser Arg Leu Tyr Asn Phe
    210                 215                 220

Ser Gly Ser Gly Asn Pro Asp Pro Thr Leu Asn Thr Thr Tyr Leu Ala
225                 230                 235                 240

Ala Leu Gln Gln Leu Cys Pro Gln Gly Gly Asn Arg Ser Val Leu Thr
                245                 250                 255

Asn Leu Asp Arg Thr Thr Pro Asp Thr Phe Asp Gly Asn Tyr Phe Ser
            260                 265                 270

Asn Leu Gln Thr Asn Glu Gly Leu Leu Gln Ser Asp Gln Glu Leu Phe
        275                 280                 285

Ser Thr Thr Gly Ala Asp Thr Ile Ala Ile Val Asn Asn Phe Ser Ser
```

```
                    290                        295                          300


        Asn Gln Thr Ala Phe Phe Glu Ser Phe Val Val Ser Met Ile Arg Met
        305                 310                 315                 320


        Gly Asn Ile Ser Pro Leu Thr Gly Thr Asp Gly Glu Ile Arg Leu Asn
                        325                 330                 335


        Cys Arg Ile Val Asn Asn Ser Thr Gly Ser Asn Ala Leu Leu Val Ser
                    340                 345                 350


        Ser Ile
```

<210> 48
<211> 1029
<212> DNA
<213> Artificial

<220>
<223> Artificial construct

<220>
<221> CDS
<222> (1)..(1026)

<400> 48

```
atg aag ttc ttc acc acc atc ctc agc acc gcc agc ctt gtt gct gct        48
Met Lys Phe Phe Thr Thr Ile Leu Ser Thr Ala Ser Leu Val Ala Ala
1               5                   10                  15

ctc ccc gcc gct gtt gac tcc cta ggt cag ctc aca cct acc ttc tac        96
Leu Pro Ala Ala Val Asp Ser Leu Gly Gln Leu Thr Pro Thr Phe Tyr
            20                  25                  30

gac ggc acc tgt ccc aac gtg tcg acc atc att cgc ggt gtg ctc gca       144
Asp Gly Thr Cys Pro Asn Val Ser Thr Ile Ile Arg Gly Val Leu Ala
            35                  40                  45

cag gcg ttg cag acc gat ccg cga att ggc gca tcg ttg att cgg ttg       192
Gln Ala Leu Gln Thr Asp Pro Arg Ile Gly Ala Ser Leu Ile Arg Leu
        50                  55                  60

cac ttc cat gac tgt ttc gtc gat ggt tgt gac ggc tcg atc ctc ctc       240
His Phe His Asp Cys Phe Val Asp Gly Cys Asp Gly Ser Ile Leu Leu
65                  70                  75                  80

gat aac acg gac aca atc gag tcc gaa aaa gag gca gca ccc aac aac       288
Asp Asn Thr Asp Thr Ile Glu Ser Glu Lys Glu Ala Ala Pro Asn Asn
                85                  90                  95

aac tcg gca agg ggc ttc gat gtc gtc gat aac atg aaa gcc gca gtc       336
Asn Ser Ala Arg Gly Phe Asp Val Val Asp Asn Met Lys Ala Ala Val
            100                 105                 110

gag aac gcc tgt ccg ggt atc gtc tcg tgt gcg gac atc ctc gcc att       384
Glu Asn Ala Cys Pro Gly Ile Val Ser Cys Ala Asp Ile Leu Ala Ile
```

```
                 115                        120                        125

    gca gcg gag gaa tcg gtg cgc ttg gca ggc ggt ccc tcc tgg acc gtc        432
    Ala Ala Glu Glu Ser Val Arg Leu Ala Gly Gly Pro Ser Trp Thr Val
        130                     135                     140

    ccg ctc gga cga cgg gat tcc ttg atc gca aac cga tcg gga gca aac        480
    Pro Leu Gly Arg Arg Asp Ser Leu Ile Ala Asn Arg Ser Gly Ala Asn
    145                     150                     155                 160

    tcc tcg att cct gca ccc tcc gaa tcc ctc gca gtg ctc aaa tcg aag        528
    Ser Ser Ile Pro Ala Pro Ser Glu Ser Leu Ala Val Leu Lys Ser Lys
                        165                     170                     175

    ttc gca gcc gtc ggc ttg aac acg tcg tcc gac ttg gtc gcg ttg tcg        576
    Phe Ala Ala Val Gly Leu Asn Thr Ser Ser Asp Leu Val Ala Leu Ser
                    180                     185                     190

    gga gca cat acg ttc ggt agg gca cag tgt ttg aac ttc att tcg agg        624
    Gly Ala His Thr Phe Gly Arg Ala Gln Cys Leu Asn Phe Ile Ser Arg
                195                     200                     205

    ctc tac aac ttc tcg ggc tcg ggc aac ccc gac ccc aca ttg aac act        672
    Leu Tyr Asn Phe Ser Gly Ser Gly Asn Pro Asp Pro Thr Leu Asn Thr
            210                     215                     220

    act tac ctc gca gcg ctc cag cag ttg tgt ccg cag gga ggt aac cga        720
    Thr Tyr Leu Ala Ala Leu Gln Gln Leu Cys Pro Gln Gly Gly Asn Arg
    225                     230                     235                 240

    tcc gtg ttg acc aac ctc gac cga aca aca ccc gac acc ttc gac ggc        768
    Ser Val Leu Thr Asn Leu Asp Arg Thr Thr Pro Asp Thr Phe Asp Gly
                        245                     250                     255

    aac tac ttc tcc aac ctc cag acc aac gaa ggc ttg ctc cag tcc gat        816
    Asn Tyr Phe Ser Asn Leu Gln Thr Asn Glu Gly Leu Leu Gln Ser Asp
                        260                     265                     270

    cag gag ttg ttc tcc aca aca gga gcc gac acg atc gcg att gtc aac        864
    Gln Glu Leu Phe Ser Thr Thr Gly Ala Asp Thr Ile Ala Ile Val Asn
                275                     280                     285

    aac ttc tcc tcc aac cag aca gcc ttc ttc gag tcc ttc gtc gtc tcg        912
    Asn Phe Ser Ser Asn Gln Thr Ala Phe Phe Glu Ser Phe Val Val Ser
            290                     295                     300

    atg atc cgg atg gga aac atc tcg ccc ttg acc ggc acc gat ggt gaa        960
    Met Ile Arg Met Gly Asn Ile Ser Pro Leu Thr Gly Thr Asp Gly Glu
    305                     310                     315                 320

    att cgg ttg aac tgt cga atc gtg aac aac tcc acc ggc tcc aac gcg       1008
    Ile Arg Leu Asn Cys Arg Ile Val Asn Asn Ser Thr Gly Ser Asn Ala
                        325                     330                     335

    ctc ctc gtc tcg tcg att tga                                          1029
    Leu Leu Val Ser Ser Ile
                        340
```

<210> 49
<211> 342
<212> PRT

<213> Artificial

<220>
<223> Synthetic Construct

<400> 49

```
Met Lys Phe Phe Thr Thr Ile Leu Ser Thr Ala Ser Leu Val Ala Ala
1               5                   10                  15

Leu Pro Ala Ala Val Asp Ser Leu Gly Gln Leu Thr Pro Thr Phe Tyr
            20                  25              30

Asp Gly Thr Cys Pro Asn Val Ser Thr Ile Ile Arg Gly Val Leu Ala
            35                  40                  45

Gln Ala Leu Gln Thr Asp Pro Arg Ile Gly Ala Ser Leu Ile Arg Leu
    50                  55                  60

His Phe His Asp Cys Phe Val Asp Gly Cys Asp Gly Ser Ile Leu Leu
65                  70                  75                  80

Asp Asn Thr Asp Thr Ile Glu Ser Glu Lys Glu Ala Ala Pro Asn Asn
            85                  90                  95

Asn Ser Ala Arg Gly Phe Asp Val Val Asp Asn Met Lys Ala Ala Val
            100                 105                 110

Glu Asn Ala Cys Pro Gly Ile Val Ser Cys Ala Asp Ile Leu Ala Ile
            115                 120                 125

Ala Ala Glu Glu Ser Val Arg Leu Ala Gly Gly Pro Ser Trp Thr Val
    130                 135                 140

Pro Leu Gly Arg Arg Asp Ser Leu Ile Ala Asn Arg Ser Gly Ala Asn
145                 150                 155                 160

Ser Ser Ile Pro Ala Pro Ser Glu Ser Leu Ala Val Leu Lys Ser Lys
            165                 170                 175

Phe Ala Ala Val Gly Leu Asn Thr Ser Ser Asp Leu Val Ala Leu Ser
            180                 185                 190

Gly Ala His Thr Phe Gly Arg Ala Gln Cys Leu Asn Phe Ile Ser Arg
    195                 200                 205

Leu Tyr Asn Phe Ser Gly Ser Gly Asn Pro Asp Pro Thr Leu Asn Thr
210                 215                 220

Thr Tyr Leu Ala Ala Leu Gln Gln Leu Cys Pro Gln Gly Gly Asn Arg
```

```
                 225                    230                    235                    240


        Ser Val Leu Thr Asn Leu Asp Arg Thr Thr Pro Asp Thr Phe Asp Gly
                         245                    250                    255


        Asn Tyr Phe Ser Asn Leu Gln Thr Asn Glu Gly Leu Leu Gln Ser Asp
                         260                    265                    270


        Gln Glu Leu Phe Ser Thr Thr Gly Ala Asp Thr Ile Ala Ile Val Asn
                     275                    280                    285


        Asn Phe Ser Ser Asn Gln Thr Ala Phe Phe Glu Ser Phe Val Val Ser
                 290                    295                    300


        Met Ile Arg Met Gly Asn Ile Ser Pro Leu Thr Gly Thr Asp Gly Glu
        305                    310                    315                    320


        Ile Arg Leu Asn Cys Arg Ile Val Asn Asn Ser Thr Gly Ser Asn Ala
                         325                    330                    335


        Leu Leu Val Ser Ser Ile
                         340
```

<210> 50
<211> 1059
<212> DNA
<213> Artificial

<220>
<223> Artificial construct

<220>
<221> CDS
<222> (1)..(1056)

<400> 50

```
atg aga tta tcg act tcg agt ctc ttc ctt tcc gtg tct ctg ctg ggg       48
Met Arg Leu Ser Thr Ser Ser Leu Phe Leu Ser Val Ser Leu Leu Gly
1               5                   10                  15

aag ctg gcc ctc ggg agc cct ttg ccc caa cag cag cga tat ggc aaa       96
Lys Leu Ala Leu Gly Ser Pro Leu Pro Gln Gln Gln Arg Tyr Gly Lys
            20                  25                  30

cgc cta ggt cag ctc aca cct acc ttc tac gac ggc acc tgt ccc aac      144
Arg Leu Gly Gln Leu Thr Pro Thr Phe Tyr Asp Gly Thr Cys Pro Asn
            35                  40                  45

gtg tcg acc atc att cgc ggt gtg ctc gca cag gcg ttg cag acc gat      192
Val Ser Thr Ile Ile Arg Gly Val Leu Ala Gln Ala Leu Gln Thr Asp
            50                  55                  60

ccg cga att ggc gca tcg ttg att cgg ttg cac ttc cat gac tgt ttc      240
Pro Arg Ile Gly Ala Ser Leu Ile Arg Leu His Phe His Asp Cys Phe
```

65 70 75 80

```
gtc gat ggt tgt gac ggc tcg atc ctc ctc gat aac acg gac aca atc          288
Val Asp Gly Cys Asp Gly Ser Ile Leu Leu Asp Asn Thr Asp Thr Ile
            85                      90                  95

gag tcc gaa aaa gag gca gca ccc aac aac aac tcg gca agg ggc ttc          336
Glu Ser Glu Lys Glu Ala Ala Pro Asn Asn Asn Ser Ala Arg Gly Phe
                100                 105                 110

gat gtc gtc gat aac atg aaa gcc gca gtc gag aac gcc tgt ccg ggt          384
Asp Val Val Asp Asn Met Lys Ala Ala Val Glu Asn Ala Cys Pro Gly
        115                 120                 125

atc gtc tcg tgt gcg gac atc ctc gcc att gca gcg gag gaa tcg gtg          432
Ile Val Ser Cys Ala Asp Ile Leu Ala Ile Ala Ala Glu Glu Ser Val
        130                 135                 140

cgc ttg gca ggc ggt ccc tcc tgg acc gtc ccg ctc gga cga cgg gat          480
Arg Leu Ala Gly Gly Pro Ser Trp Thr Val Pro Leu Gly Arg Arg Asp
145                 150                 155                 160

tcc ttg atc gca aac cga tcg gga gca aac tcc tcg att cct gca ccc          528
Ser Leu Ile Ala Asn Arg Ser Gly Ala Asn Ser Ser Ile Pro Ala Pro
                165                 170                 175

tcc gaa tcc ctc gca gtg ctc aaa tcg aag ttc gca gcc gtc ggc ttg          576
Ser Glu Ser Leu Ala Val Leu Lys Ser Lys Phe Ala Ala Val Gly Leu
                180                 185                 190

aac acg tcg tcc gac ttg gtc gcg ttg tcg gga gca cat acg ttc ggt          624
Asn Thr Ser Ser Asp Leu Val Ala Leu Ser Gly Ala His Thr Phe Gly
        195                 200                 205

agg gca cag tgt ttg aac ttc att tcg agg ctc tac aac ttc tcg ggc          672
Arg Ala Gln Cys Leu Asn Phe Ile Ser Arg Leu Tyr Asn Phe Ser Gly
        210                 215                 220

tcg ggc aac ccc gac ccc aca ttg aac act act tac ctc gca gcg ctc          720
Ser Gly Asn Pro Asp Pro Thr Leu Asn Thr Thr Tyr Leu Ala Ala Leu
225                 230                 235                 240

cag cag ttg tgt ccg cag gga ggt aac cga tcc gtg ttg acc aac ctc          768
Gln Gln Leu Cys Pro Gln Gly Gly Asn Arg Ser Val Leu Thr Asn Leu
                245                 250                 255

gac cga aca aca ccc gac acc ttc gac ggc aac tac ttc tcc aac ctc          816
Asp Arg Thr Thr Pro Asp Thr Phe Asp Gly Asn Tyr Phe Ser Asn Leu
                260                 265                 270

cag acc aac gaa ggc ttg ctc cag tcc gat cag gag ttg ttc tcc aca          864
Gln Thr Asn Glu Gly Leu Leu Gln Ser Asp Gln Glu Leu Phe Ser Thr
                275                 280                 285

aca gga gcc gac acg atc gcg att gtc aac aac ttc tcc tcc aac cag          912
Thr Gly Ala Asp Thr Ile Ala Ile Val Asn Asn Phe Ser Ser Asn Gln
        290                 295                 300

aca gcc ttc ttc gag tcc ttc gtc gtc tcg atg atc cgg atg gga aac          960
Thr Ala Phe Phe Glu Ser Phe Val Val Ser Met Ile Arg Met Gly Asn
305                 310                 315                 320

atc tcg ccc ttg acc ggc acc gat ggt gaa att cgg ttg aac tgt cga          1008
```

```
    Ile Ser Pro Leu Thr Gly Thr Asp Gly Glu Ile Arg Leu Asn Cys Arg
                325                 330                 335

    atc gtg aac aac tcc acc ggc tcc aac gcg ctc ctc gtc tcg tcg att    1056
    Ile Val Asn Asn Ser Thr Gly Ser Asn Ala Leu Leu Val Ser Ser Ile
                340                 345                 350

    tga                                                                 1059
```

<210> 51
<211> 352
<212> PRT
<213> Artificial

<220>
<223> Synthetic Construct

<400> 51

```
Met Arg Leu Ser Thr Ser Ser Leu Phe Leu Ser Val Ser Leu Leu Gly
1           5               10              15

Lys Leu Ala Leu Gly Ser Pro Leu Pro Gln Gln Gln Arg Tyr Gly Lys
        20              25              30

Arg Leu Gly Gln Leu Thr Pro Thr Phe Tyr Asp Gly Thr Cys Pro Asn
        35              40              45

Val Ser Thr Ile Ile Arg Gly Val Leu Ala Gln Ala Leu Gln Thr Asp
    50              55              60

Pro Arg Ile Gly Ala Ser Leu Ile Arg Leu His Phe His Asp Cys Phe
65              70              75              80

Val Asp Gly Cys Asp Gly Ser Ile Leu Leu Asp Asn Thr Asp Thr Ile
            85              90              95

Glu Ser Glu Lys Glu Ala Ala Pro Asn Asn Asn Ser Ala Arg Gly Phe
        100             105             110

Asp Val Val Asp Asn Met Lys Ala Ala Val Glu Asn Ala Cys Pro Gly
        115             120             125

Ile Val Ser Cys Ala Asp Ile Leu Ala Ile Ala Ala Glu Glu Ser Val
    130             135             140

Arg Leu Ala Gly Gly Pro Ser Trp Thr Val Pro Leu Gly Arg Arg Asp
145             150             155             160

Ser Leu Ile Ala Asn Arg Ser Gly Ala Asn Ser Ser Ile Pro Ala Pro
            165             170             175
```

```
        Ser Glu Ser Leu Ala Val Leu Lys Ser Lys Phe Ala Ala Val Gly Leu
                    180                 185                 190

        Asn Thr Ser Ser Asp Leu Val Ala Leu Ser Gly Ala His Thr Phe Gly
                    195                 200                 205

        Arg Ala Gln Cys Leu Asn Phe Ile Ser Arg Leu Tyr Asn Phe Ser Gly
                    210                 215                 220

        Ser Gly Asn Pro Asp Pro Thr Leu Asn Thr Thr Tyr Leu Ala Ala Leu
        225                 230                 235                 240

        Gln Gln Leu Cys Pro Gln Gly Gly Asn Arg Ser Val Leu Thr Asn Leu
                    245                 250                 255

        Asp Arg Thr Thr Pro Asp Thr Phe Asp Gly Asn Tyr Phe Ser Asn Leu
                    260                 265                 270

        Gln Thr Asn Glu Gly Leu Leu Gln Ser Asp Gln Glu Leu Phe Ser Thr
                    275                 280                 285

        Thr Gly Ala Asp Thr Ile Ala Ile Val Asn Asn Phe Ser Ser Asn Gln
                    290                 295                 300

        Thr Ala Phe Phe Glu Ser Phe Val Val Ser Met Ile Arg Met Gly Asn
        305                 310                 315                 320

        Ile Ser Pro Leu Thr Gly Thr Asp Gly Glu Ile Arg Leu Asn Cys Arg
                    325                 330                 335

        Ile Val Asn Asn Ser Thr Gly Ser Asn Ala Leu Leu Val Ser Ser Ile
                    340                 345                 350
```

<210> 52
<211> 1035
<212> DNA
<213> Artificial

<220>
<223> Artificial construct

<220>
<221> CDS
<222> (1)..(1032)

<400> 52

```
atg aag cta ctc tct ctg acc ggt gtg gct ggt gtg ctt gcg act tgc      48
Met Lys Leu Leu Ser Leu Thr Gly Val Ala Gly Val Leu Ala Thr Cys
1               5                   10                  15
```

```
gtt gca gcc act cct ttg gtg aag cgc cta ggt cag ctc aca cct acc          96
Val Ala Ala Thr Pro Leu Val Lys Arg Leu Gly Gln Leu Thr Pro Thr
            20              25              30

ttc tac gac ggc acc tgt ccc aac gtg tcg acc atc att cgc ggt gtg         144
Phe Tyr Asp Gly Thr Cys Pro Asn Val Ser Thr Ile Ile Arg Gly Val
        35              40              45

ctc gca cag gcg ttg cag acc gat ccg cga att ggc gca tcg ttg att         192
Leu Ala Gln Ala Leu Gln Thr Asp Pro Arg Ile Gly Ala Ser Leu Ile
        50              55              60

cgg ttg cac ttc cat gac tgt ttc gtc gat ggt tgt gac ggc tcg atc         240
Arg Leu His Phe His Asp Cys Phe Val Asp Gly Cys Asp Gly Ser Ile
65              70              75              80

ctc ctc gat aac acg gac aca atc gag tcc gaa aaa gag gca gca ccc         288
Leu Leu Asp Asn Thr Asp Thr Ile Glu Ser Glu Lys Glu Ala Ala Pro
                85              90              95

aac aac aac tcg gca agg ggc ttc gat gtc gtc gat aac atg aaa gcc         336
Asn Asn Asn Ser Ala Arg Gly Phe Asp Val Val Asp Asn Met Lys Ala
            100             105             110

gca gtc gag aac gcc tgt ccg ggt atc gtc tcg tgt gcg gac atc ctc         384
Ala Val Glu Asn Ala Cys Pro Gly Ile Val Ser Cys Ala Asp Ile Leu
        115             120             125

gcc att gca gcg gag gaa tcg gtg cgc ttg gca ggc ggt ccc tcc tgg         432
Ala Ile Ala Ala Glu Glu Ser Val Arg Leu Ala Gly Gly Pro Ser Trp
    130             135             140

acc gtc ccg ctc gga cga cgg gat tcc ttg atc gca aac cga tcg gga         480
Thr Val Pro Leu Gly Arg Arg Asp Ser Leu Ile Ala Asn Arg Ser Gly
145             150             155             160

gca aac tcc tcg att cct gca ccc tcc gaa tcc ctc gca gtg ctc aaa         528
Ala Asn Ser Ser Ile Pro Ala Pro Ser Glu Ser Leu Ala Val Leu Lys
            165             170             175

tcg aag ttc gca gcc gtc ggc ttg aac acg tcg tcc gac ttg gtc gcg         576
Ser Lys Phe Ala Ala Val Gly Leu Asn Thr Ser Ser Asp Leu Val Ala
            180             185             190

ttg tcg gga gca cat acg ttc ggt agg gca cag tgt ttg aac ttc att         624
Leu Ser Gly Ala His Thr Phe Gly Arg Ala Gln Cys Leu Asn Phe Ile
            195             200             205

tcg agg ctc tac aac ttc tcg ggc tcg ggc aac ccc gac ccc aca ttg         672
Ser Arg Leu Tyr Asn Phe Ser Gly Ser Gly Asn Pro Asp Pro Thr Leu
            210             215             220

aac act act tac ctc gca gcg ctc cag cag ttg tgt ccg cag gga ggt         720
Asn Thr Thr Tyr Leu Ala Ala Leu Gln Gln Leu Cys Pro Gln Gly Gly
225             230             235             240

aac cga tcc gtg ttg acc aac ctc gac cga aca aca ccc gac acc ttc         768
Asn Arg Ser Val Leu Thr Asn Leu Asp Arg Thr Thr Pro Asp Thr Phe
            245             250             255

gac ggc aac tac ttc tcc aac ctc cag acc aac gaa ggc ttg ctc cag         816
Asp Gly Asn Tyr Phe Ser Asn Leu Gln Thr Asn Glu Gly Leu Leu Gln
            260             265             270
```

```
tcc gat cag gag ttg ttc tcc aca aca gga gcc gac acg atc gcg att       864
Ser Asp Gln Glu Leu Phe Ser Thr Thr Gly Ala Asp Thr Ile Ala Ile
        275             280             285

gtc aac aac ttc tcc tcc aac cag aca gcc ttc ttc gag tcc ttc gtc       912
Val Asn Asn Phe Ser Ser Asn Gln Thr Ala Phe Phe Glu Ser Phe Val
        290             295             300

gtc tcg atg atc cgg atg gga aac atc tcg ccc ttg acc ggc acc gat       960
Val Ser Met Ile Arg Met Gly Asn Ile Ser Pro Leu Thr Gly Thr Asp
305             310             315             320

ggt gaa att cgg ttg aac tgt cga atc gtg aac aac tcc acc ggc tcc      1008
Gly Glu Ile Arg Leu Asn Cys Arg Ile Val Asn Asn Ser Thr Gly Ser
        325             330             335

aac gcg ctc ctc gtc tcg tcg att tga                                  1035
Asn Ala Leu Leu Val Ser Ser Ile
        340
```

<210> 53
<211> 344
<212> PRT
<213> Artificial

<220>
<223> Synthetic Construct

<400> 53

```
Met Lys Leu Leu Ser Leu Thr Gly Val Ala Gly Val Leu Ala Thr Cys
1               5               10              15

Val Ala Ala Thr Pro Leu Val Lys Arg Leu Gly Gln Leu Thr Pro Thr
            20              25              30

Phe Tyr Asp Gly Thr Cys Pro Asn Val Ser Thr Ile Ile Arg Gly Val
        35              40              45

Leu Ala Gln Ala Leu Gln Thr Asp Pro Arg Ile Gly Ala Ser Leu Ile
    50              55              60

Arg Leu His Phe His Asp Cys Phe Val Asp Gly Cys Asp Gly Ser Ile
65              70              75              80

Leu Leu Asp Asn Thr Asp Thr Ile Glu Ser Glu Lys Glu Ala Ala Pro
            85              90              95

Asn Asn Asn Ser Ala Arg Gly Phe Asp Val Val Asp Asn Met Lys Ala
        100             105             110

Ala Val Glu Asn Ala Cys Pro Gly Ile Val Ser Cys Ala Asp Ile Leu
        115             120             125
```

```
Ala Ile Ala Ala Glu Glu Ser Val Arg Leu Ala Gly Gly Pro Ser Trp
    130                 135             140

Thr Val Pro Leu Gly Arg Arg Asp Ser Leu Ile Ala Asn Arg Ser Gly
145                 150             155                 160

Ala Asn Ser Ser Ile Pro Ala Pro Ser Glu Ser Leu Ala Val Leu Lys
                165             170             175

Ser Lys Phe Ala Ala Val Gly Leu Asn Thr Ser Ser Asp Leu Val Ala
            180             185             190

Leu Ser Gly Ala His Thr Phe Gly Arg Ala Gln Cys Leu Asn Phe Ile
            195             200             205

Ser Arg Leu Tyr Asn Phe Ser Gly Ser Gly Asn Pro Asp Pro Thr Leu
    210             215             220

Asn Thr Thr Tyr Leu Ala Ala Leu Gln Gln Leu Cys Pro Gln Gly Gly
225             230             235             240

Asn Arg Ser Val Leu Thr Asn Leu Asp Arg Thr Thr Pro Asp Thr Phe
            245             250             255

Asp Gly Asn Tyr Phe Ser Asn Leu Gln Thr Asn Glu Gly Leu Leu Gln
            260             265             270

Ser Asp Gln Glu Leu Phe Ser Thr Thr Gly Ala Asp Thr Ile Ala Ile
        275             280             285

Val Asn Asn Phe Ser Ser Asn Gln Thr Ala Phe Phe Glu Ser Phe Val
    290             295             300

Val Ser Met Ile Arg Met Gly Asn Ile Ser Pro Leu Thr Gly Thr Asp
305             310             315             320

Gly Glu Ile Arg Leu Asn Cys Arg Ile Val Asn Asn Ser Thr Gly Ser
            325             330             335

Asn Ala Leu Leu Val Ser Ser Ile
            340
```

<210> 54
<211> 1101
<212> DNA
<213> Zea mays

<220>

<221> CDS
<222> (7)..(1092)

<400> 54

```
ggatcc atg gga ggc gtg cgc tcg tac ttc ttc atc att gca gca gcc          48
       Met Gly Gly Val Arg Ser Tyr Phe Phe Ile Ile Ala Ala Ala
       1               5                   10

gtc gtg gcg gtc gtc ctc gcc ttg ttg cct gca ggc gca acg gga gcc         96
Val Val Ala Val Val Leu Ala Leu Leu Pro Ala Gly Ala Thr Gly Ala
15                  20                  25                  30

ggt ctc aaa gtg gga ttc tac tcg aaa acg tgt ccc tcg gca gag tcg        144
Gly Leu Lys Val Gly Phe Tyr Ser Lys Thr Cys Pro Ser Ala Glu Ser
                35                  40                  45

ctc gtc cag cag gcc gtc gca gcg gca ttc aag aac aac tcg ggc atc        192
Leu Val Gln Gln Ala Val Ala Ala Ala Phe Lys Asn Asn Ser Gly Ile
            50                  55                  60

gca gcc ggt ttg atc cgg ttg cac ttc cac gac tgt ttc gtg cga gga       240
Ala Ala Gly Leu Ile Arg Leu His Phe His Asp Cys Phe Val Arg Gly
            65                  70                  75

tgt gac ggc tcc gtc ttg att gac tcg act gcc aac aac aca gcc gaa       288
Cys Asp Gly Ser Val Leu Ile Asp Ser Thr Ala Asn Asn Thr Ala Glu
        80                  85                  90

aag gat gca gtg ccc aac aac ccg tcc ttg cgt ggt ttc gag gtg atc      336
Lys Asp Ala Val Pro Asn Asn Pro Ser Leu Arg Gly Phe Glu Val Ile
95                  100                 105                 110

gac gca gcc aag aaa gcg gtg gaa gca cgc tgt ccc aag aca gtc tcc      384
Asp Ala Ala Lys Lys Ala Val Glu Ala Arg Cys Pro Lys Thr Val Ser
                115                 120                 125

tgt gcc gac atc ttg gca ttc gca gca cga gac tcc atc gca ctc gca      432
Cys Ala Asp Ile Leu Ala Phe Ala Ala Arg Asp Ser Ile Ala Leu Ala
            130                 135                 140

ggc aac aac ttg acc tac aaa gtg cct gcg gga cga cgg gat ggt cgc      480
Gly Asn Asn Leu Thr Tyr Lys Val Pro Ala Gly Arg Arg Asp Gly Arg
            145                 150                 155

gtg tcg agg gat acg gac gca aac tcg aac ctc cct tcc cct ctc tcc      528
Val Ser Arg Asp Thr Asp Ala Asn Ser Asn Leu Pro Ser Pro Leu Ser
    160                 165                 170

aca gca gcg gag ctc gtc ggc aac ttc aca cgc aag aac ctc act gcc      576
Thr Ala Ala Glu Leu Val Gly Asn Phe Thr Arg Lys Asn Leu Thr Ala
175                 180                 185                 190

gag gat atg gtc gtc ctc tcc ggt gca cat act gtc gga cgg tcc cac      624
Glu Asp Met Val Val Leu Ser Gly Ala His Thr Val Gly Arg Ser His
                195                 200                 205

tgt tcg tcc ttc acc aac cgc ttg tat gga ttc tcg aac gca tcg gac      672
Cys Ser Ser Phe Thr Asn Arg Leu Tyr Gly Phe Ser Asn Ala Ser Asp
            210                 215                 220

gtg gac ccc acc att tcg tcg gcc tac gca ctc ttg ctc cga gcc att      720
Val Asp Pro Thr Ile Ser Ser Ala Tyr Ala Leu Leu Leu Arg Ala Ile
```

                    225                     230                     235

```
tgt cct tcc aac acc tcc cag ttc ttc ccc aac aca act acg gat atg    768
Cys Pro Ser Asn Thr Ser Gln Phe Phe Pro Asn Thr Thr Thr Asp Met
    240                 245                 250

gac ttg att acc cct gcg ctc ttg gat aac cga tac tac gtg gga ctc    816
Asp Leu Ile Thr Pro Ala Leu Leu Asp Asn Arg Tyr Tyr Val Gly Leu
255                 260                 265                 270

gcc aac aac ctc ggt ctc ttc aca tcc gat cag gcg ttg ctc acc aac    864
Ala Asn Asn Leu Gly Leu Phe Thr Ser Asp Gln Ala Leu Leu Thr Asn
                275                 280                 285

gca acc ctc aag aag tcc gtc gat gcc ttc gtc aag tcc gag tcg gca    912
Ala Thr Leu Lys Lys Ser Val Asp Ala Phe Val Lys Ser Glu Ser Ala
                290                 295                 300

tgg aaa acc aag ttc gcc aag tcg atg gtc aaa atg ggc aac atc gat    960
Trp Lys Thr Lys Phe Ala Lys Ser Met Val Lys Met Gly Asn Ile Asp
            305                 310                 315

gtg ttg acc gga acg aaa ggt gag atc agg ctc aac tgt cgg gtc atc   1008
Val Leu Thr Gly Thr Lys Gly Glu Ile Arg Leu Asn Cys Arg Val Ile
    320                 325                 330

aac tcc ggc tcc tcg tcc tcg ggc ttg ttc cag ctc cac aca gcc aca   1056
Asn Ser Gly Ser Ser Ser Ser Gly Leu Phe Gln Leu His Thr Ala Thr
335                 340                 345                 350

gca tcg gac gaa gaa ttc gcc cac gtg gca acc aac tgacttaag         1101
Ala Ser Asp Glu Glu Phe Ala His Val Ala Thr Asn
                355                 360
```

<210> 55
<211> 362
<212> PRT
<213> Zea mays

<400> 55

```
Met Gly Gly Val Arg Ser Tyr Phe Phe Ile Ile Ala Ala Ala Val Val
1               5               10              15

Ala Val Val Leu Ala Leu Leu Pro Ala Gly Ala Thr Gly Ala Gly Leu
        20              25              30

Lys Val Gly Phe Tyr Ser Lys Thr Cys Pro Ser Ala Glu Ser Leu Val
        35              40              45

Gln Gln Ala Val Ala Ala Ala Phe Lys Asn Asn Ser Gly Ile Ala Ala
    50              55              60

Gly Leu Ile Arg Leu His Phe His Asp Cys Phe Val Arg Gly Cys Asp
65              70              75              80

Gly Ser Val Leu Ile Asp Ser Thr Ala Asn Asn Thr Ala Glu Lys Asp
```

<div align="center">85      90      95</div>

Ala Val Pro Asn Asn Pro Ser Leu Arg Gly Phe Glu Val Ile Asp Ala
    100      105      110

Ala Lys Lys Ala Val Glu Ala Arg Cys Pro Lys Thr Val Ser Cys Ala
    115      120      125

Asp Ile Leu Ala Phe Ala Ala Arg Asp Ser Ile Ala Leu Ala Gly Asn
    130      135      140

Asn Leu Thr Tyr Lys Val Pro Ala Gly Arg Arg Asp Gly Arg Val Ser
145      150      155      160

Arg Asp Thr Asp Ala Asn Ser Asn Leu Pro Ser Pro Leu Ser Thr Ala
    165      170      175

Ala Glu Leu Val Gly Asn Phe Thr Arg Lys Asn Leu Thr Ala Glu Asp
    180      185      190

Met Val Val Leu Ser Gly Ala His Thr Val Gly Arg Ser His Cys Ser
    195      200      205

Ser Phe Thr Asn Arg Leu Tyr Gly Phe Ser Asn Ala Ser Asp Val Asp
    210      215      220

Pro Thr Ile Ser Ser Ala Tyr Ala Leu Leu Leu Arg Ala Ile Cys Pro
225      230      235      240

Ser Asn Thr Ser Gln Phe Phe Pro Asn Thr Thr Thr Asp Met Asp Leu
    245      250      255

Ile Thr Pro Ala Leu Leu Asp Asn Arg Tyr Tyr Val Gly Leu Ala Asn
    260      265      270

Asn Leu Gly Leu Phe Thr Ser Asp Gln Ala Leu Leu Thr Asn Ala Thr
    275      280      285

Leu Lys Lys Ser Val Asp Ala Phe Val Lys Ser Glu Ser Ala Trp Lys
    290      295      300

Thr Lys Phe Ala Lys Ser Met Val Lys Met Gly Asn Ile Asp Val Leu
305      310      315      320

Thr Gly Thr Lys Gly Glu Ile Arg Leu Asn Cys Arg Val Ile Asn Ser
    325      330      335

<div align="center">118</div>

Gly Ser Ser Ser Ser Gly Leu Phe Gln Leu His Thr Ala Thr Ala Ser
        340             345             350

Asp Glu Glu Phe Ala His Val Ala Thr Asn
        355             360

<210> 56
<211> 1113
<212> DNA
<213> Artificial

<220>
<223> Artificial construct

<220>
<221> CDS
<222> (1)..(1110)

<400> 56

```
atg aag ttc ttc acc acc atc ctc agc acc gcc agc ctt gtt gct gct          48
Met Lys Phe Phe Thr Thr Ile Leu Ser Thr Ala Ser Leu Val Ala Ala
1               5               10              15

ctc ccc gcc gct gtt gac tcg aac cat acc ccg gcc gct cct gaa ctt          96
Leu Pro Ala Ala Val Asp Ser Asn His Thr Pro Ala Ala Pro Glu Leu
                20              25              30

gtt gcc cgc cta ggt gcc ggt ctc aaa gtg gga ttc tac tcg aaa acg         144
Val Ala Arg Leu Gly Ala Gly Leu Lys Val Gly Phe Tyr Ser Lys Thr
            35              40              45

tgt ccc tcg gca gag tcg ctc gtc cag cag gcc gtc gca gcg gca ttc         192
Cys Pro Ser Ala Glu Ser Leu Val Gln Gln Ala Val Ala Ala Ala Phe
        50              55              60

aag aac aac tcg ggc atc gca gcc ggt ttg atc cgg ttg cac ttc cac         240
Lys Asn Asn Ser Gly Ile Ala Ala Gly Leu Ile Arg Leu His Phe His
65              70              75              80

gac tgt ttc gtg cga gga tgt gac ggc tcc gtc ttg att gac tcg act         288
Asp Cys Phe Val Arg Gly Cys Asp Gly Ser Val Leu Ile Asp Ser Thr
                85              90              95

gcc aac aac aca gcc gaa aag gat gca gtg ccc aac aac ccg tcc ttg         336
Ala Asn Asn Thr Ala Glu Lys Asp Ala Val Pro Asn Asn Pro Ser Leu
            100             105             110

cgt ggt ttc gag gtg atc gac gca gcc aag aaa gcg gtg gaa gca cgc         384
Arg Gly Phe Glu Val Ile Asp Ala Ala Lys Lys Ala Val Glu Ala Arg
            115             120             125

tgt ccc aag aca gtc tcc tgt gcc gac atc ttg gca ttc gca gca cga         432
Cys Pro Lys Thr Val Ser Cys Ala Asp Ile Leu Ala Phe Ala Ala Arg
        130             135             140

gac tcc atc gca ctc gca ggc aac aac ttg acc tac aaa gtg cct gcg         480
Asp Ser Ile Ala Leu Ala Gly Asn Asn Leu Thr Tyr Lys Val Pro Ala
145             150             155             160

gga cga cgg gat ggt cgc gtg tcg agg gat acg gac gca aac tcg aac         528
```

```
        Gly Arg Arg Asp Gly Arg Val Ser Arg Asp Thr Asp Ala Asn Ser Asn
                        165                 170                 175

        ctc cct tcc cct ctc tcc aca gca gcg gag ctc gtc ggc aac ttc aca        576
        Leu Pro Ser Pro Leu Ser Thr Ala Ala Glu Leu Val Gly Asn Phe Thr
                        180                 185                 190

        cgc aag aac ctc act gcc gag gat atg gtc gtc ctc tcc ggt gca cat        624
        Arg Lys Asn Leu Thr Ala Glu Asp Met Val Val Leu Ser Gly Ala His
                195                 200                 205

        act gtc gga cgg tcc cac tgt tcg tcc ttc acc aac cgc ttg tat gga        672
        Thr Val Gly Arg Ser His Cys Ser Ser Phe Thr Asn Arg Leu Tyr Gly
                210                 215                 220

        ttc tcg aac gca tcg gac gtg gac ccc acc att tcg tcg gcc tac gca        720
        Phe Ser Asn Ala Ser Asp Val Asp Pro Thr Ile Ser Ser Ala Tyr Ala
        225                 230                 235                 240

        ctc ttg ctc cga gcc att tgt cct tcc aac acc tcc cag ttc ttc ccc        768
        Leu Leu Leu Arg Ala Ile Cys Pro Ser Asn Thr Ser Gln Phe Phe Pro
                        245                 250                 255

        aac aca act acg gat atg gac ttg att acc cct gcg ctc ttg gat aac        816
        Asn Thr Thr Thr Asp Met Asp Leu Ile Thr Pro Ala Leu Leu Asp Asn
                        260                 265                 270

        cga tac tac gtg gga ctc gcc aac aac ctc ggt ctc ttc aca tcc gat        864
        Arg Tyr Tyr Val Gly Leu Ala Asn Asn Leu Gly Leu Phe Thr Ser Asp
                        275                 280                 285

        cag gcg ttg ctc acc aac gca acc ctc aag aag tcc gtc gat gcc ttc        912
        Gln Ala Leu Leu Thr Asn Ala Thr Leu Lys Lys Ser Val Asp Ala Phe
                290                 295                 300

        gtc aag tcc gag tcg gca tgg aaa acc aag ttc gcc aag tcg atg gtc        960
        Val Lys Ser Glu Ser Ala Trp Lys Thr Lys Phe Ala Lys Ser Met Val
        305                 310                 315                 320

        aaa atg ggc aac atc gat gtg ttg acc gga acg aaa ggt gag atc agg       1008
        Lys Met Gly Asn Ile Asp Val Leu Thr Gly Thr Lys Gly Glu Ile Arg
                        325                 330                 335

        ctc aac tgt cgg gtc atc aac tcc ggc tcc tcg tcc tcg ggc ttg ttc       1056
        Leu Asn Cys Arg Val Ile Asn Ser Gly Ser Ser Ser Ser Gly Leu Phe
                        340                 345                 350

        cag ctc cac aca gcc aca gca tcg gac gaa gaa ttc gcc cac gtg gca       1104
        Gln Leu His Thr Ala Thr Ala Ser Asp Glu Glu Phe Ala His Val Ala
                        355                 360                 365

        acc aac tga                                                          1113
        Thr Asn
                370


        <210> 57
        <211> 370
        <212> PRT
        <213> Artificial

        <220>
```

<223> Synthetic Construct

<400> 57

```
Met Lys Phe Phe Thr Thr Ile Leu Ser Thr Ala Ser Leu Val Ala Ala
1               5               10              15

Leu Pro Ala Ala Val Asp Ser Asn His Thr Pro Ala Ala Pro Glu Leu
        20              25              30

Val Ala Arg Leu Gly Ala Gly Leu Lys Val Gly Phe Tyr Ser Lys Thr
        35              40              45

Cys Pro Ser Ala Glu Ser Leu Val Gln Gln Ala Val Ala Ala Ala Phe
    50              55              60

Lys Asn Asn Ser Gly Ile Ala Ala Gly Leu Ile Arg Leu His Phe His
65              70              75              80

Asp Cys Phe Val Arg Gly Cys Asp Gly Ser Val Leu Ile Asp Ser Thr
            85              90              95

Ala Asn Asn Thr Ala Glu Lys Asp Ala Val Pro Asn Asn Pro Ser Leu
            100             105             110

Arg Gly Phe Glu Val Ile Asp Ala Ala Lys Lys Ala Val Glu Ala Arg
        115             120             125

Cys Pro Lys Thr Val Ser Cys Ala Asp Ile Leu Ala Phe Ala Ala Arg
    130             135             140

Asp Ser Ile Ala Leu Ala Gly Asn Asn Leu Thr Tyr Lys Val Pro Ala
145             150             155             160

Gly Arg Arg Asp Gly Arg Val Ser Arg Asp Thr Asp Ala Asn Ser Asn
            165             170             175

Leu Pro Ser Pro Leu Ser Thr Ala Ala Glu Leu Val Gly Asn Phe Thr
        180             185             190

Arg Lys Asn Leu Thr Ala Glu Asp Met Val Val Leu Ser Gly Ala His
        195             200             205

Thr Val Gly Arg Ser His Cys Ser Ser Phe Thr Asn Arg Leu Tyr Gly
    210             215             220

Phe Ser Asn Ala Ser Asp Val Asp Pro Thr Ile Ser Ser Ala Tyr Ala
225             230             235             240
```

```
Leu Leu Leu Arg Ala Ile Cys Pro Ser Asn Thr Ser Gln Phe Phe Pro
            245             250                 255

Asn Thr Thr Thr Asp Met Asp Leu Ile Thr Pro Ala Leu Leu Asp Asn
            260             265                 270

Arg Tyr Tyr Val Gly Leu Ala Asn Asn Leu Gly Leu Phe Thr Ser Asp
            275             280                 285

Gln Ala Leu Leu Thr Asn Ala Thr Leu Lys Lys Ser Val Asp Ala Phe
            290             295                 300

Val Lys Ser Glu Ser Ala Trp Lys Thr Lys Phe Ala Lys Ser Met Val
305             310                 315                 320

Lys Met Gly Asn Ile Asp Val Leu Thr Gly Thr Lys Gly Glu Ile Arg
            325             330                 335

Leu Asn Cys Arg Val Ile Asn Ser Gly Ser Ser Ser Ser Gly Leu Phe
            340             345                 350

Gln Leu His Thr Ala Thr Ala Ser Asp Glu Glu Phe Ala His Val Ala
            355             360                 365

Thr Asn
370
```

<210> 58
<211> 1077
<212> DNA
<213> Artificial

<220>
<223> Artificial construct

<220>
<221> CDS
<222> (1)..(1074)

<400> 58

```
atg aag ttc ttc acc acc atc ctc agc acc gcc agc ctt gtt gct gct        48
Met Lys Phe Phe Thr Thr Ile Leu Ser Thr Ala Ser Leu Val Ala Ala
1               5                   10                  15

ctc ccc gcc gct gtt gac tcc cta ggt gcc ggt ctc aaa gtg gga ttc        96
Leu Pro Ala Ala Val Asp Ser Leu Gly Ala Gly Leu Lys Val Gly Phe
            20                  25                  30

tac tcg aaa acg tgt ccc tcg gca gag tcg ctc gtc cag cag gcc gtc       144
Tyr Ser Lys Thr Cys Pro Ser Ala Glu Ser Leu Val Gln Gln Ala Val
        35                  40                  45

gca gcg gca ttc aag aac aac tcg ggc atc gca gcc ggt ttg atc cgg       192
```

```
Ala Ala Ala Phe Lys Asn Asn Ser Gly Ile Ala Ala Gly Leu Ile Arg
 50                  55                  60

ttg cac ttc cac gac tgt ttc gtg cga gga tgt gac ggc tcc gtc ttg      240
Leu His Phe His Asp Cys Phe Val Arg Gly Cys Asp Gly Ser Val Leu
 65                  70                  75                  80

att gac tcg act gcc aac aac aca gcc gaa aag gat gca gtg ccc aac      288
Ile Asp Ser Thr Ala Asn Asn Thr Ala Glu Lys Asp Ala Val Pro Asn
                     85                  90                  95

aac ccg tcc ttg cgt ggt ttc gag gtg atc gac gca gcc aag aaa gcg      336
Asn Pro Ser Leu Arg Gly Phe Glu Val Ile Asp Ala Ala Lys Lys Ala
                100                 105                 110

gtg gaa gca cgc tgt ccc aag aca gtc tcc tgt gcc gac atc ttg gca      384
Val Glu Ala Arg Cys Pro Lys Thr Val Ser Cys Ala Asp Ile Leu Ala
            115                 120                 125

ttc gca gca cga gac tcc atc gca ctc gca ggc aac aac ttg acc tac      432
Phe Ala Ala Arg Asp Ser Ile Ala Leu Ala Gly Asn Asn Leu Thr Tyr
        130                 135                 140

aaa gtg cct gcg gga cga cgg gat ggt cgc gtg tcg agg gat acg gac      480
Lys Val Pro Ala Gly Arg Arg Asp Gly Arg Val Ser Arg Asp Thr Asp
145                 150                 155                 160

gca aac tcg aac ctc cct tcc cct ctc tcc aca gca gcg gag ctc gtc      528
Ala Asn Ser Asn Leu Pro Ser Pro Leu Ser Thr Ala Ala Glu Leu Val
                165                 170                 175

ggc aac ttc aca cgc aag aac ctc act gcc gag gat atg gtc gtc ctc      576
Gly Asn Phe Thr Arg Lys Asn Leu Thr Ala Glu Asp Met Val Val Leu
                180                 185                 190

tcc ggt gca cat act gtc gga cgg tcc cac tgt tcg tcc ttc acc aac      624
Ser Gly Ala His Thr Val Gly Arg Ser His Cys Ser Ser Phe Thr Asn
            195                 200                 205

cgc ttg tat gga ttc tcg aac gca tcg gac gtg gac ccc acc att tcg      672
Arg Leu Tyr Gly Phe Ser Asn Ala Ser Asp Val Asp Pro Thr Ile Ser
        210                 215                 220

tcg gcc tac gca ctc ttg ctc cga gcc att tgt cct tcc aac acc tcc      720
Ser Ala Tyr Ala Leu Leu Leu Arg Ala Ile Cys Pro Ser Asn Thr Ser
225                 230                 235                 240

cag ttc ttc ccc aac aca act acg gat atg gac ttg att acc cct gcg      768
Gln Phe Phe Pro Asn Thr Thr Thr Asp Met Asp Leu Ile Thr Pro Ala
                245                 250                 255

ctc ttg gat aac cga tac tac gtg gga ctc gcc aac aac ctc ggt ctc      816
Leu Leu Asp Asn Arg Tyr Tyr Val Gly Leu Ala Asn Asn Leu Gly Leu
                260                 265                 270

ttc aca tcc gat cag gcg ttg ctc acc aac gca acc ctc aag aag tcc      864
Phe Thr Ser Asp Gln Ala Leu Leu Thr Asn Ala Thr Leu Lys Lys Ser
            275                 280                 285

gtc gat gcc ttc gtc aag tcc gag tcg gca tgg aaa acc aag ttc gcc      912
Val Asp Ala Phe Val Lys Ser Glu Ser Ala Trp Lys Thr Lys Phe Ala
        290                 295                 300
```

```
aag tcg atg gtc aaa atg ggc aac atc gat gtg ttg acc gga acg aaa      960
Lys Ser Met Val Lys Met Gly Asn Ile Asp Val Leu Thr Gly Thr Lys
305             310             315             320

ggt gag atc agg ctc aac tgt cgg gtc atc aac tcc ggc tcc tcg tcc     1008
Gly Glu Ile Arg Leu Asn Cys Arg Val Ile Asn Ser Gly Ser Ser Ser
            325             330             335

tcg ggc ttg ttc cag ctc cac aca gcc aca gca tcg gac gaa gaa ttc     1056
Ser Gly Leu Phe Gln Leu His Thr Ala Thr Ala Ser Asp Glu Glu Phe
        340             345             350

gcc cac gtg gca acc aac tga                                          1077
Ala His Val Ala Thr Asn
        355
```

<210> 59
<211> 358
<212> PRT
<213> Artificial

<220>
<223> Synthetic Construct

<400> 59

```
Met Lys Phe Phe Thr Thr Ile Leu Ser Thr Ala Ser Leu Val Ala Ala
1               5               10              15


Leu Pro Ala Ala Val Asp Ser Leu Gly Ala Gly Leu Lys Val Gly Phe
        20              25              30


Tyr Ser Lys Thr Cys Pro Ser Ala Glu Ser Leu Val Gln Gln Ala Val
        35              40              45


Ala Ala Ala Phe Lys Asn Asn Ser Gly Ile Ala Ala Gly Leu Ile Arg
        50              55              60


Leu His Phe His Asp Cys Phe Val Arg Gly Cys Asp Gly Ser Val Leu
65              70              75              80


Ile Asp Ser Thr Ala Asn Asn Thr Ala Glu Lys Asp Ala Val Pro Asn
            85              90              95


Asn Pro Ser Leu Arg Gly Phe Glu Val Ile Asp Ala Ala Lys Lys Ala
        100             105             110


Val Glu Ala Arg Cys Pro Lys Thr Val Ser Cys Ala Asp Ile Leu Ala
        115             120             125


Phe Ala Ala Arg Asp Ser Ile Ala Leu Ala Gly Asn Asn Leu Thr Tyr
        130             135             140
```

```
Lys Val Pro Ala Gly Arg Arg Asp Gly Arg Val Ser Arg Asp Thr Asp
145             150             155             160


Ala Asn Ser Asn Leu Pro Ser Pro Leu Ser Thr Ala Ala Glu Leu Val
            165             170             175


Gly Asn Phe Thr Arg Lys Asn Leu Thr Ala Glu Asp Met Val Val Leu
            180             185             190


Ser Gly Ala His Thr Val Gly Arg Ser His Cys Ser Ser Phe Thr Asn
            195             200             205


Arg Leu Tyr Gly Phe Ser Asn Ala Ser Asp Val Asp Pro Thr Ile Ser
    210             215             220


Ser Ala Tyr Ala Leu Leu Leu Arg Ala Ile Cys Pro Ser Asn Thr Ser
225             230             235             240


Gln Phe Phe Pro Asn Thr Thr Thr Asp Met Asp Leu Ile Thr Pro Ala
            245             250             255


Leu Leu Asp Asn Arg Tyr Tyr Val Gly Leu Ala Asn Asn Leu Gly Leu
            260             265             270


Phe Thr Ser Asp Gln Ala Leu Leu Thr Asn Ala Thr Leu Lys Lys Ser
            275             280             285


Val Asp Ala Phe Val Lys Ser Glu Ser Ala Trp Lys Thr Lys Phe Ala
    290             295             300


Lys Ser Met Val Lys Met Gly Asn Ile Asp Val Leu Thr Gly Thr Lys
305             310             315             320


Gly Glu Ile Arg Leu Asn Cys Arg Val Ile Asn Ser Gly Ser Ser Ser
            325             330             335


Ser Gly Leu Phe Gln Leu His Thr Ala Thr Ala Ser Asp Glu Glu Phe
            340             345             350


Ala His Val Ala Thr Asn
            355
```

<210> 60
<211> 1107
<212> DNA
<213> Artificial

<220>

128

<223> Artificial construct

<220>
<221> CDS
<222> (1)..(1104)

<400> 60

```
atg aga tta tcg act tcg agt ctc ttc ctt tcc gtg tct ctg ctg ggg          48
Met Arg Leu Ser Thr Ser Ser Leu Phe Leu Ser Val Ser Leu Leu Gly
1               5               10              15

aag ctg gcc ctc ggg agc cct ttg ccc caa cag cag cga tat ggc aaa          96
Lys Leu Ala Leu Gly Ser Pro Leu Pro Gln Gln Gln Arg Tyr Gly Lys
            20              25              30

cgc cta ggt gcc ggt ctc aaa gtg gga ttc tac tcg aaa acg tgt ccc          144
Arg Leu Gly Ala Gly Leu Lys Val Gly Phe Tyr Ser Lys Thr Cys Pro
        35              40              45

tcg gca gag tcg ctc gtc cag cag gcc gtc gca gcg gca ttc aag aac          192
Ser Ala Glu Ser Leu Val Gln Gln Ala Val Ala Ala Ala Phe Lys Asn
    50              55              60

aac tcg ggc atc gca gcc ggt ttg atc cgg ttg cac ttc cac gac tgt          240
Asn Ser Gly Ile Ala Ala Gly Leu Ile Arg Leu His Phe His Asp Cys
65              70              75              80

ttc gtg cga gga tgt gac ggc tcc gtc ttg att gac tcg act gcc aac          288
Phe Val Arg Gly Cys Asp Gly Ser Val Leu Ile Asp Ser Thr Ala Asn
            85              90              95

aac aca gcc gaa aag gat gca gtg ccc aac aac ccg tcc ttg cgt ggt          336
Asn Thr Ala Glu Lys Asp Ala Val Pro Asn Asn Pro Ser Leu Arg Gly
            100             105             110

ttc gag gtg atc gac gca gcc aag aaa gcg gtg gaa gca cgc tgt ccc          384
Phe Glu Val Ile Asp Ala Ala Lys Lys Ala Val Glu Ala Arg Cys Pro
        115             120             125

aag aca gtc tcc tgt gcc gac atc ttg gca ttc gca gca cga gac tcc          432
Lys Thr Val Ser Cys Ala Asp Ile Leu Ala Phe Ala Ala Arg Asp Ser
        130             135             140

atc gca ctc gca ggc aac aac ttg acc tac aaa gtg cct gcg gga cga          480
Ile Ala Leu Ala Gly Asn Asn Leu Thr Tyr Lys Val Pro Ala Gly Arg
145             150             155             160

cgg gat ggt cgc gtg tcg agg gat acg gac gca aac tcg aac ctc cct          528
Arg Asp Gly Arg Val Ser Arg Asp Thr Asp Ala Asn Ser Asn Leu Pro
            165             170             175

tcc cct ctc tcc aca gca gcg gag ctc gtc ggc aac ttc aca cgc aag          576
Ser Pro Leu Ser Thr Ala Ala Glu Leu Val Gly Asn Phe Thr Arg Lys
            180             185             190

aac ctc act gcc gag gat atg gtc gtc ctc tcc ggt gca cat act gtc          624
Asn Leu Thr Ala Glu Asp Met Val Val Leu Ser Gly Ala His Thr Val
            195             200             205

gga cgg tcc cac tgt tcg tcc ttc acc aac cgc ttg tat gga ttc tcg          672
Gly Arg Ser His Cys Ser Ser Phe Thr Asn Arg Leu Tyr Gly Phe Ser
            210             215             220
```

```
aac gca tcg gac gtg gac ccc acc att tcg tcg gcc tac gca ctc ttg        720
Asn Ala Ser Asp Val Asp Pro Thr Ile Ser Ser Ala Tyr Ala Leu Leu
225             230             235             240

ctc cga gcc att tgt cct tcc aac acc tcc cag ttc ttc ccc aac aca        768
Leu Arg Ala Ile Cys Pro Ser Asn Thr Ser Gln Phe Phe Pro Asn Thr
            245             250             255

act acg gat atg gac ttg att acc cct gcg ctc ttg gat aac cga tac        816
Thr Thr Asp Met Asp Leu Ile Thr Pro Ala Leu Leu Asp Asn Arg Tyr
            260             265             270

tac gtg gga ctc gcc aac aac ctc ggt ctc ttc aca tcc gat cag gcg        864
Tyr Val Gly Leu Ala Asn Asn Leu Gly Leu Phe Thr Ser Asp Gln Ala
            275             280             285

ttg ctc acc aac gca acc ctc aag aag tcc gtc gat gcc ttc gtc aag        912
Leu Leu Thr Asn Ala Thr Leu Lys Lys Ser Val Asp Ala Phe Val Lys
            290             295             300

tcc gag tcg gca tgg aaa acc aag ttc gcc aag tcg atg gtc aaa atg        960
Ser Glu Ser Ala Trp Lys Thr Lys Phe Ala Lys Ser Met Val Lys Met
305             310             315             320

ggc aac atc gat gtg ttg acc gga acg aaa ggt gag atc agg ctc aac       1008
Gly Asn Ile Asp Val Leu Thr Gly Thr Lys Gly Glu Ile Arg Leu Asn
            325             330             335

tgt cgg gtc atc aac tcc ggc tcc tcg tcc tcg ggc ttg ttc cag ctc       1056
Cys Arg Val Ile Asn Ser Gly Ser Ser Ser Ser Gly Leu Phe Gln Leu
            340             345             350

cac aca gcc aca gca tcg gac gaa gaa ttc gcc cac gtg gca acc aac       1104
His Thr Ala Thr Ala Ser Asp Glu Glu Phe Ala His Val Ala Thr Asn
            355             360             365

tga                                                                    1107
```

<210> 61
<211> 368
<212> PRT
<213> Artificial

<220>
<223> Synthetic Construct

<400> 61

```
Met Arg Leu Ser Thr Ser Ser Leu Phe Leu Ser Val Ser Leu Leu Gly
1               5               10              15

Lys Leu Ala Leu Gly Ser Pro Leu Pro Gln Gln Gln Arg Tyr Gly Lys
        20              25                  30

Arg Leu Gly Ala Gly Leu Lys Val Gly Phe Tyr Ser Lys Thr Cys Pro
        35              40              45

Ser Ala Glu Ser Leu Val Gln Gln Ala Val Ala Ala Ala Phe Lys Asn
    50              55              60
```

```
Asn Ser Gly Ile Ala Ala Gly Leu Ile Arg Leu His Phe His Asp Cys
65              70              75                      80

Phe Val Arg Gly Cys Asp Gly Ser Val Leu Ile Asp Ser Thr Ala Asn
                85              90                      95

Asn Thr Ala Glu Lys Asp Ala Val Pro Asn Asn Pro Ser Leu Arg Gly
                100             105             110

Phe Glu Val Ile Asp Ala Ala Lys Lys Ala Val Glu Ala Arg Cys Pro
            115             120             125

Lys Thr Val Ser Cys Ala Asp Ile Leu Ala Phe Ala Ala Arg Asp Ser
            130             135             140

Ile Ala Leu Ala Gly Asn Asn Leu Thr Tyr Lys Val Pro Ala Gly Arg
145             150             155                     160

Arg Asp Gly Arg Val Ser Arg Asp Thr Asp Ala Asn Ser Asn Leu Pro
                165             170             175

Ser Pro Leu Ser Thr Ala Ala Glu Leu Val Gly Asn Phe Thr Arg Lys
            180             185             190

Asn Leu Thr Ala Glu Asp Met Val Val Leu Ser Gly Ala His Thr Val
            195             200             205

Gly Arg Ser His Cys Ser Ser Phe Thr Asn Arg Leu Tyr Gly Phe Ser
    210             215             220

Asn Ala Ser Asp Val Asp Pro Thr Ile Ser Ser Ala Tyr Ala Leu Leu
225             230             235                     240

Leu Arg Ala Ile Cys Pro Ser Asn Thr Ser Gln Phe Phe Pro Asn Thr
                245             250             255

Thr Thr Asp Met Asp Leu Ile Thr Pro Ala Leu Leu Asp Asn Arg Tyr
            260             265             270

Tyr Val Gly Leu Ala Asn Asn Leu Gly Leu Phe Thr Ser Asp Gln Ala
            275             280             285

Leu Leu Thr Asn Ala Thr Leu Lys Lys Ser Val Asp Ala Phe Val Lys
    290             295             300

Ser Glu Ser Ala Trp Lys Thr Lys Phe Ala Lys Ser Met Val Lys Met
```

```
                305                     310                     315                     320


        Gly Asn Ile Asp Val Leu Thr Gly Thr Lys Gly Glu Ile Arg Leu Asn
                            325                     330                     335


        Cys Arg Val Ile Asn Ser Gly Ser Ser Ser Gly Leu Phe Gln Leu
                        340                     345                     350


        His Thr Ala Thr Ala Ser Asp Glu Glu Phe Ala His Val Ala Thr Asn
                    355                     360                     365
```

<210> 62
<211> 1083
<212> DNA
<213> Artificial

<220>
<223> Artificial construct

<220>
<221> CDS
<222> (1)..(1080)

<400> 62

```
atg aag cta ctc tct ctg acc ggt gtg gct ggt gtg ctt gcg act tgc          48
Met Lys Leu Leu Ser Leu Thr Gly Val Ala Gly Val Leu Ala Thr Cys
1                   5                  10                  15

gtt gca gcc act cct ttg gtg aag cgc cta ggt gcc ggt ctc aaa gtg          96
Val Ala Ala Thr Pro Leu Val Lys Arg Leu Gly Ala Gly Leu Lys Val
                20                  25                  30

gga ttc tac tcg aaa acg tgt ccc tcg gca gag tcg ctc gtc cag cag         144
Gly Phe Tyr Ser Lys Thr Cys Pro Ser Ala Glu Ser Leu Val Gln Gln
            35                  40                  45

gcc gtc gca gcg gca ttc aag aac aac tcg ggc atc gca gcc ggt ttg         192
Ala Val Ala Ala Ala Phe Lys Asn Asn Ser Gly Ile Ala Ala Gly Leu
        50                  55                  60

atc cgg ttg cac ttc cac gac tgt ttc gtg cga gga tgt gac ggc tcc         240
Ile Arg Leu His Phe His Asp Cys Phe Val Arg Gly Cys Asp Gly Ser
65                  70                  75                  80

gtc ttg att gac tcg act gcc aac aac aca gcc gaa aag gat gca gtg         288
Val Leu Ile Asp Ser Thr Ala Asn Asn Thr Ala Glu Lys Asp Ala Val
                85                  90                  95

ccc aac aac ccg tcc ttg cgt ggt ttc gag gtg atc gac gca gcc aag         336
Pro Asn Asn Pro Ser Leu Arg Gly Phe Glu Val Ile Asp Ala Ala Lys
               100                 105                 110

aaa gcg gtg gaa gca cgc tgt ccc aag aca gtc tcc tgt gcc gac atc         384
Lys Ala Val Glu Ala Arg Cys Pro Lys Thr Val Ser Cys Ala Asp Ile
               115                 120                 125

ttg gca ttc gca gca cga gac tcc atc gca ctc gca ggc aac aac ttg         432
Leu Ala Phe Ala Ala Arg Asp Ser Ile Ala Leu Ala Gly Asn Asn Leu
```

                130                        135                        140

acc tac aaa gtg cct gcg gga cga cgg gat ggt cgc gtg tcg agg gat          480
Thr Tyr Lys Val Pro Ala Gly Arg Arg Asp Gly Arg Val Ser Arg Asp
145                 150                 155                 160

acg gac gca aac tcg aac ctc cct tcc cct ctc tcc aca gca gcg gag          528
Thr Asp Ala Asn Ser Asn Leu Pro Ser Pro Leu Ser Thr Ala Ala Glu
                    165                 170                 175

ctc gtc ggc aac ttc aca cgc aag aac ctc act gcc gag gat atg gtc          576
Leu Val Gly Asn Phe Thr Arg Lys Asn Leu Thr Ala Glu Asp Met Val
                180                 185                 190

gtc ctc tcc ggt gca cat act gtc gga cgg tcc cac tgt tcg tcc ttc          624
Val Leu Ser Gly Ala His Thr Val Gly Arg Ser His Cys Ser Ser Phe
                195                 200                 205

acc aac cgc ttg tat gga ttc tcg aac gca tcg gac gtg gac ccc acc          672
Thr Asn Arg Leu Tyr Gly Phe Ser Asn Ala Ser Asp Val Asp Pro Thr
    210                 215                 220

att tcg tcg gcc tac gca ctc ttg ctc cga gcc att tgt cct tcc aac          720
Ile Ser Ser Ala Tyr Ala Leu Leu Leu Arg Ala Ile Cys Pro Ser Asn
225                 230                 235                 240

acc tcc cag ttc ttc ccc aac aca act acg gat atg gac ttg att acc          768
Thr Ser Gln Phe Phe Pro Asn Thr Thr Thr Asp Met Asp Leu Ile Thr
                    245                 250                 255

cct gcg ctc ttg gat aac cga tac tac gtg gga ctc gcc aac aac ctc          816
Pro Ala Leu Leu Asp Asn Arg Tyr Tyr Val Gly Leu Ala Asn Asn Leu
                260                 265                 270

ggt ctc ttc aca tcc gat cag gcg ttg ctc acc aac gca acc ctc aag          864
Gly Leu Phe Thr Ser Asp Gln Ala Leu Leu Thr Asn Ala Thr Leu Lys
            275                 280                 285

aag tcc gtc gat gcc ttc gtc aag tcc gag tcg gca tgg aaa acc aag          912
Lys Ser Val Asp Ala Phe Val Lys Ser Glu Ser Ala Trp Lys Thr Lys
            290                 295                 300

ttc gcc aag tcg atg gtc aaa atg ggc aac atc gat gtg ttg acc gga          960
Phe Ala Lys Ser Met Val Lys Met Gly Asn Ile Asp Val Leu Thr Gly
305                 310                 315                 320

acg aaa ggt gag atc agg ctc aac tgt cgg gtc atc aac tcc ggc tcc          1008
Thr Lys Gly Glu Ile Arg Leu Asn Cys Arg Val Ile Asn Ser Gly Ser
                325                 330                 335

tcg tcc tcg ggc ttg ttc cag ctc cac aca gcc aca gca tcg gac gaa          1056
Ser Ser Ser Gly Leu Phe Gln Leu His Thr Ala Thr Ala Ser Asp Glu
            340                 345                 350

gaa ttc gcc cac gtg gca acc aac tga                                      1083
Glu Phe Ala His Val Ala Thr Asn
            355                 360

<210> 63
<211> 360
<212> PRT

<213> Artificial

<220>
<223> Synthetic Construct

<400> 63

<213> Artificial

<220>
<223> Synthetic Construct

Met Lys Leu Leu Ser Leu Thr Gly Val Ala Gly Val Leu Ala Thr Cys
1               5               10              15

Val Ala Ala Thr Pro Leu Val Lys Arg Leu Gly Ala Gly Leu Lys Val
            20              25              30

Gly Phe Tyr Ser Lys Thr Cys Pro Ser Ala Glu Ser Leu Val Gln Gln
        35              40              45

Ala Val Ala Ala Ala Phe Lys Asn Asn Ser Gly Ile Ala Ala Gly Leu
    50              55              60

Ile Arg Leu His Phe His Asp Cys Phe Val Arg Gly Cys Asp Gly Ser
65              70              75              80

Val Leu Ile Asp Ser Thr Ala Asn Asn Thr Ala Glu Lys Asp Ala Val
            85              90              95

Pro Asn Asn Pro Ser Leu Arg Gly Phe Glu Val Ile Asp Ala Ala Lys
            100             105             110

Lys Ala Val Glu Ala Arg Cys Pro Lys Thr Val Ser Cys Ala Asp Ile
        115             120             125

Leu Ala Phe Ala Ala Arg Asp Ser Ile Ala Leu Ala Gly Asn Asn Leu
    130             135             140

Thr Tyr Lys Val Pro Ala Gly Arg Arg Asp Gly Arg Val Ser Arg Asp
145             150             155             160

Thr Asp Ala Asn Ser Asn Leu Pro Ser Pro Leu Ser Thr Ala Ala Glu
            165             170             175

Leu Val Gly Asn Phe Thr Arg Lys Asn Leu Thr Ala Glu Asp Met Val
        180             185             190

Val Leu Ser Gly Ala His Thr Val Gly Arg Ser His Cys Ser Ser Phe
        195             200             205

Thr Asn Arg Leu Tyr Gly Phe Ser Asn Ala Ser Asp Val Asp Pro Thr
    210             215             220

Ile Ser Ser Ala Tyr Ala Leu Leu Leu Arg Ala Ile Cys Pro Ser Asn

138

225       230       235       240

Thr Ser Gln Phe Phe Pro Asn Thr Thr Thr Asp Met Asp Leu Ile Thr
              245           250           255

Pro Ala Leu Leu Asp Asn Arg Tyr Tyr Val Gly Leu Ala Asn Asn Leu
           260           265           270

Gly Leu Phe Thr Ser Asp Gln Ala Leu Leu Thr Asn Ala Thr Leu Lys
           275           280           285

Lys Ser Val Asp Ala Phe Val Lys Ser Glu Ser Ala Trp Lys Thr Lys
           290           295           300

Phe Ala Lys Ser Met Val Lys Met Gly Asn Ile Asp Val Leu Thr Gly
305           310           315           320

Thr Lys Gly Glu Ile Arg Leu Asn Cys Arg Val Ile Asn Ser Gly Ser
           325           330           335

Ser Ser Ser Gly Leu Phe Gln Leu His Thr Ala Thr Ala Ser Asp Glu
           340           345           350

Glu Phe Ala His Val Ala Thr Asn
           355           360

<210> 64
<211> 1065
<212> DNA
<213> Artificial

<220>
<223> Artificial construct

<220>
<221> CDS
<222> (1)..(1062)

<400> 64

```
atg aag cta ctc tct ctg acc ggt gtg gct ggt gtg ctt gcg act tgc       48
Met Lys Leu Leu Ser Leu Thr Gly Val Ala Gly Val Leu Ala Thr Cys
1               5               10              15

gtt gca gcc cta ggt gcc ggt ctc aaa gtg gga ttc tac tcg aaa acg       96
Val Ala Ala Leu Gly Ala Gly Leu Lys Val Gly Phe Tyr Ser Lys Thr
            20              25              30

tgt ccc tcg gca gag tcg ctc gtc cag cag gcc gtc gca gcg gca ttc      144
Cys Pro Ser Ala Glu Ser Leu Val Gln Gln Ala Val Ala Ala Ala Phe
        35              40              45

aag aac aac tcg ggc atc gca gcc ggt ttg atc cgg ttg cac ttc cac      192
Lys Asn Asn Ser Gly Ile Ala Ala Gly Leu Ile Arg Leu His Phe His
```

```
                    50                      55                      60

        gac tgt ttc gtg cga gga tgt gac ggc tcc gtc ttg att gac tcg act      240
        Asp Cys Phe Val Arg Gly Cys Asp Gly Ser Val Leu Ile Asp Ser Thr
        65                      70                      75                  80

        gcc aac aac aca gcc gaa aag gat gca gtg ccc aac aac ccg tcc ttg      288
        Ala Asn Asn Thr Ala Glu Lys Asp Ala Val Pro Asn Asn Pro Ser Leu
                            85                      90                      95

        cgt ggt ttc gag gtg atc gac gca gcc aag aaa gcg gtg gaa gca cgc      336
        Arg Gly Phe Glu Val Ile Asp Ala Ala Lys Lys Ala Val Glu Ala Arg
                            100                     105                     110

        tgt ccc aag aca gtc tcc tgt gcc gac atc ttg gca ttc gca gca cga      384
        Cys Pro Lys Thr Val Ser Cys Ala Asp Ile Leu Ala Phe Ala Ala Arg
                            115                     120                     125

        gac tcc atc gca ctc gca ggc aac aac ttg acc tac aaa gtg cct gcg      432
        Asp Ser Ile Ala Leu Ala Gly Asn Asn Leu Thr Tyr Lys Val Pro Ala
                            130                     135                     140

        gga cga cgg gat ggt cgc gtg tcg agg gat acg gac gca aac tcg aac      480
        Gly Arg Arg Asp Gly Arg Val Ser Arg Asp Thr Asp Ala Asn Ser Asn
        145                     150                     155                 160

        ctc cct tcc cct ctc tcc aca gca gcg gag ctc gtc ggc aac ttc aca      528
        Leu Pro Ser Pro Leu Ser Thr Ala Ala Glu Leu Val Gly Asn Phe Thr
                            165                     170                     175

        cgc aag aac ctc act gcc gag gat atg gtc gtc ctc tcc ggt gca cat      576
        Arg Lys Asn Leu Thr Ala Glu Asp Met Val Val Leu Ser Gly Ala His
                            180                     185                     190

        act gtc gga cgg tcc cac tgt tcg tcc ttc acc aac cgc ttg tat gga      624
        Thr Val Gly Arg Ser His Cys Ser Ser Phe Thr Asn Arg Leu Tyr Gly
                            195                     200                     205

        ttc tcg aac gca tcg gac gtg gac ccc acc att tcg tcg gcc tac gca      672
        Phe Ser Asn Ala Ser Asp Val Asp Pro Thr Ile Ser Ser Ala Tyr Ala
                            210                     215                     220

        ctc ttg ctc cga gcc att tgt cct tcc aac acc tcc cag ttc ttc ccc      720
        Leu Leu Leu Arg Ala Ile Cys Pro Ser Asn Thr Ser Gln Phe Phe Pro
        225                     230                     235                 240

        aac aca act acg gat atg gac ttg att acc cct gcg ctc ttg gat aac      768
        Asn Thr Thr Thr Asp Met Asp Leu Ile Thr Pro Ala Leu Leu Asp Asn
                            245                     250                     255

        cga tac tac gtg gga ctc gcc aac aac ctc ggt ctc ttc aca tcc gat      816
        Arg Tyr Tyr Val Gly Leu Ala Asn Asn Leu Gly Leu Phe Thr Ser Asp
                            260                     265                     270

        cag gcg ttg ctc acc aac gca acc ctc aag aag tcc gtc gat gcc ttc      864
        Gln Ala Leu Leu Thr Asn Ala Thr Leu Lys Lys Ser Val Asp Ala Phe
                            275                     280                     285

        gtc aag tcc gag tcg gca tgg aaa acc aag ttc gcc aag tcg atg gtc      912
        Val Lys Ser Glu Ser Ala Trp Lys Thr Lys Phe Ala Lys Ser Met Val
                            290                     295                     300

        aaa atg ggc aac atc gat gtg ttg acc gga acg aaa ggt gag atc agg      960
```

141

```
Lys Met Gly Asn Ile Asp Val Leu Thr Gly Thr Lys Gly Glu Ile Arg
305                 310             315                 320

ctc aac tgt cgg gtc atc aac tcc ggc tcc tcg tcc tcg ggc ttg ttc    1008
Leu Asn Cys Arg Val Ile Asn Ser Gly Ser Ser Ser Ser Gly Leu Phe
                325             330                 335

cag ctc cac aca gcc aca gca tcg gac gaa gaa ttc gcc cac gtg gca    1056
Gln Leu His Thr Ala Thr Ala Ser Asp Glu Glu Phe Ala His Val Ala
                340             345                 350

acc aac tga                                                        1065
Thr Asn
```

<210> 65
<211> 354
<212> PRT
<213> Artificial

<220>
<223> Synthetic Construct

<400> 65

Met Lys Leu Leu Ser Leu Thr Gly Val Ala Gly Val Leu Ala Thr Cys
1                   5                   10                  15

Val Ala Ala Leu Gly Ala Gly Leu Lys Val Gly Phe Tyr Ser Lys Thr
            20                  25                  30

Cys Pro Ser Ala Glu Ser Leu Val Gln Gln Ala Val Ala Ala Ala Phe
            35                  40                  45

Lys Asn Asn Ser Gly Ile Ala Ala Gly Leu Ile Arg Leu His Phe His
    50                  55                  60

Asp Cys Phe Val Arg Gly Cys Asp Gly Ser Val Leu Ile Asp Ser Thr
65                  70                  75                  80

Ala Asn Asn Thr Ala Glu Lys Asp Ala Val Pro Asn Asn Pro Ser Leu
            85                  90                  95

Arg Gly Phe Glu Val Ile Asp Ala Ala Lys Lys Ala Val Glu Ala Arg
            100                 105                 110

Cys Pro Lys Thr Val Ser Cys Ala Asp Ile Leu Ala Phe Ala Ala Arg
            115                 120                 125

Asp Ser Ile Ala Leu Ala Gly Asn Asn Leu Thr Tyr Lys Val Pro Ala
    130                 135                 140

Gly Arg Arg Asp Gly Arg Val Ser Arg Asp Thr Asp Ala Asn Ser Asn

145 150 155 160

Leu Pro Ser Pro Leu Ser Thr Ala Ala Glu Leu Val Gly Asn Phe Thr
165 170 175

Arg Lys Asn Leu Thr Ala Glu Asp Met Val Val Leu Ser Gly Ala His
180 185 190

Thr Val Gly Arg Ser His Cys Ser Ser Phe Thr Asn Arg Leu Tyr Gly
195 200 205

Phe Ser Asn Ala Ser Asp Val Asp Pro Thr Ile Ser Ser Ala Tyr Ala
210 215 220

Leu Leu Leu Arg Ala Ile Cys Pro Ser Asn Thr Ser Gln Phe Phe Pro
225 230 235 240

Asn Thr Thr Thr Asp Met Asp Leu Ile Thr Pro Ala Leu Leu Asp Asn
245 250 255

Arg Tyr Tyr Val Gly Leu Ala Asn Asn Leu Gly Leu Phe Thr Ser Asp
260 265 270

Gln Ala Leu Leu Thr Asn Ala Thr Leu Lys Lys Ser Val Asp Ala Phe
275 280 285

Val Lys Ser Glu Ser Ala Trp Lys Thr Lys Phe Ala Lys Ser Met Val
290 295 300

Lys Met Gly Asn Ile Asp Val Leu Thr Gly Thr Lys Gly Glu Ile Arg
305 310 315 320

Leu Asn Cys Arg Val Ile Asn Ser Gly Ser Ser Ser Ser Gly Leu Phe
325 330 335

Gln Leu His Thr Ala Thr Ala Ser Asp Glu Glu Phe Ala His Val Ala
340 345 350

Thr Asn

<210> 66
<211> 972
<212> DNA
<213> Nicotiana tabacum

<220>
<221> CDS
<222> (1)..(972)

<400> 66

```
atg tcg ttc ctc cgc ttc gtg gga gcg atc ctc ttc ctc gtc gcg atc          48
Met Ser Phe Leu Arg Phe Val Gly Ala Ile Leu Phe Leu Val Ala Ile
1               5                   10                  15

ttc gga gcg tcg aac gcc cag ttg tcc gcg act ttc tat gat acc act          96
Phe Gly Ala Ser Asn Ala Gln Leu Ser Ala Thr Phe Tyr Asp Thr Thr
                20                  25                  30

tgt ccc aac gtg aca tcg atc gtg cgt ggc gtc atg gac cag agg cag         144
Cys Pro Asn Val Thr Ser Ile Val Arg Gly Val Met Asp Gln Arg Gln
            35                  40                  45

cgc acg gat gcg cga gcc ggt gcc aaa atc atc cga ttg cat ttc cat         192
Arg Thr Asp Ala Arg Ala Gly Ala Lys Ile Ile Arg Leu His Phe His
        50                  55                  60

gac tgt ttc gtg aac ggc tgt gac ggc tcg atc ttg ctc gac aca gac         240
Asp Cys Phe Val Asn Gly Cys Asp Gly Ser Ile Leu Leu Asp Thr Asp
65                  70                  75                  80

ggt acg cag acc gag aag gat gcc cct gcc aac gtc gga gcg ggt ggt         288
Gly Thr Gln Thr Glu Lys Asp Ala Pro Ala Asn Val Gly Ala Gly Gly
                85                  90                  95

ttc gac atc gtg gac gat atc aaa act gcc ttg gag aac gtc tgt cct         336
Phe Asp Ile Val Asp Asp Ile Lys Thr Ala Leu Glu Asn Val Cys Pro
            100                 105                 110

ggc gtc gtc tcc tgt gcc gac atc ctc gcg ctc gcc tcg gaa atc ggc         384
Gly Val Val Ser Cys Ala Asp Ile Leu Ala Leu Ala Ser Glu Ile Gly
            115                 120                 125

gtg gtg ctc gcg aaa gga ccc tcg tgg cag gtc ttg ttc ggc agg aag         432
Val Val Leu Ala Lys Gly Pro Ser Trp Gln Val Leu Phe Gly Arg Lys
        130                 135                 140

gac tcg ttg act gcc aac agg tcc gga gcc aac tcg gac atc ccc tcg         480
Asp Ser Leu Thr Ala Asn Arg Ser Gly Ala Asn Ser Asp Ile Pro Ser
145                 150                 155                 160

ccc ttc gag acg ttg gcc gtc atg atc cct cag ttc acc aac aag ggc         528
Pro Phe Glu Thr Leu Ala Val Met Ile Pro Gln Phe Thr Asn Lys Gly
                165                 170                 175

atg gac ctc acc gac ttg gtc gcg ttg tcg gga gcc cac acc ttc gga         576
Met Asp Leu Thr Asp Leu Val Ala Leu Ser Gly Ala His Thr Phe Gly
            180                 185                 190

agg gcc agg tgt ggc acc ttc gag cag cga ctc ttc aac ttc aac ggc         624
Arg Ala Arg Cys Gly Thr Phe Glu Gln Arg Leu Phe Asn Phe Asn Gly
            195                 200                 205

tcg ggt aac ccc gat ttg acc gtg gac gcc act ttc ctc cag aca ttg         672
Ser Gly Asn Pro Asp Leu Thr Val Asp Ala Thr Phe Leu Gln Thr Leu
            210                 215                 220

cag ggc atc tgt ccc cag ggt gga aac aac ggc aac acg ttc acg aac         720
Gln Gly Ile Cys Pro Gln Gly Gly Asn Asn Gly Asn Thr Phe Thr Asn
225                 230                 235                 240
```

```
ctc gac atc tcc act ccg aac gac ttc gac aac gac tac ttc acc aac        768
Leu Asp Ile Ser Thr Pro Asn Asp Phe Asp Asn Asp Tyr Phe Thr Asn
            245             250             255

ttg cag tcg aac cag ggc ctc ttg cag acg gat cag gag ttg ttc tcg        816
Leu Gln Ser Asn Gln Gly Leu Leu Gln Thr Asp Gln Glu Leu Phe Ser
            260             265             270

aca tcc ggt tcc gcc aca att gca att gtc aac agg tat gca ggc tcg        864
Thr Ser Gly Ser Ala Thr Ile Ala Ile Val Asn Arg Tyr Ala Gly Ser
            275             280             285

cag aca cag ttc ttc gat gat ttc gtg tcg tcc atg atc aag ctc ggt        912
Gln Thr Gln Phe Phe Asp Asp Phe Val Ser Ser Met Ile Lys Leu Gly
            290             295             300

aac att tcg cct ctc acc ggt acc aac ggc cag atc agg acc gat tgt        960
Asn Ile Ser Pro Leu Thr Gly Thr Asn Gly Gln Ile Arg Thr Asp Cys
305                 310             315             320

aag cgc gtg aac                                                        972
Lys Arg Val Asn
```

<210> 67
<211> 324
<212> PRT
<213> Nicotiana tabacum

<400> 67

```
        Met Ser Phe Leu Arg Phe Val Gly Ala Ile Leu Phe Leu Val Ala Ile
        1               5               10              15

        Phe Gly Ala Ser Asn Ala Gln Leu Ser Ala Thr Phe Tyr Asp Thr Thr
                    20              25              30

        Cys Pro Asn Val Thr Ser Ile Val Arg Gly Val Met Asp Gln Arg Gln
                    35              40              45

        Arg Thr Asp Ala Arg Ala Gly Ala Lys Ile Ile Arg Leu His Phe His
                    50              55              60

        Asp Cys Phe Val Asn Gly Cys Asp Gly Ser Ile Leu Leu Asp Thr Asp
        65              70              75              80

        Gly Thr Gln Thr Glu Lys Asp Ala Pro Ala Asn Val Gly Ala Gly Gly
                    85              90              95

        Phe Asp Ile Val Asp Asp Ile Lys Thr Ala Leu Glu Asn Val Cys Pro
                    100             105             110

        Gly Val Val Ser Cys Ala Asp Ile Leu Ala Leu Ala Ser Glu Ile Gly
                    115             120             125
```

```
Val Val Leu Ala Lys Gly Pro Ser Trp Gln Val Leu Phe Gly Arg Lys
    130                 135                 140

Asp Ser Leu Thr Ala Asn Arg Ser Gly Ala Asn Ser Asp Ile Pro Ser
145                 150                 155                 160

Pro Phe Glu Thr Leu Ala Val Met Ile Pro Gln Phe Thr Asn Lys Gly
                165                 170                 175

Met Asp Leu Thr Asp Leu Val Ala Leu Ser Gly Ala His Thr Phe Gly
                180                 185                 190

Arg Ala Arg Cys Gly Thr Phe Glu Gln Arg Leu Phe Asn Phe Asn Gly
        195                 200                 205

Ser Gly Asn Pro Asp Leu Thr Val Asp Ala Thr Phe Leu Gln Thr Leu
    210                 215                 220

Gln Gly Ile Cys Pro Gln Gly Gly Asn Asn Gly Asn Thr Phe Thr Asn
225                 230                 235                 240

Leu Asp Ile Ser Thr Pro Asn Asp Phe Asp Asn Asp Tyr Phe Thr Asn
                245                 250                 255

Leu Gln Ser Asn Gln Gly Leu Leu Gln Thr Asp Gln Glu Leu Phe Ser
                260                 265                 270

Thr Ser Gly Ser Ala Thr Ile Ala Ile Val Asn Arg Tyr Ala Gly Ser
        275                 280                 285

Gln Thr Gln Phe Phe Asp Asp Phe Val Ser Ser Met Ile Lys Leu Gly
    290                 295                 300

Asn Ile Ser Pro Leu Thr Gly Thr Asn Gly Gln Ile Arg Thr Asp Cys
305                 310                 315                 320

Lys Arg Val Asn
```

<210> 68
<211> 7
<212> PRT
<213> Artificial

<220>
<223> Motif

<220>
<221> misc_feature
<222> (4)..(4)

<223> Xaa is Ala or Gly

<220>
<221> misc_feature
<222> (6)..(6)
<223> Xaa is Val or Ile

<400> 68

```
Gly Cys Asp Xaa Ser Xaa Leu
1               5
```

<210> 69
<211> 8
<212> PRT
<213> Artificial

<220>
<223> Motif

<220>
<221> MISC_FEATURE
<222> (4)..(4)
<223> Xaa is Ala or Gly

<220>
<221> MISC_FEATURE
<222> (6)..(6)
<223> Xaa is Val or Ile

<220>
<221> MISC_FEATURE
<222> (7)..(7)
<223> Xaa is Ile or Leu

<220>
<221> MISC_FEATURE
<222> (8)..(8)
<223> Xaa is Ile or Leu

<400> 69

```
Gly Cys Asp Xaa Ser Xaa Xaa Xaa
1               5
```

<210> 70
<211> 7
<212> PRT
<213> Artificial

<220>
<223> Motif

<220>
<221> MISC_FEATURE
<222> (4)..(4)
<223> Xaa is Ala or Ser

<220>
<221> MISC_FEATURE
<222> (6)..(6)
<223> Xaa is Ile or Leu

<220>
<221> MISC_FEATURE
<222> (7)..(7)
<223> Xaa is Ile or Leu

<400> 70

```
Val Ser Cys Xaa Asp Xaa Xaa
1               5
```

## Claims

1. A method for recombinant expression of a plant peroxidase, comprising expressing in an *Aspergillus* host organism a nucleic acid sequence encoding a class III peroxidase from EC 1.11.1.7, wherein the amino acid sequence of the peroxidase comprises the amino acid motifs:

   HFHDCFV;
   GCD[A,G]S[V,I][I,L][I,L]; and
   VSC[A,S]D[I,L][I,L].

2. The method of claim 1, wherein the amino acid motifs are selected from the group consisting of:

   HFHDCFV;
   GCD[A,G]S[V,I]LL; and
   VSC[A,S]D[I,L]L.

3. The method of any of claims 1-2, wherein the amino acid sequence of the peroxidase has at least 65% identity, preferably at least 70% identity, at least 75% identity, at least 80% identity, at least 85% identity, at least 90% identity, or at least 95% identity, to the amino acid sequence of SEQ ID NO:2, SEQ ID NO:4, amino acids 38 to 354 of SEQ ID NO: 45, amino acids 30 to 362 of SEQ ID NO: 55, or amino acids 23 to 324 of SEQ ID NO: 67.

4. The method of any of claims 1-3, wherein the peroxidase consists of the amino acid sequence of SEQ ID NO:2, SEQ ID NO:4, amino acids 38 to 354 of SEQ ID NO: 45, amino acids 30 to 362 of SEQ ID NO: 55, or amino acids 23 to 324 of SEQ ID NO: 67.

5. The method of any of claims 1-4, wherein the nucleic acid sequence is codon optimized in at least 10% of the codons, preferably at least 20% of the codons, more preferably at least 30% of the codons, more preferably at least 50% of the codons, and most preferably at least 75% of the codons.

6. The method of claim 5, wherein the optimized codon(s) corresponds to the codon usage of alpha amylase from *Aspergillus oryzae*.

7. The method of any of claims 1-6, wherein the *Aspergillus* host organism is *Aspergillus oryzae* or *Aspergillus niger*.

8. An *Aspergillus* host organism comprising a nucleic acid sequence encoding a class III peroxidase from EC 1.11.1.7, capable of expressing at least 20 mg/L of peroxidase, wherein the amino acid sequence of the peroxidase comprises the amino acid motifs:

   HFHDCFV;
   GCD[A,G]S[V,I][I,L][I,L]; and
   VSC[A,S]D[I,L][I,L].

9. The *Aspergillus* host organism of claim 8, wherein the amino acid motifs are:

   HFHDCFV;
   GCD[A,G]S[V,I]LL; and
   VSC[A,S]D[I,L]L.

10. The *Aspergillus* host organism of claim 8 or 9, wherein the amino acid sequence of the peroxidase has at least 65% identity, preferably at least 70% identity, at least 75% identity, at least 80% identity, at least 85% identity, at least 90% identity, or at least 95% identity, to the amino acid sequence of SEQ ID NO:2, SEQ ID NO:4, amino acids 38 to 354 of SEQ ID NO: 45, amino acids 30 to 362 of SEQ ID NO: 55, or amino acids 23 to 324 of SEQ ID NO: 67.

11. The *Aspergillus* host organism of any of claims 8-10, wherein the peroxidase consists of the amino acid sequence of SEQ ID NO:2, SEQ ID NO:4, amino acids 38 to 354 of SEQ ID NO: 45, amino acids 30 to 362 of SEQ ID NO: 55, or amino acids 23 to 324 of SEQ ID NO: 67.

12. The *Aspergillus* host organism of any of claims 8-11, wherein the nucleic acid sequence is codon optimized in at least 10% of the codons, preferably at least 20% of the codons, more preferably at least 30% of the codons, more preferably at least 50% of the codons, and most preferably at least 75% of the codons.

13. The *Aspergillus* host organism of any of claims 8-12, wherein the optimized codon(s) corresponds to the codon usage of alpha amylase from *Aspergillus oryzae.*

14. The *Aspergillus* host organism of any of claims 8-13, wherein the *Aspergillus* host organism is *Aspergillus oryzae* or *Aspergillus niger.*

**Patentansprüche**

1. Verfahren zur rekombinanten Expression einer Pflanzenperoxidase, umfassend Exprimieren in einem Aspergillus-Wirtsorganismus einer Nukleinsäuresequenz, die eine Klasse III-Peroxidase von EC 1.11.1.7 kodiert, wobei die Aminosäuresequenz der Peroxidase die Aminosäuremotive umfasst:

   HFHDCFV;
   GCD[A,G]S[V,I][I,L][I,L]; und
   VSC[A,S]D[I,L][I,L].

2. Verfahren nach Anspruch 1, wobei die Aminosäuremotive ausgewählt sind aus der Gruppe bestehend aus:

   HFHDCFV;
   GCD[A,G]S[V,I]LL; und
   VSC[A,S]D[I,L]L.

3. Verfahren nach einem beliebigen der Ansprüche 1-2, wobei die Aminosäuresequenz der Peroxidase mindestens 65% Identität, bevorzugt mindestens 70% Identität, mindestens 75% Identität, mindestens 80% Identität, mindestens 85% Identität, mindestens 90% Identität oder mindestens 95% Identität zur Aminosäuresequenz von SEQ ID NO:2, SEQ ID NO:4, Aminosäuren 38 bis 354 von SEQ ID NO: 45, Aminosäuren 30 bis 362 von SEQ ID NO: 55, oder Aminosäuren 23 bis 324 von SEQ ID NO: 67 aufweist.

4. Verfahren nach einem beliebigen der Ansprüche 1-3, wobei die Peroxidase aus der Aminosäuresequenz von SEQ ID NO:2, SEQ ID NO:4, Aminosäuren 38 bis 354 von SEQ ID NO: 45, Aminosäuren 30 bis 362 von SEQ ID NO: 55, oder Aminosäuren 23 bis 324 von SEQ ID NO: 67 besteht.

5. Verfahren nach einem beliebigen der Ansprüche 1-4, wobei die Nukleinsäuresequenz in mindestens 10% der Codons, bevorzugt mindestens 20% der Codons, stärker bevorzugt mindestens 30% der Codons, stärker bevorzugt mindestens 50% der Codons und am stärksten bevorzugt mindestens 75% der Codons codonoptimiert ist.

6. Verfahren nach Anspruch 5, wobei das/die optimierte(n) Codon(s) der Codonverwendung von alpha-Amylase aus *Aspergillus oryzae* entspricht.

7. Verfahren nach einem beliebigen der Ansprüche 1-6, wobei der *Aspergillus*-Wirtsorganismus *Aspergillus oryzae* oder *Aspergillus niger* ist.

8. Aspergillus-Wirtsorganismus umfassend eine Nukleinsäuresequenz, die eine Klasse III-Peroxidase aus EC 1.11.1.7 kodiert, der zum Exprimieren von mindestens 20 mg/ml der Peroxidase fähig ist, wobei die Aminosäuresequenz der Peroxidase die Aminosäuremotive umfasst:

   HFHDCFV;
   GCD[A,G]S[V,I][I,L][I,L]; und
   VSC[A,S]D[I,L][I,L].

9. Aspergillus-Wirtsorganismus nach Anspruch 8, wobei die Aminosäuremotive sind:

   HFHDCFV;
   GCD[A,G]S[V,I]LL; und
   VSC[A,S]D[I,L]L.

10. Aspergillus-Wirtsorganismus nach Anspruch 8 oder 9, wobei die Aminosäuresequenz der Peroxidase mindestens 65% Identität, bevorzugt mindestens 70% Identität, mindestens 75% Identität, mindestens 80% Identität, mindestens 85% Identität, mindestens 90% Identität oder mindestens 95% Identität zur Aminosäuresequenz von SEQ ID NO:2, SEQ ID NO:4, Aminosäuren 38 bis 354 von SEQ ID NO: 45, Aminosäuren 30 bis 362 von SEQ ID NO: 55, oder Aminosäuren 23 bis 324 von SEQ ID NO: 67 aufweist.

11. Aspergillus-Wirtsorganismus nach einem beliebigen der Ansprüche 8-10, wobei die Peroxidase aus der Aminosäuresequenz von SEQ ID NO:2, SEQ ID NO:4, Aminosäuren 38 bis 354 von SEQ ID NO: 45, Aminosäuren 30 bis 362 von SEQ ID NO: 55, oder Aminosäuren 23 bis 324 von SEQ ID NO: 67 besteht.

12. Aspergillus-Wirtsorganismus nach einem beliebigen der Ansprüche 8-11, wobei die Nukleinsäuresequenz in mindestens 10% der Codons, bevorzugt mindestens 20% der Codons, stärker bevorzugt mindestens 30% der Codons, stärker bevorzugt mindestens 50% der Codons und am stärksten bevorzugt mindestens 75% der Codons codonoptimiert ist.

13. Aspergillus-Wirtsorganismus nach einem beliebigen der Ansprüche 8-12, wobei das/die optimierte(n) Codon(s) der Codonverwendung von alpha-Amylase aus *Aspergillus oryzae* entspricht.

14. Aspergillus-Wirtsorganismus nach einem beliebigen der Ansprüche 8-13, wobei der Aspergillus-Wirtsorganismus *Aspergillus oryzae* oder *Aspergillus niger* ist.

**Revendications**

1. Méthode pour l'expression recombinante d'une peroxydase de plante, comprenant l'expression dans un organisme hôte *Aspergillus* d'une séquence d'acide nucléique codant pour une peroxydase de classe III issue de EC 1.11.1.7, la séquence d'acides aminés de la peroxydase comprenant les motifs d'acides aminés :

   HFHDCFV ;
   GCD[A,G]S[V,I][I,L][I,L] ; et
   VSC[A,S]D[I,L][I,L].

2. Méthode selon la revendication 1, dans laquelle les motifs d'acides aminés sont choisis dans le groupe constitué de :

   HFHDCFV ;
   GCD[A,G]S[V,I]LL ; et
   VSC[A,S]D[I,L]L.

3. Méthode selon l'une quelconque des revendications 1 à 2, dans laquelle la séquence d'acides aminés de la peroxydase présente au moins 65 % d'identité, de préférence au moins 70 % d'identité, au moins 75 % d'identité, au moins 80 % d'identité, au moins 85 % d'identité, au moins 90 % d'identité, ou au moins 95 % d'identité, avec la séquence d'acides aminés de SEQ ID NO : 2, de SEQ ID NO : 4, des acides aminés 38 à à 354 de SEQ ID NO : 45, des acides aminés 30 à 362 de SEQ ID NO : 55, ou des acides aminés 23 à 324 de SEQ ID NO : 67.

4. Méthode selon l'une quelconque des revendications 1 à 3, dans laquelle la peroxydase est constituée de la séquence d'acides aminés de SEQ ID NO : 2, de SEQ ID NO : 4, des acides aminés 38 à 354 de SEQ ID NO : 45, des acides aminés 30 à 362 de SEQ ID NO : 55, ou des acides aminés 23 à 324 de SEQ ID NO : 67.

5. Méthode selon l'une quelconque des revendications 1 à 4, dans laquelle la séquence d'acide nucléique est optimisée pour les codons dans au moins 10 % des codons, de préférence au moins 20 % des codons, de façon davantage préférée au moins 30 % des codons, de façon davantage préférée au moins 50 % des codons, et de façon préférée entre toutes au moins 75 % des codons.

6. Méthode selon la revendication 5, dans laquelle le ou les codons optimisés correspondent à l'usage des codons de l'alpha-amylase issue d'*Aspergillus oryzae*.

7. Méthode selon l'une quelconque des revendications 1 à 6, dans laquelle l'organisme hôte *Aspergillus* est *Aspergillus oryzae* ou *Aspergillus niger*.

8. Organisme hôte *Aspergillus* comprenant une séquence d'acide nucléique codant pour une peroxydase de classe III issue de EC.1.11.1.7, capable d'exprimer au moins 20 mg/l de peroxydase, dans lequel la séquence d'acides aminés de la peroxydase comprend les motifs d'acides aminés :

HFHDCFV ;
GCD[A,G]S[V,I][I,L][I,L] ; et
VSC[A,S]D[I,L][I,L].

9. Organisme hôte *Aspergillus* selon la revendication 8, dans lequel les motifs d'acides aminés sont :

HFHDCFV ;
GCD[A,G]S[V,I]LL ; et
VSC[A,S]D[I,L]L.

10. Organisme hôte *Aspergillus* selon la revendication 8 ou 9, dans lequel la séquence d'acides aminés de la peroxydase présente au moins 65 % d'identité, de préférence au moins 70 % d'identité, au moins 75 % d'identité, au moins 80 % d'identité, au moins 85 % d'identité, au moins 90 % d'identité, ou au moins 95 % d'identité, avec la séquence d'acides aminés de SEQ ID NO : 2, de SEQ ID NO : 4, des acides aminés 38 à 354 de SEQ ID NO : 45, des acides aminés 30 à 362 de SEQ ID NO : 55, ou des acides aminés 23 à 324 de SEQ ID NO : 67.

11. Organisme hôte *Aspergillus* selon l'une quelconque des revendications 8 à 10, dans lequel la peroxydase est constituée de la séquence d'acides aminés de SEQ ID NO : 2, de SEQ ID NO : 4, des acides aminés 38 à 354 de SEQ ID NO : 45, des acides aminés 30 à 362 de SEQ ID NO : 55, ou des acides aminés 23 à 324 de SEQ ID NO : 67.

12. Organisme hôte *Aspergillus* selon l'une quelconque des revendications 8 à 11, dans lequel la séquence d'acide nucléique est optimisée pour les codons dans au moins 10 % des codons, de préférence au moins 20 % des codons, de façon davantage préférée au moins 30 % des codons, de façon davantage préférée au moins 50 % des codons, et de façon préférée entre toutes au moins 75 % des codons.

13. Organisme hôte *Aspergillus* selon l'une quelconque des revendications 8 à 12, dans lequel le ou les codons optimisés correspondent à l'usage des codons de l'alpha-amylase issue d'*Aspergillus oryzae*.

14. Organisme hôte *Aspergillus* selon l'une quelconque des revendications 8 à 13, l'organisme hôte *Aspergillus* étant *Aspergillus oryzae* ou *Aspergillus niger*.

**EP 2 665 812 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9855629 A
- US 6818752 B
- WO 2005019443 A
- WO 9735956 A
- EP 429490 A
- WO 9614404 A
- WO 0168864 A
- EP 238023 A
- WO 9600787 A
- WO 2004069872 A
- WO 2005070962 A

### Non-patent literature cited in the description

- **NEEDLEMAN ; WUNSCH.** *J. Mol. Biol.,* 1970, vol. 48, 443-453
- **RICE et al.** EMBOSS: The European Molecular Biology Open Software Suite. *Trends Genet.,* 2000, vol. 16, 276-277
- **RICE et al.** *EMBOSS: The European Molecular Biology Open Software Suite,* 2000
- Nomenclature Committee of the International Union of Biochemistry and Molecular Biology. Academic Press Inc, 1992
- **PASSARDI et al.** The class III peroxidase multigenic family in rice and its evolution in land plants. *Phytochemistry,* 2004, vol. 65 (13), 1879-93
- **RYMOND ; ROSBASH.** The Molecular and Cellular Biology of the Yeast Saccharomyces. Cold Spring Harbor Laboratory Press, 1992, 143-192
- **GURR et al.** Gene Structure in Eukaryotic Microbes. IRL Press, 1987, 93-139
- **T. KUMAZAKI et al.** *J. Cell. Sci,* 1999, vol. 112, 1449-1453
- **YELTON et al.** *Proceedings of the National Academy of Sciences USA,* 1984, vol. 81, 1470-1474
- **MALARDIER et al.** *Gene,* 1989, vol. 78, 147-156
- Guide to Yeast Genetics and Molecular Biology. **BECKER ; GUARENTE.** Methods in Enzymology. Academic Press, Inc, vol. 194, 182-187
- **ITO et al.** *Journal of Bacteriology,* 1983, vol. 153, 163
- **HINNEN et al.** *Proceedings of the National Academy of Sciences USA,* 1978, vol. 75, 1920
- Protein Purification. VCH Publishers, 1989